# EUROPEAN PATENT APPLICATION

(11) **EP 4 567 034 A1**
(43) Date of publication of application: **11.06.2025**
(21) Application number: 22954093.5
(22) Date of filing: 01.08.2022
(51) Int. Cl.: C07D 421/10, C07D 421/14, C07D 293/06, C07D 413/10, C07D 413/14, A61K 31/095, A61P 25/00, A61P 37/00, A23L 33/10, A23K 20/137

(54) **NOVEL HETEROCYCLYL-PHENYL-METHYLAMINE DERIVATIVE, PREPARATION METHOD THEREFOR, AND USE THEREOF FOR PREVENTION, ALLEVIATION, OR TREATMENT OF MULTIPLE SCLEROSIS**

(71) Applicant: Korea Institute of Science and Technology, Seoul 02792 (KR)
(72) Inventor: PARK, Ki Duk, Seoul 02792 (KR); PARK, Jong Hyun, Seoul 02792 (KR); PARK, Sun Jun, Seoul 02792 (KR); KIM, Yoowon, Seoul 02792 (KR); KIM, Hyeon Jeong, Seoul 02792 (KR); KIM, Si Won, Seoul 02792 (KR); KIM, Jushin, Seoul 02792 (KR); CHOI, Ji Won, Seoul 02792 (KR); KIM, Byung Eun, Seoul 02792 (KR); LEE, Elijah Hwejin, Seoul 02792 (KR)
(74) Representative: f & e patent
(86) International application number: PCT/KR2022/011333
(87) International publication number: WO 2024/029638

(57) **Abstract**

The present disclosure relates to a series of novel heterocyclyl-phenyl-methylamine derivatives, a preparation method thereof, and use thereof for prevention, alleviation, or treatment of multiple sclerosis.

## Description

### [Technical Field]

The present disclosure relates to a series of novel heterocyclyl-phenyl-methylamine derivatives, a preparation method thereof, and use thereof for prevention, alleviation, or treatment of multiple sclerosis.

### [Background Art]

Multiple sclerosis (MS) can occur at any age; however, it is especially common between the ages of 20 and 40. Multiple sclerosis was first described in 1868 by Jean-Martin Charcot, and is twice as common in women as in men. Additionally, it is more common in European Caucasians than in Asians or people of African descent. In addition, some patients develop the disease due to genes inherited from their parents. In modern society, the disease is increasing not only in Western countries but also in Asian countries due to westernized diets. Multiple sclerosis can cause lesions to appear in various parts of the central nervous system (CNS). However, they mainly appear in the brain, spinal cord, white matter, and optic nerves. They show different neurological abnormalities depending on where the lesions appear. Even among patients with the same type of multiple sclerosis, symptoms may vary for each person. It is a chronic inflammatory and demyelinating central nervous system autoimmune disease associated with immune factors such as antibodies and complement, usually caused by inflammatory demyelination. Demyelination occurs when the myelin sheath of the nerve is damaged by immune cells (T cells). In particular, scarring forms and sclerosis begins. When the portion of the myelin sheath that transmits signals is damaged and demyelination occurs, the rate of signal transmission becomes much slower than in individuals without lesions. Even if the myelin sheath reforms as the wound heals, the reaction rate of the nerve endings does not return to the original state, tending to be slow.
There are several types of symptoms in patients with multiple sclerosis. The most common type is relapsing-remitting multiple sclerosis. The main symptoms of multiple sclerosis include sensory and motor paralysis, optic nerve inflammation, Lhermitte's symptom, double vision, Uhthoff's sign, *etc.* The symptoms improve and worsen repeatedly. Accumulated nerve damage causes the disease to become more severe, and in some cases, this can lead to a chronic disease. Autoreactive CD4 + T cells migrate from the blood to the central nervous system, react with myelin, and become activated. Substances secreted by CD4 + T cells induce inflammation and prompt macrophages and B cells to enter the central nervous system. Autoreactive CD8 + T cells, which destroy cells containing antigenic determinants, also migrate from the blood to the central nervous system and reactivate, replicating and proliferating independently. Macrophages and microglia in the central nervous system secrete BAFF (B cell activating factor belonging to the TNF family), thereby activating B cells. Multiple sclerosis caused by an autoimmune response is linked to macrophages that destroy myelin and produce pro-inflammatory cytokines that phagocytose and destroy myelin marked with antibodies and complement. Currently, Novartis' FTY720 (fingolimod) is widely used as a therapeutic agent for multiple sclerosis. However, it causes bradycardia, one of the most significant side effects, highlighting the necessity for the development of a treatment with fewer side effects or none at all. It has been reported that these side effects are due to the activation of the S1P3 receptor, and thus, new therapeutic agents are being developed with a focus on activating S1P1 and S1P5 without activating the S1P3 receptor. Novartis' BAF312, which was approved by the FDA in 2019, was highly anticipated as a therapeutic agent that showed excellent activity against S1P1 and S1P5 without activating S1P3, yet bradycardia, a side effect of FTY720, still occurred. The mechanism of these drugs is to down-regulate S1P1 present in lymphocyte immune cells by treatment with S1P1 agonists which prevents them from entering the central nervous system. In particular, the S1P1 receptors form colonies, enter cells through endocytosis, and then internalize and degrade, producing an inhibitory effect. Thus, there is an urgent need to develop drugs and/or treatment technologies that have no or few side effects and produce excellent therapeutic effects.

### [Disclosure]

### [Technical Problem]

The present inventors have conducted extensive research to discover novel small-molecule compounds that exhibit excellent activity as sphingosine-1-phosphate receptor (S1P₁) agonists with few side effects, and as a result, they have confirmed that a series of heterocyclyl-phenyl-methylamine derivatives and salts thereof not only significantly decreases the blood lymphocyte count, but also effectively delays or significantly recovers the onset of symptoms of motor dysfunction in EAE mice, a multiple sclerosis animal model, thereby completing the present disclosure.

### [Technical Solution]

An object of the present disclosure is to provide a series of novel heterocyclyl-phenyl-methylamine derivatives or pharmaceutically acceptable salts thereof.

Another object of the present disclosure is to provide a method for preparing the heterocyclyl-phenyl-methylamine derivatives or pharmaceutically acceptable salts thereof.

Still another object of the present disclosure is to provide a pharmaceutical composition for preventing or treating multiple sclerosis (MS), comprising the heterocyclyl-phenyl-methylamine derivatives or pharmaceutically acceptable salts thereof, as an active ingredient.

Still another object of the present disclosure is to provide a method for preventing or treating multiple sclerosis, comprising administering the pharmaceutical composition to a subject in need thereof.

Still another object of the present disclosure is to provide a food composition for preventing or alleviating multiple sclerosis, comprising the heterocyclyl-phenyl-methylamine derivatives or food-acceptable salts thereof, as an active ingredient.

Still another object of the present disclosure is to provide a feed composition for preventing or alleviating multiple sclerosis, comprising the heterocyclyl-phenyl-methylamine derivatives or feed-acceptable salts thereof, as an active ingredient

### [Advantageous Effects]

The heterocyclyl-phenyl-methylamine derivatives of the present disclosure can regulate receptor internalization and blood lymphocyte count through their agonist activity for the S1P1 receptor, and further suppress the loss of reflexes and paralysis, which are symptoms caused by multiple sclerosis, while exhibiting excellent effects in reducing blood inflammatory cells, and therefore can be usefully used for the prevention or treatment of multiple sclerosis.

### [Brief Description of the Drawings]

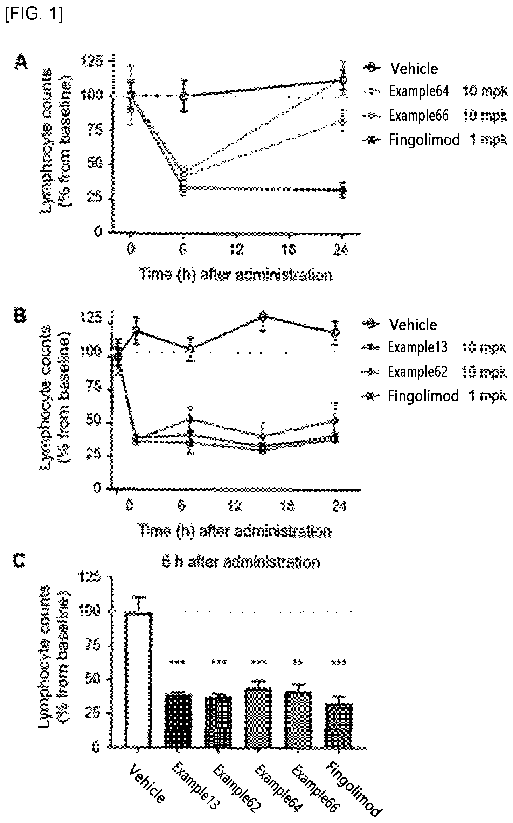

FIG. 1 is a diagram showing the change in the peripheral blood lymphocyte count over time in normal rats administered Compounds 13, 62, 64, or 66 of the present disclosure. FIGS. 1A and 1B show the change in the blood lymphocyte count over time after oral administration of Compounds 64 and 66, and 13 and 62 of the present disclosure at a dose of 10 mg/kg, respectively, which is calculated as a percentage of the value before administration, together with the results for the positive control group administered with fingolimod, a drug of the same mechanism that has been clinically approved. FIG. 1C shows the lymphocyte count measured 6 hours after oral administration of the vehicle or drug, expressed as a percentage based on the vehicle-administered negative control group.

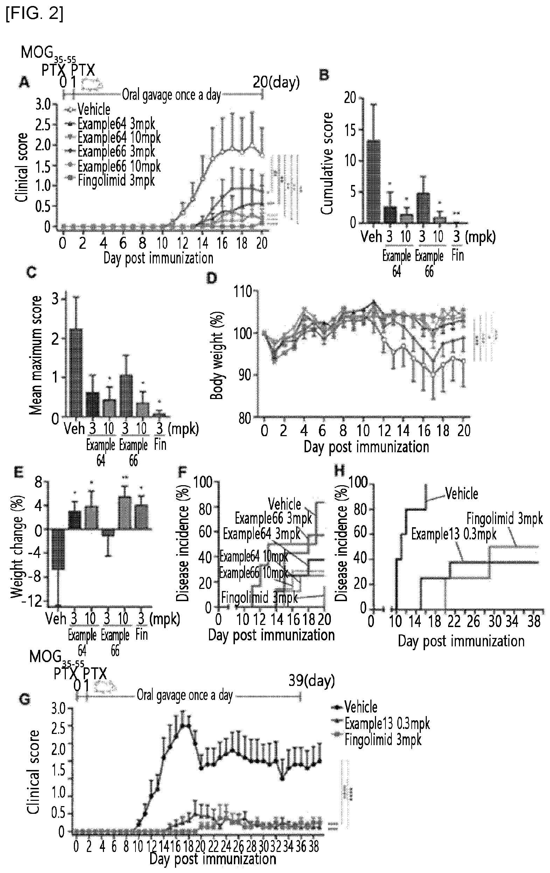

FIG. 2 is a diagram showing the preparation of a multiple sclerosis rodent model, an outline of drug administration, and the alleviation of symptoms of multiple sclerosis due to administration of the compounds of the present disclosure in a multiple sclerosis rodent model. FIG. 2A shows the change in the daily clinical symptom index (numericalization of the degree of paralysis of the tail and legs). FIGS. 2B and 2C show the area under the graph of each group from FIG. 2A. FIG. 2D shows the change in daily body weight of each group, and FIG. 2E shows the degree of change in body weight on the last day of the test in each group based on the start date of induction of the experimental animal model. FIG. 2F shows the time and rate at which multiple sclerosis symptoms appear in each group. FIG. 2G shows the preparation of a multiple sclerosis animal model, an outline of drug administration, and the daily clinical symptom index for administration of Compound 13, and FIG. 2H shows the time and rate at which multiple sclerosis symptoms appear in each group.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

Further, throughout the whole specification, when a certain portion "includes" or "comprises" a certain component, this indicates that other components are not excluded and may be further included unless explicitly described to the contrary.

Hereinbelow, the present disclosure will be described in detail.

A first aspect of the present disclosure to achieve the objects above provides a compound represented by Formula 1 below or a pharmaceutically acceptable salt thereof:

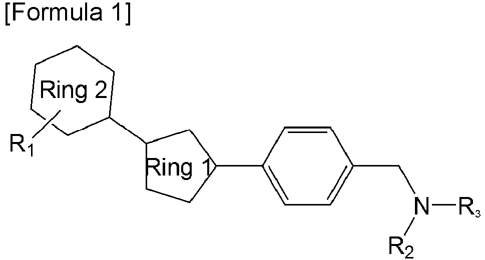

In the Formula 1,
Ring 1 is selenazolenyl or isoxazolenyl;
Ring 2 is C₆₋₁₀ aryl or 5 to 10-membered heteroaryl;
R₁ is one or more substituents identical or different from each other selected from the group consisting of hydrogen, cyano, halo(gen), hydroxy, C₁₋₆ alkyl, C₁₋₆ alkoxy, and C₁₋₆ haloalkyl;
R₂ and R₃ are each independently hydrogen, C₁₋₆ alkoxycarbonyl-C₁₋₆ alkyl, or sodium carboxy-C₁₋₆ alkyl; or
R₂ and R₃ are connected to each other to form an unsubstituted or substituted 3 to 10-membered heterocyclyl including the nitrogen to which the R₂ and R₃ are attached.

For example, the compound of the present disclosure may be represented by any one of Formulae 2 to 4 below, but is not limited thereto:

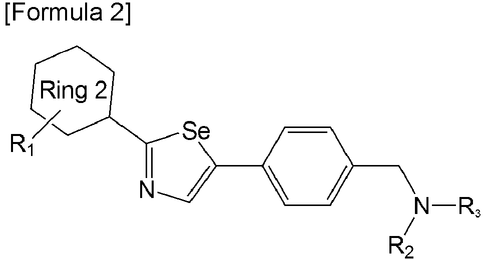

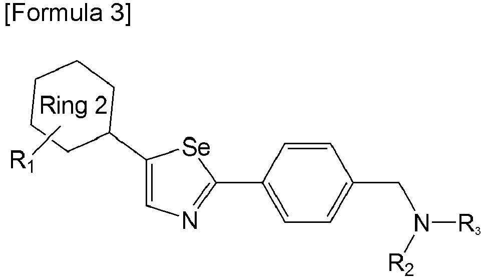

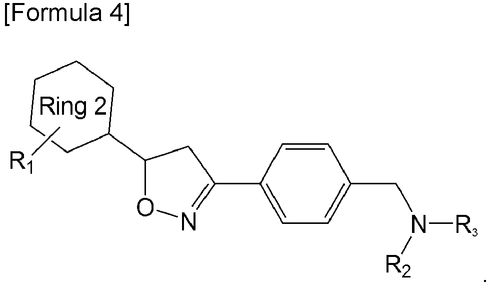

For example, in the Formulae 1 to 4, Ring 2 may be phenyl or pyridinyl, but is not limited thereto.

For example, in the Formulae 1 to 4, R₁ may be none, cyano, bromo, chloro, hydroxy, ethyl, propyl, tert-butyl, methoxy, ethoxy, isopropoxy, chloromethyl, or trifluoromethyl, but is not limited thereto. Specifically, R₁ may be any one or two or more substituents identical or different from each other selected from the substituents described above, but is not limited thereto.

For example, in the Formulae 1 to 4, R₂ may be hydrogen, and R₃ may be ethoxycarbonylmethyl, methoxycarbonylethyl, sodium carboxymethyl, or sodium carboxyethyl, but is not limited thereto.

Alternatively, in the Formulae 1 to 4, R₂ and R₃ may be connected to each other to form azetidinyl, pyrrolidinyl, or piperidinyl, which is unsubstituted or substituted with one or more selected from the group consisting of carboxy, methoxycarbonyl, ethoxycarbonyl, tert-butoxycarbonyl, and sodium carboxy including the nitrogen to which the R₂ and R₃ are attached, but is not limited thereto.

Specifically, the compound may be
1. Methyl 1-(4-(2-(4-tert-butylphenyl)-1,3-selenazole-5-yl)benzyl)azetidine-3-carboxylate;
2. Sodium 1-(4-(2-(4-tert-butylphenyl)-1,3-selenazole-5-yl)benzyl)azetidine-3-carboxylate;
3. Ethyl 1-(4-(2-(4-tert-butylphenyl)-1,3-selenazole-5-yl)benzyl)azetidine-3-carboxylate;
4. Tert-butyl 1-(4-(2-(4-tert-butylphenyl)-1,3-selenazole-5-yl)benzyl)azetidine-3-carboxylate;
5. Methyl 1-(4-(2-(3-isocyano-4-isopropoxyphenyl)-1,3-selenazole-5-yl)benzyl)azetidine-3-carboxylate;
6. Sodium 1-(4-(2-(3-isocyano-4-isopropoxyphenyl)-1,3-selenazole-5-yl)benzyl)azetidine-3-carboxylate;
7. Ethyl 1-(4-(2-(3-cyano-4-isopropoxyphenyl)-1,3-selenazole-5-yl)benzyl)azetidine-3-carboxylate;
8. Tert-butyl 1-(4-(2-(3-cyano-4-isopropoxyphenyl)-1,3-selenazole-5-yl)benzyl)azetidine-3-carboxylate;
9. Methyl 1-(4-(2-(4-isopropoxy-3-(trifluoromethyl)phenyl)-1,3-selenazole-5-yl)benzyl)azetidine-3-carboxylate;
10. 1-(4-(2-(4-isopropoxy-3-(trifluoromethyl)phenyl)-1,3-selenazole-5-yl)benzyl)azetidine-3-carboxylic acid;
11. Ethyl 1-(4-(2-(4-isopropoxy-3-(trifluoromethyl)phenyl)-1,3-selenazole-5-yl)benzyl)azetidine-3-carboxylate;
12. Methyl 1-(4-(2-(3-chloro-4-isopropoxyphenyl)-1,3-selenazole-5-yl)benzyl)azetidine-3-carboxylate;
13. Sodium 1-(4-(2-(3-chloro-4-isopropoxyphenyl)-1,3-selenazole-5-yl)benzyl)azetidine-3-carboxylate;
14. Methyl 1-(4-(2-(3-chloro-4-ethoxyphenyl)-1,3-selenazole-5-yl)benzyl)azetidine-3-carboxylate;
15. Sodium 1-(4-(2-(3-chloro-4-ethoxyphenyl)-1,3-selenazole-5-yl)benzyl)azetidine-3-carboxylate;
16. Methyl 1-(4-(2-(4-methoxyphenyl)-1,3-selenazole-5-yl)benzyl)azetidine-3-carboxylate;
17. 1-(4-(2-(4-methoxyphenyl)-1,3-selenazole-5-yl)benzyl)azetidine-3-carboxylic acid;
18. Methyl 1-(4-(2-(4-isopropoxyphenyl)-1,3-selenazole-5-yl)benzyl)azetidine-3-carboxylate;
19. 1-(4-(2-(4-isopropoxyphenyl)-1,3-selenazole-5-yl)benzyl)azetidine-3-carboxylic acid;
20. Ethyl 1-(4-(2-(4-isopropoxyphenyl)-1,3-selenazole-5-yl)benzyl)azetidine-3-carboxylate;
21. Methyl 1-(4-(2-(3-isopropoxyphenyl)-1,3-selenazole-5-yl)benzyl)azetidine-3-carboxylate;
22. 1-(4-(2-(3-isopropoxyphenyl)-1,3-selenazole-5-yl)benzyl)azetidine-3-carboxylic acid;
23. Methyl 1-(4-(2-(2-isopropoxyphenyl)-1,3-selenazole-5-yl)benzyl)azetidine-3-carboxylate;
24. 1-(4-(2-(2-isopropoxyphenyl)-1,3-selenazole-5-yl)benzyl)azetidine-3-carboxylic acid;
25. Ethyl 1-(4-(2-(2-isopropoxyphenyl)-1,3-selenazole-5-yl)benzyl)azetidine-3-carboxylate;
26. Methyl 1-(4-(2-(pyridine-2-yl)-1,3-selenazole-5-yl)benzyl)azetidine-3-carboxylate;
27. Sodium 1-(4-(2-(pyridine-2-yl)-1,3-selenazole-5-yl)benzyl)azetidine-3-carboxylate;
28. Methyl 1-(4-(2-(pyridine-3-yl)-1,3-selenazole-5-yl)benzyl)azetidine-3-carboxylate;
29. Sodium 1-(4-(2-(pyridine-3-yl)-1,3-selenazole-5-yl)benzyl)azetidine-3-carboxylate;
30. Methyl 1-(4-(2-(4-tert-butylphenyl)-1,3-selenazole-5-yl)benzyl)pyrrolidine-3-carboxylate;
31. Sodium 1-(4-(2-(4-tert-butylphenyl)-1,3-selenazole-5-yl)benzyl)pyrrolidine-3-carboxylate;
32. Methyl 1-(4-(2-(4-tert-butylphenyl)-1,3-selenazole-5-yl)benzyl)piperidine-4-carboxylate;
33. Methyl 1-(4-(2-(3-chloro-4-isopropoxyphenyl)-1,3-selenazole-5-yl)benzyl)pyrrolidine-3-carboxylate;
34. Sodium 1-(4-(2-(3-chloro-4-isopropoxyphenyl)-1,3-selenazole-5-yl)benzyl)pyrrolidine-3-carboxylate;
35. Ethyl 2-(4-(2-(3-chloro-4-isopropoxyphenyl)-1,3-selenazole-5-yl)benzylamino)acetate;
36. Sodium 2-(4-(2-(3-chloro-4- isopropoxyphenyl)-1,3-selenazole-5-yl)benzylamino)acetate;
37. Methyl 3-(4-(2-(3-chloro-4-isopropoxyphenyl)-1,3-selenazole-5-yl)benzylamino)propanoate;
38. Sodium 3-(4-(2-(3-chloro-4-isopropoxyphenyl)-1,3-selenazole-5-yl)benzylamino)propanoate;
39. Methyl 1-(4-(5-(4-tert-butylphenyl)-1,3-selenazole-2-yl)benzyl)azetidine-3-carboxylate;
40. Sodium 1-(4-(5-(4-tert-butylphenyl)-1,3-selenazole-2-yl)benzyl)azetidine-3-carboxylate;
41. Methyl 1-(4-(5-(3-chloro-4-isopropoxyphenyl)-1,3-selenazole-2-yl)benzyl)azetidine-3-carboxylate;
42. Sodium 1-(4-(5-(3-chloro-4-isopropoxyphenyl)-1,3-selenazole-2-yl)benzyl)azetidine-3-carboxylate;
43. Methyl 1-(4-(5-phenyl-4,5-dihydroisoxazole-3-yl)benzyl)azetidine-3-carboxylate;
44. 1-(4-(5-phenyl-4,5-dihydroisoxazole-3-yl)benzyl)azetidine-3-carboxylic acid;
45. Methyl 1-(4-(5-(2-bromophenyl)-4,5-dihydroisoxazole-3-yl)benzyl)azetidine-3-carboxylate;
46. 1-(4-(5-(2-bromophenyl)-4,5-dihydroisoxazole-3-yl)benzyl)azetidine-3-carboxylic acid;
47. Methyl 1-(4-(5-(3-bromophenyl)-4,5-dihydroisoxazole-3-yl)benzyl)azetidine-3-carboxylate;
48. 1-(4-(5-(3-bromophenyl)-4,5-dihydroisoxazole-3-yl)benzyl)azetidine-3-carboxylic acid;
49. Methyl 1-(4-(5-(4-bromophenyl)-4,5-dihydroisoxazole-3-yl)benzyl)azetidine-3-carboxylate;
50. 1-(4-(5-(4-bromophenyl)-4,5-dihydroisoxazole-3-yl)benzyl)azetidine-3-carboxylic acid;
51. 1-(4-(5-(4-hydroxyphenyl)-4,5-dihydroisoxazole-3-yl)benzyl)azetidine-3-carboxylic acid;
52. Methyl 1-(4-(5-(4-(chloromethyl)phenyl)-4,5-dihydroisoxazole-3-yl)benzyl)azetidine-3-carboxylate;
53. Sodium 1-(4-(5-(4-(chloromethyl)phenyl)-4,5-dihydroisoxazole-3-yl)benzyl)azetidine-3-carboxylate;
54. Methyl 1-(4-(5-(4-ethylphenyl)-4,5-dihydroisoxazole-3-yl)benzyl)azetidine-3-carboxylate;
55. Sodium 1-(4-(5-(4-ethylphenyl)-4,5-dihydroisoxazole-3-yl)benzyl)azetidine-3-carboxylate;
56. Sodium 1-(4-(5-(4-propylphenyl)-4,5-dihydroisoxazole-3-yl)benzyl)azetidine-3-carboxylate;
57. Methyl 1-(4-(5-(4-tert-butylphenyl)-4,5-dihydroisoxazole-3-yl)benzyl)azetidine-3-carboxylate;
58. Sodium 1-(4-(5-(4-tert-butylphenyl)-4,5-dihydroisoxazole-3-yl)benzyl)azetidine-3-carboxylate;
59. Methyl 1-(4-(5-(3-cyano-4-isopropoxyphenyl)-4,5-dihydroisoxazole-3-yl)benzyl)azetidine-3-carboxylate;
60. 1-(4-(5-(3-cyano-4-isopropoxyphenyl)-4,5-dihydroisoxazole-3-yl)benzyl)azetidine-3-carboxylic acid;
61. Methyl 1-(4-(5-(3-chloro-4-ethoxyphenyl)-4,5-dihydroisoxazole-3-yl)benzyl)azetidine-3-carboxylate;
62. Sodium 1-(4-(5-(3-chloro-4-ethoxyphenyl)-4,5-dihydroisoxazole-3-yl)benzyl)azetidine-3-carboxylate;
63. Methyl 1-(4-(5-(3-chloro-4-isopropoxyphenyl)-4,5-dihydroisoxazole-3-yl)benzyl)azetidine-3-carboxylate;
64. Sodium 1-(4-(5-(3-chloro-4-isopropoxyphenyl)-4,5-dihydroisoxazole-3-yl)benzyl)azetidine-3-carboxylate;
65. Methyl 1-(4-(5-(4-isopropoxy-3-(trifluoromethyl)phenyl)-4,5-dihydroisoxazole-3-yl)benzyl)azetidine-3-carboxylate;
66. Sodium 1-(4-(5-(4-isopropoxy-3-(trifluoromethyl)phenyl)-4,5-dihydroisoxazole-3-yl)benzyl)azetidine-3-carboxylate;
67. Methyl 1-(4-(5-(pyridine-2-yl)-4,5-dihydroisoxazole-3-yl)benzyl)azetidine-3-carboxylate;
68. Sodium 1-(4-(5-(pyridine-2-yl)-4,5-dihydroisoxazole-3-yl)benzyl)azetidine-3-carboxylate;
69. Methyl 1-(4-(5-(3-chloro-4-isopropoxyphenyl)-4,5-dihydroisoxazole-3-yl)benzyl)pyrrolidine-3-carboxylate;
70. Sodium 1-(4-(5-(3-chloro-4-isopropoxyphenyl)-4,5-dihydroisoxazole-3-yl)benzyl)pyrrolidine-3-carboxylate; or
71. Sodium 1-(4-(5-(3-chloro-4-isopropoxyphenyl)-4,5-dihydroisoxazole-3-yl)benzyl)piperidine-4-carboxylate, but is not limited thereto.

The compound of the present disclosure may exist in the form of a pharmaceutically acceptable salt. For the salt, acid addition salts formed by pharmaceutically acceptable free acids may be useful. As used herein, the term "pharmaceutically acceptable salt" refers to any organic or inorganic addition salt of the compound, which has a concentration for exhibiting an effective action, is relatively nontoxic and unharmful to patients, and the adverse effects resulting from the salt do not deteriorate the advantageous effects of the compound represented by Formula 1.

Acid addition salts may be prepared by a conventional method, for example, by dissolving the compound in an excess amount of aqueous acid solution, followed by precipitating the salt in a water-miscible organic solvent, *e*.*g*., methanol, ethanol, acetone, or acetonitrile. An equimolar amount of a compound and an acid or alcohol in water (*e*.*g*., glycol monomethylether) may be heated and then the mixture may be dried by evaporation, or the precipitated salt may be subjected to suction filtration.

In particular, organic acids and inorganic acids may be used as a free acid. Examples of the inorganic acids may include hydrochloric acid, phosphoric acid, sulfuric acid, nitric acid, tartaric acid, *etc*.; organic carbonic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, *etc.,* but are not limited thereto.

Additionally, a pharmaceutically acceptable metal salt may be prepared using a base. An alkali metal salt or alkali earth metal salt may be prepared, for example, by dissolving a compound in an excess amount of an alkali metal hydroxide or alkali earth metal hydroxide solution, filtering a non-dissolved compound salt obtained therefrom, and evaporating the filtrate, followed by drying. In particular, examples of a pharmaceutically acceptable metal salt to be prepared may include sodium, potassium, or calcium salts, but are not limited thereto. Additionally, a corresponding silver salt may be prepared by reacting an alkali metal or alkali earth metal salt with an appropriate silver salt (*e*.*g*., silver nitrate), but the preparation method is not limited thereto.

The pharmaceutically acceptable salt of the compound of the present disclosure may include salts of an acidic or basic group that can be present in the compound of Formula 1, unless indicated otherwise. For example, the pharmaceutically acceptable salt may include sodium, calcium, or potassium salts of a hydroxyl group, *etc.,* and other pharmaceutically acceptable salts of an amino group may include hydrobromide, sulfate, hydrogen sulfate, phosphate, hydrogen phosphate, dihydrogen phosphate, acetate, succinate, citrate, tartrate, lactate, mandelate, methanesulfonate (mesylate), p-toluenesulfonate (tosylate), *etc.,* and they can be prepared by a preparation method known in the art.

As a salt of the heterocyclyl-phenyl-methylamine derivative compounds, which are pharmaceutically acceptable salts, any salt of heterocyclyl-phenyl-methylamine derivative compounds which exhibits the same pharmacological activity as the heterocyclyl-phenyl-methylamine derivative compounds can be used without limitation.

Additionally, the compound represented by Formula 1 according to the present disclosure includes not only pharmaceutically acceptable salts thereof, but also solvates such as hydrates that can be prepared therefrom, and all possible stereoisomers, without limitation. The solvate and stereoisomer of the compound represented by Formula 1 may be prepared from the compound represented by Formula 1 using common methods known in the art.

Further, the compound represented by Formula 1 according to the present disclosure may be prepared either in a crystalline form or in a non-crystalline form, and when the compound is prepared in a crystalline form, it may be optionally hydrated or solvated. In the present disclosure, the compound represented by Formula 1 may not only include a stoichiometric hydrate, but also include compounds containing various amounts of water. The solvate of the compound represented by Formula 1 according to the present disclosure includes both stoichiometric solvates and non-stoichiometric solvates.

A second aspect of the present disclosure provides a method for preparing the compound of any one of claims 1 to 7, or a pharmaceutically acceptable salt thereof, comprising: aminating an aldehyde group of a compound represented by Formula 5 below:

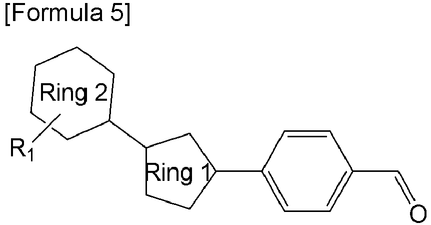

In the Formula 1,
Ring 1 is selenazolenyl or isoxazolenyl;
Ring 2 is C₆₋₁₀ aryl or 5 to 10-membered heteroaryl; and
R₁ is one or more substituents identical or different from each other selected from the group consisting of hydrogen, cyano, halo(gen), hydroxy, C₁₋₆ alkyl, C₁₋₆ alkoxy, and C₁₋₆ haloalkyl.

For example, the compound of Formula 5 may be prepared by:
Step a of reacting R₁-Ring 2-nitrile or 4-(hydroxymethyl)benzonitrile with selenium and carbon monoxide to produce R₁-Ring 2-substituted selenoamide or 4-hydroxybenzoselenamide;
Step b of reacting the product of Step a with 2-bromo-1,1-diethoxyethane and p-toluenesulfonic acid to form a 1,3-selenazole ring in which Ring 2 is substituted at carbon 5 or phenyl is substituted at carbon 2;
Step c of halogenating the unsubstituted carbon 2 or carbon 5 of the 1,3-selenazole ring of the product of Step b; and
Step d of reacting the product of Step c with 4-formylphenylboronic acid or R₁-Ring 2-boronic acid to introduce an additional substituent at the halogenated carbon 2 or carbon 5, but is not limited thereto.

For example, through the series of reactions above, a compound represented by Formula 2 and a precursor compound of Formula 3 may be obtained, but is not limited thereto.

When 4-(hydroxymethyl)benzonitrile is reacted according to the above-described series of processes as a reactant, a precursor compound including a hydroxymethyl group instead of a formyl group on the phenyl ring of Formula 3 may be obtained, which can be converted into a compound of Formula 3 by additionally performing Step e of oxidizing the hydroxy group on the phenyl ring after Step d. In particular, the reaction of Step e may be performed using pyridinium chlorochromate (PCC), but is not limited thereto.

For example, the compound of Formula 5 may be prepared by:
Step a' of reacting N-hydroxy-4-(hydroxymethyl)benzimidoyl chloride with triethylamine to remove hydrochloric acid;
Step b' of reacting the product of Step a' with ethenyl-substituted R₁-Ring 2 to produce (4-(5-(R₁-Ring 2)-4,5-dihydroisoxazole-3-yl)phenyl)methanol; and
Step c' of oxidizing the hydroxyl group on the phenyl ring of the product of Step b', but is not limited thereto.

For example, through the series of reactions above, a compound represented by Formula 4 may be obtained, but is not limited thereto.

The specific preparation method is merely an example, and the scope of the present disclosure is not limited thereto, and related reactions known in the art may be performed as described or by appropriate modification. Furthermore, the reactants of each step may be purchased commercially or synthesized by a known method, but are not limited thereto. Additionally, the preparation method of the present disclosure can optionally comprise a separation and/or purification step after each step in order to increase the purity and/or yield of the product, but is not limited thereto.

A third aspect of the present disclosure provides a pharmaceutical composition for preventing or treating multiple sclerosis (MS), comprising the compound of the first aspect or a pharmaceutically acceptable salt thereof, as an active ingredient.

A fourth aspect of the present disclosure provides a method for preventing or treating multiple sclerosis, comprising administering the pharmaceutical composition of the third aspect to a subject in need thereof.

The method of the present disclosure for preventing or treating multiple sclerosis may be performed together with rehabilitation, but is not limited thereto.

The terms "the compound of the first aspect" and "pharmaceutically acceptable salt" are as described above.

As used herein, the term "multiple sclerosis (MS)" refers to a demyelinating disease caused by damage to the myelin, the insulating covers that protect nerve cells in the brain and spinal cord. This damage impairs the ability to transmit signals in parts of the nervous system, leading to a wide range of signs and symptoms, including physical, mental, and sometimes psychiatric problems. Specific clinical manifestations may include vision loss, eye movement disorders (*e*.*g*., nystagmus), sensory symptoms (*e*.*g*., numbness, tingling, throbbing in the skin), weakness, convulsion, ataxia, bladder disorders, cognitive disorders, emotional disorders, and seizure disorders. Multiple sclerosis can take various forms, from relapsing forms that appear as isolated attacks to progressive forms which accumulate over time. Symptoms may completely disappear between attacks, but permanent neurological problems may still remain, and in particular, disease progression may be accompanied. The underlying mechanism of multiple sclerosis is known to be destruction by the immune system or errors in myelin-producing cells, but genetic factors and environmental factors such as triggers by viral infections have been suggested as causes thereof, and there is no known cure.

For example, the pharmaceutical composition of the present disclosure may comprise the compound in the form of a sodium salt, but is not limited thereto.

For example, the compound represented by Formula 1, which is an effective ingredient of the pharmaceutical composition of the present disclosure, may act as a sphingosine-1-phosphate receptor (S1P₁) modulator.

As used herein, the term "sphingosine-1-phosphate receptor (S1P₁)" refers to a type of G protein-coupled receptor that is the target of the lipid signalling molecule sphingosine-1-phosphate (S1P), is one of five subtypes divided into S1P₁, S1P₂, S1P₃, S1P₄, and S1P₅, and is the first member of the S1P receptor family to be cloned from endothelial cells in 1990. These subtypes are expressed in a wide variety of tissues, with each subtype exhibiting a different cell specificity, although they are found at their highest density on leukocytes. It is known that S1P₁ are expressed ubiquitously, and their known agonist includes fingolimod, which is mainly used in the treatment of multiple sclerosis as an immunomodulator. The pharmaceutical composition comprising the compound of the present disclosure can act as an S1P1 agonist and thereby alleviate clinical symptoms at the same level as fingolimod and/or delay the onset thereof in a multiple sclerosis animal model, and thus can be used as a substitute for fingolimod in the prevention and/or treatment of multiple sclerosis.

Further, the pharmaceutical composition of the present disclosure can alleviate autoimmune reactions by decreasing the blood lymphocyte count.

Additionally, the pharmaceutical composition of the present disclosure can alleviate symptoms of stiffness or paralysis, as well as delay the onset of these symptoms of multiple sclerosis.

As used herein, the term "prevention" refers to all actions to inhibit or delay the occurrence, spread, or recurrence of multiple sclerosis by the administration of the pharmaceutical composition of the present disclosure, and the term "treatment" refers to all actions that improve or beneficially change the symptoms of the above disease by the administration of the pharmaceutical composition of the present disclosure.

In a specific embodiment of the present disclosure, the treatment may achieve the reduction or alleviation of signs of multiple sclerosis, a reduction of the severity of the disease, a delay or slowing of the disease, palliation or stabilization of the disease condition, and other advantageous results, but is not limited thereto.

As used herein, the term "subject" refers to any animal comprising monkeys, cows, horses, sheep, pigs, chickens, turkeys, quails, cats, dogs, mice, rats, rabbits, or guinea pigs in addition to humans, which have developed or may develop multiple sclerosis, as a subject suspected of having multiple sclerosis, and the subject disease may be effectively prevented or treated by administering the pharmaceutical composition of the present disclosure to the subject. In addition, the pharmaceutical composition of the present disclosure can exhibit synergistic effects when administered in combination with a conventional therapeutic agent.

As used herein, the term "administration" refers to providing a predetermined substance to a patient by any suitable method, and the administration route of the composition of the present disclosure may be achieved through any general route as long as it can reach target tissues. The composition may be administered through intraperitoneal administration, intravenous administration, intramuscular administration, subcutaneous administration, intradermal administration, oral administration, topical administration, intranasal administration, intrapulmonary administration, or rectal administration, but the administration route is not limited thereto. Additionally, the pharmaceutical composition of the present disclosure may be administered by any device capable of delivering an active substance to a target cell. Preferred administration routes and formulations include intravenous injection drugs, subcutaneous injection drugs, intradermal injection drugs, intramuscular injection drugs, dropwise injection drugs, *etc.* The injection drugs may be prepared using an aqueous solvent such as a physiological saline solution, Ringer's solution, *etc.,* or a non-aqueous solvent such as vegetable oils, higher fatty acid esters (*e*.*g*., ethyl oleate, *etc.),* and alcohols (*e*.*g*., ethanol, benzyl alcohol, propylene glycol, glycerin, *etc.),* and may include a pharmaceutical carrier such as a stabilizer for preventing denaturation (*e*.*g*., ascorbic acid, sodium bisulfite, sodium pyrosulfite, BHA, tocopherol, EDTA, *etc.),* an emulsifier, a buffer for pH control, a preservative for inhibiting microbial growth (e.g., phenylmercuric nitrate, thiomersal, benzalkonium chloride, phenol, cresol, benzyl alcohol, *etc.).*

For example, the composition of the present disclosure may further comprise a pharmaceutically acceptable carrier, a diluent, or an excipient, may be formulated and used in various forms including oral formulations such as powders, granules, tablets, capsules, suspensions, emulsions, syrups, aerosols, *etc.,* and injection drugs of sterile injection solutions, *etc.* according to a general method for each purpose of use, and may be administered orally, or through various routes including intravenous, intraperitoneal, subcutaneous, rectal, topical administrations, *etc.* Examples of the suitable carrier, excipient, or diluent that can be included in the composition may include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propyl hydroxybenzoate, talc, magnesium stearate, and mineral oil. Further, the composition of the present disclosure may further include a filler, an anti-aggregating agent, a lubricant, a wetting agent, a flavoring agent, an emulsifier, a preservative, *etc.*

Solid preparations for oral administration include tablets, pills, powders, granules, capsules, *etc.,* and such a solid preparation is formulated by mixing one or more excipients, such as starch, calcium carbonate, sucrose, lactose, gelatin, *etc.* with the composition. Further, in addition to a simple excipient, a lubricant such as magnesium stearate or talc may be used.

Liquid preparations for oral administration include a suspension, a solution for internal use, an emulsion, a syrup, *etc.,* and may include various excipients, such as a wetting agent, a sweetening agent, a fragrance, a preservative, *etc.,* in addition to water and liquid paraffin, which are commonly used as a simple diluent.

Preparations for parenteral administration include an aqueous solvent, a non-aqueous solvent, a suspension agent, an emulsifier, a lyophilized preparation, and a suppository, which are sterilized. As the non-aqueous solvent or the suspension agent, propylene glycol, polyethylene glycol, vegetable oil such as olive oil, injectable ester such as ethyl oleate, *etc.,* may be used. As a base of the suppository, witepsol, macrogol, twin 61, cacao butter, laurin butter, glycerogelatin, *etc.* may be used. Meanwhile, injection drugs may include conventional additives such as a solubilizing agent, an isotonic agent, a suspension agent, an emulsifier, a stabilizing agent, a preservative, *etc.*

The formulation may be prepared by a conventional mixing, granulating, or coating method, and may contain an active ingredient in an amount of about 0.1 wt % to 75 wt %, preferably about 0.1 wt % to 50 wt %. The unit formulation for a mammal weighing about 50 kg to 70 kg contains about 10 mg to 200 mg of an active ingredient.

In particular, the composition of the present disclosure is administered in a pharmaceutically effective amount. As used herein, the term "pharmaceutically effective amount" refers to an amount sufficient to treat a disease at a reasonable benefit/risk ratio applicable to medical treatment and does not cause side effects, whereas the level of the effective amount may be determined depending on the patient's health status, type of disease, severity, activity of the drug, sensitivity to the drug, administration method, administration time, administration route, excretion rate, treatment period, and factors including drugs used in combination or concurrently, and other factors well known in the medical field. The composition of the present disclosure may be administered as an individual therapeutic agent or administered in combination with other therapeutic agents, may be administered sequentially or simultaneously with a conventional therapeutic agent, and may be administered in a single dose or multiple doses. It is important to administer an amount that can achieve the maximum effect with a minimum amount without adverse effects in consideration of all of the above factors, which may be easily determined by those skilled in the art.

For example, since the administration dose may increase or decrease depending on administration route, disease severity, sex, weight, age, *etc.,* the administration dose does not limit the scope of the present disclosure in any way.

The preferred administration dose of the compound of the present disclosure varies depending on the condition and weight of a patient, the degree of disease, the form of drug, and the route and duration of administration, but may be appropriately selected by those skilled in the art. However, for a desired effect, the compound of the present disclosure may be administered in an amount of 0.0001 mg/kg to 100 mg/kg (body weight), preferably, 0.001 mg/kg to 100 mg/kg (body weight) per day. The compound may be administered once a day or in a divided dose through an oral or parenteral route.

The composition for preventing or treating multiple sclerosis of the present disclosure may be prepared in a pharmaceutical formulation by any conventional method known in the art to provide rapid, sustained or delayed release of the active ingredient after the administration thereof to a mammal. In the preparation of the formulation, the active ingredient may be mixed or diluted with a carrier, or encapsulated in a carrier in the form of a container.

Accordingly, the pharmaceutical composition of the present disclosure may further comprise a pharmaceutically acceptable carrier, a diluent, or an excipient, and may be formulated and used in various forms including oral formulations such as powders, granules, tablets, capsules, suspensions, emulsions, syrups, aerosols, *etc.,* and injection drugs of sterile injection solutions, *etc.* according to a general method for each purpose of use, and may be administered orally, or through various routes including intravenous, intraperitoneal, subcutaneous, rectal, topical administrations, *etc.* Examples of the suitable carrier, excipient, or diluent that can be included in the composition may include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propyl hydroxybenzoate, talc, magnesium stearate, and mineral oil. Further, the composition of the present disclosure may further include a filler, an anti-aggregating agent, a lubricant, a wetting agent, a flavoring agent, an emulsifier, a preservative, *etc.*

Solid preparations for oral administration include tablets, pills, powders, granules, capsules, *etc.,* and such a solid preparation is formulated by mixing one or more excipients, such as starch, calcium carbonate, sucrose, lactose, gelatin, *etc.* with the composition. Further, in addition to a simple excipient, a lubricant such as magnesium stearate or talc may be used.

Liquid preparations for oral administration include a suspension, a solution for internal use, an emulsion, a syrup, *etc.,* and may include various excipients, such as a wetting agent, a sweetening agent, a fragrance, a preservative, *etc.,* in addition to water and liquid paraffin, which are commonly used as a simple diluent.

Preparations for parenteral administration include an aqueous solvent, a non-aqueous solvent, a suspension agent, an emulsifier, a lyophilized preparation, and a suppository, which are sterilized. As the non-aqueous solvent or the suspension agent, propylene glycol, polyethylene glycol, vegetable oil such as olive oil, injectable ester such as ethyl oleate, *etc.,* may be used. As a base of the suppository, witepsol, macrogol, twin 61, cacao butter, laurin butter, glycerogelatin, *etc.* may be used. Meanwhile, injection drugs may include conventional additives such as a solubilizing agent, an isotonic agent, a suspension agent, an emulsifier, a stabilizing agent, a preservative, *etc.*

The formulation may be prepared by a conventional mixing, granulating, or coating method, and may contain an active ingredient in an amount of about 0.1 wt % to 75 wt %, preferably about 0.1 wt % to 50 wt %. The unit formulation for a mammal weighing about 50 kg to 70 kg contains about 10 mg to 200 mg of an active ingredient.

In particular, the composition of the present disclosure is administered in a pharmaceutically effective amount. As used herein, the term "pharmaceutically effective amount" refers to an amount sufficient to treat a disease at a reasonable benefit/risk ratio applicable to medical treatment and does not cause side effects, whereas the level of the effective amount may be determined depending on the patient's health status, type of disease, severity, activity of the drug, sensitivity to the drug, administration method, administration time, administration route, excretion rate, treatment period, and factors including drugs used in combination or concurrently, and other factors well known in the medical field.

The preferred administration dose of the compound of the present disclosure varies depending on the condition and weight of a patient, the degree of disease, the form of drug, and the route and duration of administration, but may be appropriately selected by those skilled in the art. However, for a desired effect, the compound of the present disclosure may be administered in an amount of 0.0001 mg/kg to 100 mg/kg (body weight), preferably, 0.001 mg/kg to 100 mg/kg (body weight) per day. The compound may be administered once a day or in a divided dose through an oral or parenteral route.

For example, since the administration dose may increase or decrease depending on administration route, disease severity, sex, weight, age, *etc.,* the administration dose does not limit the scope of the present disclosure in any way.

For the prevention or treatment of multiple sclerosis, the pharmaceutical composition of the present disclosure may be used alone or in combination with surgical operation, hormone therapy, drug therapy, and a method of using a biological response regulator. For the purpose of the present disclosure, the pharmaceutical composition according to the present disclosure may be administered in combination with one or more additional drugs which are conventionally used in the treatment of multiple sclerosis.

When the pharmaceutical composition according to the present disclosure is administered for the purpose of treating multiple sclerosis, the pharmaceutical composition may be administered in combination with one or more components and/or drugs effective in the treatment or prevention of multiple sclerosis. In particular, it is important to administer an amount that can achieve the maximum effect with a minimum amount without adverse effects in consideration of all of the above factors, which may be easily determined by those skilled in the art.

Specifically, the compound of the present disclosure or a pharmaceutically acceptable salt thereof in combination with the one or more additional drugs may be each administered concurrently, sequentially, or in reverse order. Additionally, these may be administered in a combination of suitable effective amounts, or may be administered concurrently, sequentially, or in reverse order after storage in separate containers.

A fifth aspect of the present disclosure provides a food composition for preventing or alleviating multiple sclerosis, comprising the compound of the first aspect or a food-acceptable salt thereof, as an active ingredient.

The "multiple sclerosis" and "prevention" are as described above.

Further, the term "food-acceptable salt" may be defined in the same manner as the pharmaceutically acceptable salt.

As used herein, the term "alleviation" refers to all actions to improve or beneficially change the symptoms of a subject suspected of having or suffering from a subject disease by using a composition including the compound of Formula 1 or a food-acceptable salt thereof.

The food composition according to the present disclosure may include a formulation such as pills, powders, granules, infusions, tablets, capsules, or liquids, etc. Foods to which the composition of the present disclosure can be added may include, for example, various kinds of foods such as beverages, chewing gums, teas, vitamin complexes, health supplement food, *etc.*

Essential ingredients that can be included in the food composition of the present disclosure may include any other ingredients without limitation, in addition to the compound of Formula 1 or a food-acceptable salt thereof, and may contain various herbal medicine extracts, food supplement additives, or natural carbohydrates, *etc.* as additional ingredients, like common foods.

Additionally, the food supplement additive may include a conventional food supplement additive used in the art, for example, flavoring agents, savoring agents, coloring agents, fillers, stabilizers, *etc.*

Examples of the natural carbohydrate may include common saccharides such as monosaccharides (*e*.*g*., glucose, fructose, *etc*.); disaccharides (*e*.*g*., maltose, sucrose, *etc*.); and polysaccharides (*e*.*g*., dextrin, cyclodextrin, *etc.),* and sugar alcohols such as xylitol, sorbitol, erythritol, *etc.* In addition to those described above, as flavoring agents, natural flavoring agents (*e*.*g*., rebaudioside A, glycyrrhizine, *etc.)* and synthetic flavoring agents (saccharin, aspartame, *etc.)* may be effectively used.

Additionally, the food composition of the present disclosure may contain various nutritional supplements, vitamins, minerals (electrolytes), savoring agents including synthetic savoring agents, natural savoring agents, *etc.,* coloring agents, and fillers (cheese, chocolate, *etc.),* pectic acid and salts thereof, alginic acid and salts thereof, organic acids, protective colloidal thickeners, pH adjusting agents, stabilizers, preservatives, glycerin, alcohols, carbonating agents used in carbonated drinks, *etc.* In addition, the food composition of the present disclosure may contain natural fruit juices and fruit flesh for the preparation of fruit juices and vegetable drinks. These ingredients may be used independently or in combination.

In the present disclosure, the food composition includes health functional foods and health supplement foods, *etc.*

The health functional food, which is the same term as food for special health use (FoSHU), is a food prepared and/or processed using raw materials or ingredients with functional properties useful to the human body, and refers to a food having high medicinal and medical effects, which is processed to efficiently exhibit biologically modulating functions as well as to supply nutrients. In particular, the term "functional" refers to obtaining a useful effect for health purposes such as regulating nutrients or physiological action for the structure and function of the human body. As described above, foods including ingredients useful to the human body, of which function and safety have been recognized by the Ministry of Food and Drug Safety, are called health functional foods. Foods that have not been approved by the Ministry of Food and Drug Safety but are considered to have beneficial effects on the human body are classified as health (supplement) foods. The food of the present disclosure may be prepared by methods commonly used in the art, and may be prepared by adding raw materials and ingredients commonly added in the art during preparation. In addition, the food may also be prepared in any formulation without limitation, as long as the formulation is recognized as a food. The food composition of the present disclosure may be prepared in various types of formulations. Unlike general drugs, the food composition uses a food as a raw material, and thus has an advantage in that there are no side effects, *etc.* which may be caused by long-term intake of a drug, and has excellent portability.

A sixth aspect of the present disclosure provides a feed composition for preventing or alleviating multiple sclerosis, comprising the compound of the first aspect or a feed-acceptable salt thereof, as an active ingredient.

The "multiple sclerosis", "prevention", and "alleviation" are as described above.

Further, the term "feed-acceptable salt" may be defined in the same manner as the pharmaceutically acceptable salt.

As used herein, the term "feed" refers to any appropriate natural or artificial diet, single meal, *etc.* for animals to eat, ingest, and digest, or an element of the single meal.

The type of the feed is not particularly limited, but any feed conventionally used in the technical field of known art can be used. Non-limiting examples of the feed include vegetable feeds, such as grains, nuts, food processed by-products, millet, fibers, pharmaceutical by-products, fats, starches, gourds, or grain by-products, *etc*.; and animal feeds, such as proteins, inorganic substances, fats, minerals, single-cell proteins, animal planktons, or food, *etc*. These may be used alone or in combination of two or more thereof.

The feed of the present disclosure may include a powder feed, a solid feed, a moist pellet feed, a dry pellet feed, an extruder pellet (EP) feed, a raw feed, *etc.,* but is not limited thereto.

The feed composition of the present disclosure may include binders, emulsifiers, preservatives, *etc.* which can be added to prevent quality deterioration. The feed composition may include a feed additive. The feed additive may include amino acids, vitamins, enzymes, flavoring agents, non-protein nitrogen compounds, silicates, buffers, extracts, oligosaccharides, *etc.* which are added to a feed to increase utility. In addition, the feed additive may further include a feed mixture(s), *etc.,* but is not limited thereto.

### [Mode for Carrying Out the Invention]

Hereinafter, the present disclosure will be described in more detail through exemplary embodiments. However, these exemplary embodiments are for illustrative purposes only and are not intended to limit the scope of the present disclosure.

### Example 1: Preparation of 2-(ring 1)-5-phenyl substituted-1,3-selenazole derivatives (Compounds 1 to 38)

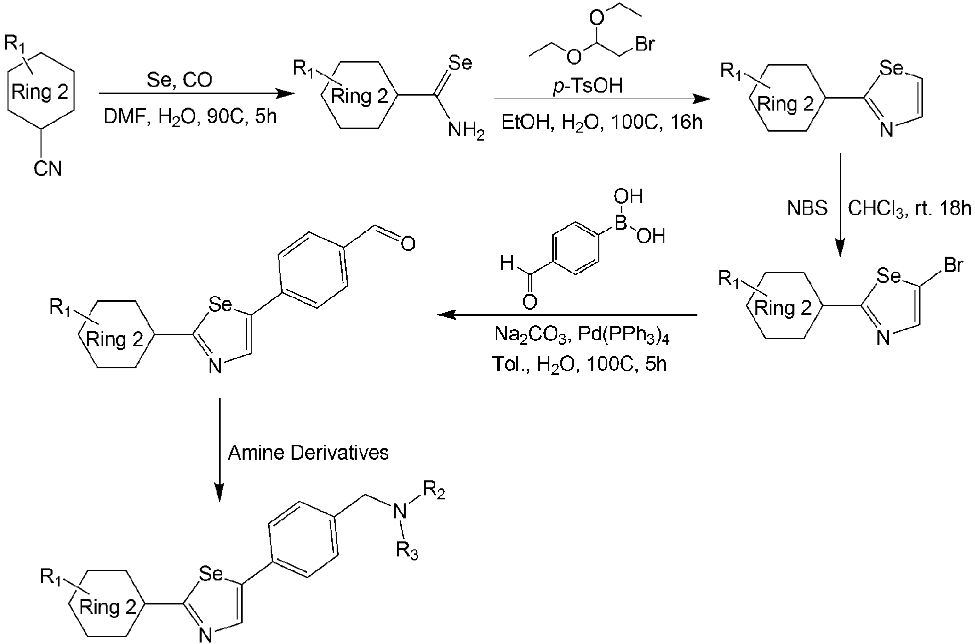

### Example 2: Preparation of 5-(ring 1)-2-phenyl substituted-1,3-selenazole derivatives (Compounds 39 to 42)

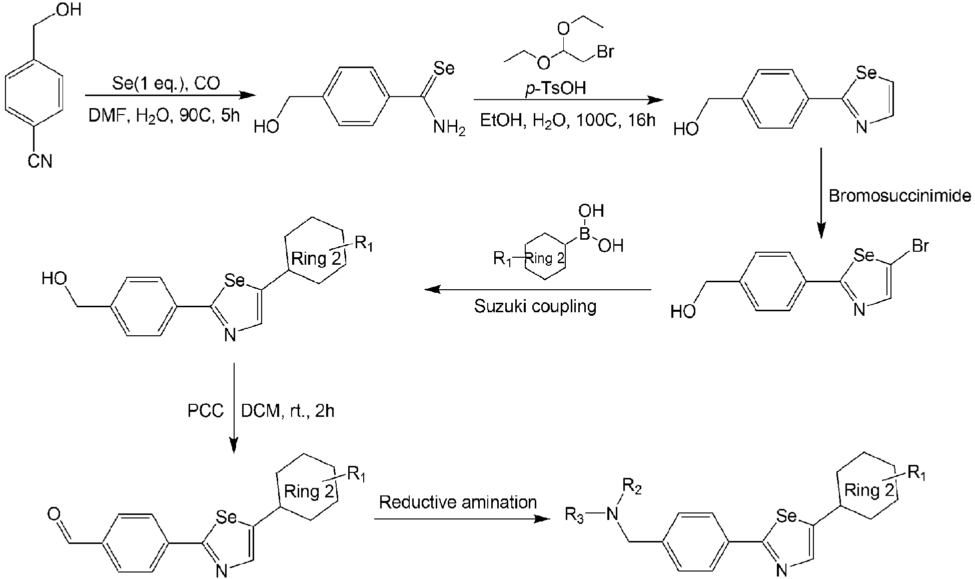

### Example 3: Preparation of 5-(ring 1)-2-phenyl substituted-4,5-dihydroisoxazole derivatives (Compounds 43 to 71)

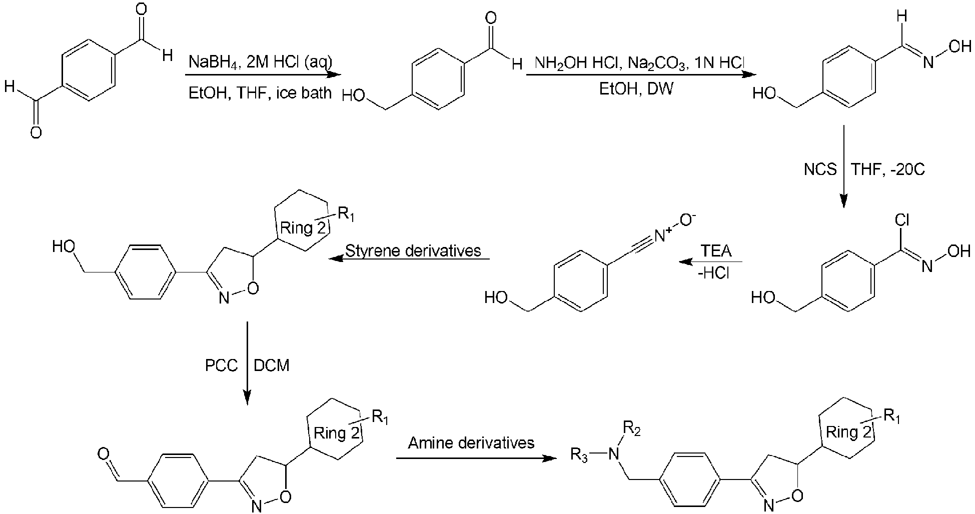

Compounds 1 to 71 were synthesized according to the Reaction Schemes of Examples 1 to 3 above. The information on names, structural formulas, ¹H NMR, *etc.* of the obtained compounds are summarized below.

### Compound 1. Methyl 1-(4-(2-(4-tert-butylphenyl)-1,3-selenazole-5-yl)benzyl)azetidine-3-carboxylate

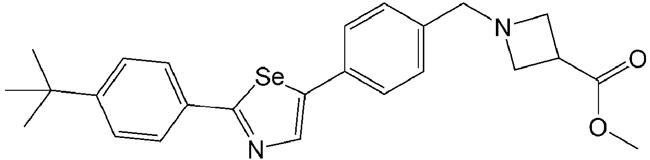

Yellow solid;
Yield: 43%;
¹H NMR (CDCl₃, 400 MHz) *δ* 7.94 (s, selenazole ring-**H**), 7.81-7.83 (m, 2 Ar**H**), 7.43-7.50 (m, 4 Ar**H**), 7.26-7.30 (m, 2 Ar**H**), 3.71 (s, COOC**H**₃), 3.62 (s, NC**H**₂), 3.52-3.55 (m, 2 azetidine ring-**H**), 3.33-3.35 (m, 3 azetidine ring-**H**), 1.35 (s, Ar(C**H**₃)₃);
¹³C NMR (CDCl₃, 100 MHz) *δ* 173.92 (**NC**Se), 173.54 (**C**(O)), 153.67, 145.40, 139.57, 137.87, 133.77, 132.68, 129.16, 127.35, 126.59, 125.99 (ArC), 62.98 (N**C**H₂), 56.84 (azetidine ring-C), 51.97 (COO**C**H₃), 34.93 (**C**(CH₃)₃), 33.97 (azetidine ring-**C**), 31.17 (C(**C**H₃)₃).

### Compound 2. Sodium 1-(4-(2-(4-tert-butylphenyl)-1,3-selenazole-5-yl)benzyl)azetidine-3-carboxylate

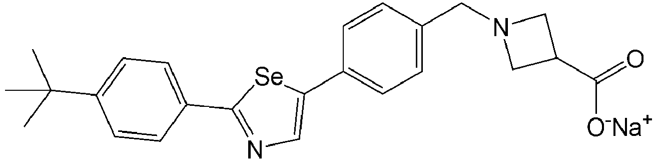

Whild solid;
Yield: 82%;
¹H NMR (CD₃OD, 400 MHz) *δ* 8.14 (s, selenazole ring-**H**), 7.88 (d, *J* = 8.4 Hz, 2 Ar**H**), 7.78 (d, *J* = 8.2 Hz, 2 Ar**H**), 7.55-7.57 (m, 4 ArH), 4.45 (s, NC**H**₂), 4.32-4.33 (m, 4 azetidine ring-**H**), 3.59-3.67 (m, 1 azetidine ring-**H**), 1.39 (s, Ar(C**H**₃)₃);
¹³C NMR (CD₃OD, 100 MHz) *δ* 175.45 (NCSe), 174.18 (**C**(O)), 154.20, 144.41, 139.97, 135.08, 133.22, 130.53, 130.03, 127.62, 126.30, 125.86 (Ar**C**), 57.73 (N**C**H₂), 56.40 (azetidine ring-**C**), 34.43 (**C**(CH₃)₃), 33.81 (azetidine ring-**C**), 30.09 (C(**C**H₃)₃).

### Compound 3. Ethyl 1-(4-(2-(4-tert-butylphenyl)-1,3-selenazole-5-yl)benzyl)azetidine-3-carboxylate

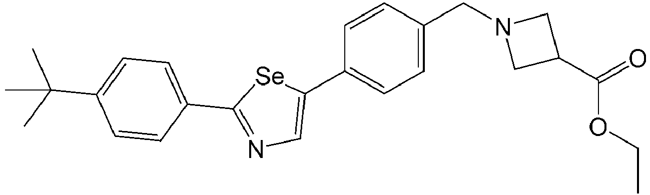

Yellow solid;
Yield: 25%;
¹H NMR (CDCl₃, 400 MHz) *δ* 7.93 (s, selenazole ring-**H**), 7.82 (d, *J* = 8.5 Hz, 2 Ar**H**), 7.49 (d, *J* = 8.1 Hz, 2 Ar**H**), 7.44 (d, *J* = 8.5 Hz, 2 Ar**H**), 7.29 (d, *J* = 8.2 Hz, 2Ar**H**), 4.15 (q, 7.1 Hz, C**H**₂CH₃), 3.63 (s, NC**H**₂), 3.55 (br s, 2 azetidine ring-**H**), 3.32-3.33 (m, 3 azetidine ring-**H**), 1.34 (s, Ar(C**H**₃)₃), 1.26 (t, *J* = 7.1 Hz, CH₂C**H**₃);
¹³C NMR (CDCl₃, 100 MHz) *δ* 173.93 (NCSe), 173.11 (**C**(O)), 153.68, 145.40, 139.57, 137.79, 133.77, 132.68, 129.17, 127.35, 126.58, 125.99 (Ar**C**), 62.98 (N**C**H₂), 60.82 (**C**H₂CH₃), 56.84 (azetidine ring-**C**), 34.93, 34.11, 31.17 (**C**(CH₃)₃), 14.19 (CH₂**C**H₃).

### Compound 4. Tert-butyl 1-(4-(2-(4-tert-butylphenyl)-1,3-selenazole-5-yl)benzyl)azetidine-3-carboxylate

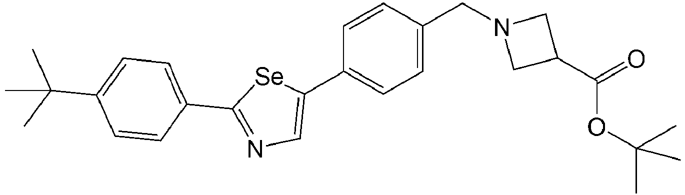

Ivory solid;
Yield: 75%;
¹H NMR (CDCl₃, 400 MHz) *δ* 7.96 (s, selenazole ring-**H**), 7.85 (d, *J* = 8.3 Hz, 2 Ar**H**), 7.46-7.53 (m, 4 Ar**H**), 7.32 (d, *J* = 8.0 Hz, 2 Ar**H**), 3.66 (s, NC**H**₂), 3.54-3.59 (m, 2 azetidine ring-**H**), 3.27-3.33 (m, 3 azetidine ring-**H**), 1.47 (s, COOC(C**H**₃)₃), 1.37 (s, Ar(C**H**₃)₃);
¹³C NMR (CDCl₃, 100 MHz) *δ* 173.92 (**NC**Se), 172.41 (**C**(O)), 153.66, 145.40, 139.56, 137.81, 133.77, 132.68, 129.21, 127.34, 126.59, 125.99 (Ar**C**), 80.81, 62.90 (N**C**H₂), 56.78 (azetidine ring-**C**), 34.99, 34.92 (azetidine ring-**C**), 31.17 (COOC(C**H**₃)₃), 29.71 (COO**C**(CH₃)₃), 1.37 (s, Ar(**C**H₃)₃).

### Compound 5. Methyl 1-(4-(2-(3-isocyano-4-isopropoxyphenyl)-1,3-selenazole-5-yl)benzyl)azetidine-3-carboxylate

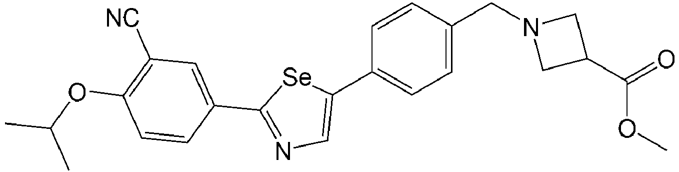

Yellow oil;
Yield: 72%;
¹H NMR (CDCl₃, 400 MHz) *δ* 8.08 (s, selenazole ring-**H**), 8.07-8.02 (m, 1 Ar**H**), 7.92 (s, 1 Ar**H**), 7.49 (d, *J* = 8.1 Hz, 2 Ar**H**), 7.33 (d, *J* = 7.8 Hz, 2 Ar**H**), 7.01 (d, *J* = 8.9 Hz, 1 Ar**H**), 4.72 (sept, *J* = 6.0 Hz, OC**H**(CH₃)₂), 3.73 (s, COOC**H**₃), 3.66 (br s, NC**H**₂), 3.58 (br s, 2 azetidine ring-**H**), 3.37 (br s, 3 azetidine ring-**H**), 1.60 (d, *J* = 5.9 Hz, OCH(C**H**₃)₂ );
¹³C NMR (CDCl₃, 100 MHz) *δ* 173.30 (**NC**Se), 170.86 (**C**(O)), 161.01, 146.21, 139.69, 137.40, 132.52, 132.28, 132.21, 129.42, 129.35, 127.49, 15.91, 113.79, 103.63 (Ar**C**), 72.39, 62.58 (N**C**H₂), 56.69 (azetidine ring-**C**), 52.11 (O**C**H₃), 33.83 (azetidine ring-**C**), 21.82 (OCH(**C**H₃)₂).

### Compound 6. Sodium 1-(4-(2-(3-isocyano-4-isopropoxyphenyl)-1,3-selenazole-5-yl)benzyl)azetidine-3-carboxylate

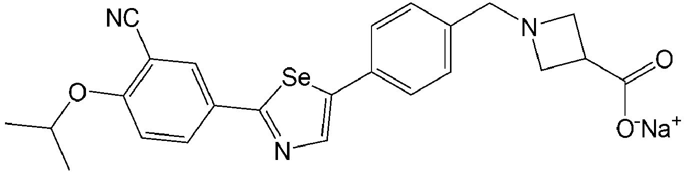

Yellow solid;
Yield: 97%;
¹H NMR (CD₃OD, 400 MHz) *δ* 8.10-8.06 (m, 3 Ar**H**), 7.70 (d, *J* = 8.1 Hz, 2 Ar**H**), 7.55 (d, *J* = 8.2 Hz, 2 Ar**H**), 7.25 (d, *J* =8.8 Hz, 1 Ar**H**), 4.82-4.88 (m, OC**H**(CH₃)₂), 4.46 (s, 2 NC**H**₂), 4.28-4.39 (m, 4 azetidine ring-**H**), 3.63-3.71 (m, 1 azetidine ring-**H**), 1.42 (d, *J* = 6.0 Hz, OCH(C**H**₃)₂);
¹³C NMR (CD₃OD, 100 MHz) *δ* 172.90 (**NC**Se), 172.10 (**C**(O)), 161.19, 145.13, 140.20, 134.87, 132.72, 131.47, 130.72, 129.83, 128.88, 127.63 (Ar**C**), 115.31, 114.08, 102.82, 72.28 (O**C**H(CH₃)₂), 57.53 (N**C**H₂), 55.71 (azetidine ring-**C**), 32.92 (azetidine ring-**C**), 20.70 (OCH(**C**H₃)₂).

### Compound 7. Ethyl 1-(4-(2-(3-cyano-4-isopropoxyphenyl)-1,3-selenazole-5-yl)benzyl)azetidine-3-carboxylate

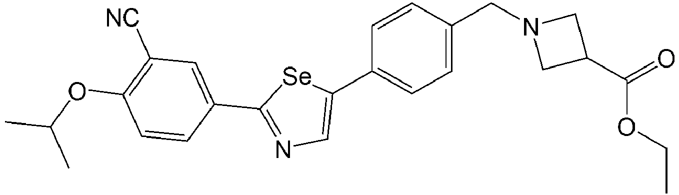

Ivory oil;
Yield: 70%;
¹H NMR (CDCl₃, 400 MHz) *δ* 8.01-8.07 (m, 2 Ar**H**), 7.92 (s, selenazole ring **H**), 7.52 (d, *J* = 8.4 Hz, 2 Ar**H**), 7.43 (d, *J* = 8.0 Hz, 2 Ar**H**), 7.01 (d, *J =* 8.7 Hz, 1 Ar**H**), 4.71-4.74 (m, OC**H**(CH₃)₂), 4.19 (q, *J* = 7.2 Hz, COOC**H**₂CH₃), 3.87-3.91 (m, 2 azetidine ring, NC**H**₂), 3.46-3.57 (m, 3 azetidine ring **H**), 1.42 (d, *J* = 6.0 Hz, OCH(C**H**₃)₂), 1.27 (t, *J* = 7.2 Hz, COOCH₂C**H**₃);
¹³C NMR (CDCl₃, 100 MHz) *δ* 172.23 (**NCS**e), 171.30 (**C**(O)), 161.06, 155.96, 145.83, 139.84, 132.29, 132.25, 129.88, 129.35, 127.70, 115.86, 113.75, 103.66, 72.40 (COO**C**H₂CH₃), 61.42 (N**C**H₂), 55.95 (azetidine ring-C), 33.45 (azetidine ring-C), 21.81 (OCH(**C**H₃)₂), 14.14 (COOCH₂**C**H₃).

### Compound 8. Tert-butyl 1-(4-(2-(3-cyano-4-isopropoxyphenyl)-1,3-selenazole-5-yl)benzyl)azetidine-3-carboxylate

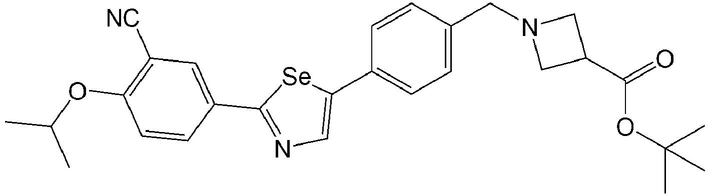

Ivory oil;
Yield: 70%;
¹H NMR (CDCl₃, 400 MHz) *δ* 8.04-8.10 (m, 2 Ar**H**), 7.94 (s, selenazole ring **H**), 7.53 (d, *J* = 8.0 Hz, 2 Ar**H**), 7.39 (d, *J* = 7.5 Hz, 2 Ar**H**), 7.04 (d, *J* = 8.9 Hz, 1 Ar**H**), 4.72-4.78 (m, OC**H**(CH₃)₂), 3.57-3.85 (m, 2 azetidine ring, NC**H**₂), 3.23-3.33 (m, 3 azetidine ring **H**), 1.46-1.48 (m, C(C**H**₃)₃, OCH(C**H**₃)₂);
¹³C NMR (CDCl₃, 100 MHz) *δ* 172.90 (**NC**Se), 171.58 (**C**(O)), 140.04, 132.24, 129.39, 128.42, 127.48, 120.18, 119.45, 115.94, 113.81, 103.62 (Ar**C**), 72.42, 62.79, 56.95, 34.75, 28.06 (OCH(**C**H₃)₂, 21.83 (C(**C**H₃)₃).

### Compound 9. Methyl 1-(4-(2-(4-isopropoxy-3-(trifluoromethyl)phenyl)-1,3-selenazole-5-yl)benzyl)azetidine-3-carboxylate

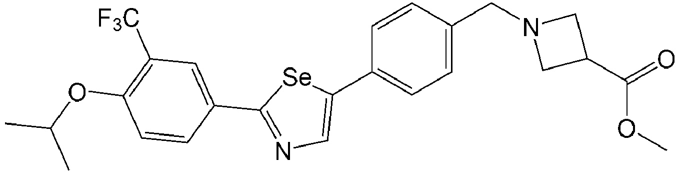

Yellow solid;
Yield: 68%;
¹H NMR (CDCl₃, 400 MHz) *δ* 8.10-8.12 (m, 1 Ar**H**), 8.01 (d, *J* = 8.6 Hz, 1 Ar**H**), 7.94 (s, selenazole ring-**H**), 7.51 (d, *J* = 8.2 Hz, 2 ArH), 7.33 (d, *J* = 8.2 Hz, 2 Ar**H**), 7.06 (d, *J* = 8.7 Hz, Ar**H**), 4.71-4.77 (m, OC**H**(CH₃)₂), 3.81 (s, COOC**H**₃), 3.74 (s, NC**H**₂), 3.58-3.66 (m, 2 azetidine ring **H**), 3.55-3.57 (m, 3 azetidine ring-**H**), 1.42 (d, *J* = 6.04 Hz, OCH(C**H**₃)₂);
¹³C NMR (CDCl₃, 100 MHz) *δ* 173.53 (**NC**Se), 172.00 (**C**(O)), 157.53, 145.80, 139.54, 138.10, 132.46, 131.27, 129.19, 128.56, 127.37, 125.95, 125.88, 125.07 (Ar**C**), 121.46, 121.05, 120.64, 120.23 (q, *J*_{C-F} = 41 Hz), 114.39, (Ar**C**), 62.98 (N**C**H₂), 56.88, 51.97, 33.99 (azetidine ring-**C**), 21.83 (OCH(**C**H₃)₂).

### Compound 10. 1-(4-(2-(4-isopropoxy-3-(trifluoromethyl)phenyl)-1,3-selenazole-5-yl)benzyl)azetidine-3-carboxylic acid

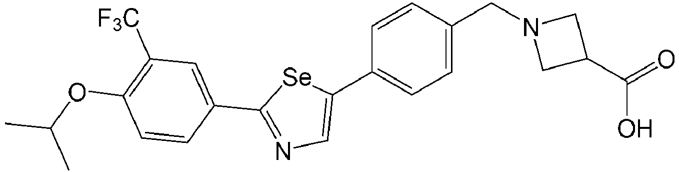

Yellow solid;
Yield: 59%;
¹H NMR (CD₃OD, 400 MHz) *δ* 8.05 (d, *J* = 2.04, 1 Ar**H**), 8.01 (s, selenazole ring-**H**), 7.94 (dd, *J* = 2.0, 8.7 Hz, 1 Ar**H**), 7.63 (d, *J* = 8.1 Hz, 2 Ar**H**), 7.54 (d, *J* = 8.0 Hz, 2 Ar**H**), 7.19 (d, *J* = 8.8 Hz, 1 Ar**H**), 4.74-4.97 (m, OC**H**(CH₃)₂), 4.43 (s, 2 NC**H**₂), 4.23-4.35 (m, 4 azetidine ring-**H**), 3.54-3.63 (m, 1 azetidine ring-**H**), 1.35 (d, *J* = 6.0 Hz, OCH(C**H**₃)₂);
¹³C NMR (CD₃OD, 100 MHz) *δ* 174.53 (**NC**Se), 173.00 (**C**(O)), 157.67, 144.74, 140.06, 134.73, 131.98, 130.72, 130.03, 127.88, 127.49, 124.66, 124.60 (Ar **C**), 127.38, 124.55, 121.98, 119.27 (q, *J*_{C-F} = 283.0 Hz), 120.12, 119.81, 119.50, 119.20(q, *J*_{C-F} = 30.0 Hz), 114.58 (Ar**C**), 71.54 (O**C**H(CH₃)₂), 57.41 (N**C**H₂), 56.03 (azetidine ring-**C**), 33.79 (azetidine ring-**C**), 20.73 (OCH(**C**H₃)₂).

### Compound 11. Ethyl 1-(4-(2-(4-isopropoxy-3-(trifluoromethyl)phenyl)-1,3-selenazole-5-yl)benzyl)azetidine-3-carboxylate

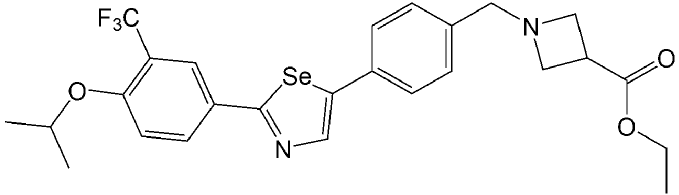

Ivory oil;
Yield: 70%;
¹H NMR (CDCl₃, 400 MHz) *δ* 8.11 (d, *J* = 2.0 Hz, 1 Ar**H**), 8.01 (dd, *J* = 2.0, 8.6 Hz, 1 Ar**H**), 7.95 (s, selenazole ring **H),** 7.53 (d, *J* = 8.1 Hz, 2 Ar**H**), 7.38 (d, *J* = 8.0 Hz, 2 Ar**H**), 7.07 (d, *J* = 8.8 Hz, 1 Ar**H**), 4.71-4.77 (m, OC**H**(CH₃)₂), 4.21 (q, *J* = 7.1 Hz, COOC**H**₂CH₃), 3.56-3.88 (m, 2 azetidine ring, NC**H**₂), 3.41-3.47 (m, 3 azetidine ring **H**), 1.43 (d, *J* = 6.0 Hz, OCH(C**H**₃)₂), 1.30 (t, *J* = 7.2 Hz, COOCH₂C**H**₃);
¹³C NMR (CDCl₃, 100 MHz) *δ* 172.78 (**NC**Se), 172.16 (**C**(O)), 162.86, 157.56, 151.32, 145.59, 143.33, 139.67, 133.91, 133.39, 132.87, 131.30, (q, *J*_{C-F} = 208.3 Hz), 129.46, 128.50, 127.48, 126.01, 125.95, 125.88, 125.84 (q, *J*_{C-F} = 18.2 Hz) 114.38 (Ar**C**), 71.73 (COO**C**H₂CH₃), 61.06 (N**C**H₂), 56.51 (azetidine ring-**C**), 33.86 (azetidine ring-**C**), 21.83 (OCH(**C**H₃)₂), 14.18 (COOCH₂**C**H₃).

### Compound 12. Methyl 1-(4-(2-(3-chloro-4-isopropoxyphenyl)-1,3-selenazole-5-yl)benzyl)azetidine-3-carboxylate

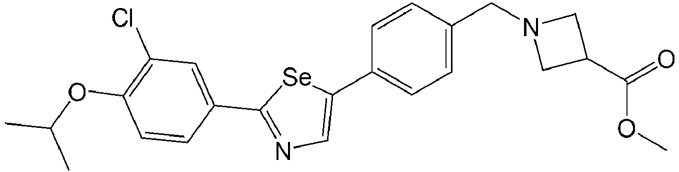

Yellow solid;
Yield: 47%;
¹H NMR (CD₃OD, 400 MHz) *δ* 8.00 (s, selenazole ring-**H**), 7.95 (d, *J* = 2.2 Hz, Ar**H**), 7.78 (dd, *J* = 2.2, 8.6 Hz, 2 Ar**H**), 7.58-7.60 (m, 2 Ar**H**), 7.35 (d, *J* = 8.2 Hz, 2ArH), 7.17 (d, *J* = 8.8 Hz, 1 Ar**H**), 4.73-4.79 (m, OC**H**(CH₃)₂), 3.77 (s, COOC**H**₃), 3.73 (s, NC**H**₂), 3.53-3.66 (m, 2 azetidine ring **H**), 3.33-3.44 (m, 3 azetidine ring-**H**), 1.40 (d, *J* = 6.0 Hz, OCH(C**H**₃)₂);
¹³C NMR (CD₃OD, 100 MHz) *δ* 173.42 (NCSe), 172.50 (C(O)), 155.32, 145.66, 139.01, 137.36, 132.48, 129.40, 129.35, 127.70, 126.90, 126.44, 124.08, 114.92 (ArC), 71.69 (O**C**H(CH₃)₂), 62.12 (N**C**H₂), 56.11, 51.12, 33.54 (azetidine ring-**C**), 20.81 (OCH(**C**H₃)₂).

### Compound 13. Sodium 1-(4-(2-(3-chloro-4-isopropoxyphenyl)-1,3-selenazole-5-yl)benzyl)azetidine-3-carboxylate

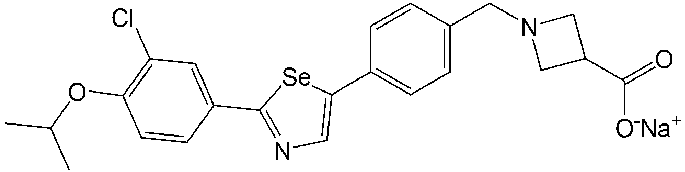

Whild solid;
Yield: 92%;
¹H NMR (CD₃OD, 400 MHz) *δ* 8.22 (s, selenazole ring-**H**), 7.96 (d, *J* = 2.1 Hz, 1 Ar**H**), 7.83 (dd, *J* = 2.1, 8.6 Hz, 1 ArH), 7.63-7.67 (m, 2 Ar**H**), 7.41-7.46 (m, 2 ArH), 7.29 (d, *J* = 8.8 Hz, 1 ArH), 4.76-4.82 (m, OC**H**(CH₃)₂), 3.98 (s, NC**H**₂), 3.77-3.83 (m, 2 azetidine ring **H**), 3.65-3.68 (m, 2 azetidine ring-**H**), 3.34-3.38 (m, azetidine ring-**H**), 1.34 (d, *J* = 6.0 Hz, OCH(C**H**₃)₂);
¹³C NMR (CD₃OD, 100 MHz) *δ* 173.49 (**NC**Se), 171.46 (**C**(O)), 155.11, 145.48, 140.86, 133.19, 133.15, 130.53, 129.61, 127.93, 127.61, 127.44, 123.51, 116.01 (Ar**C**), 71.94 (O**C**H(CH₃)₂), 59.69, 55.88, 33.35 (azetidine ring-**C**), 22.19 (OCH(**C**H₃)₂).

### Compound 14. Methyl 1-(4-(2-(3-chloro-4-ethoxyphenyl)-1,3-selenazole-5-yl)benzyl)azetidine-3-carboxylate

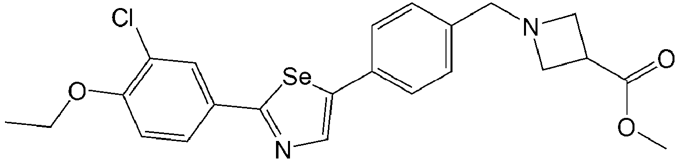

Yellow solid;
Yield: 55%;
¹H NMR (CDCl₃, 400 MHz) *δ* 7.95 (d, *J* = 1.7 Hz, 1 Ar**H**), 7.92 (s, selenazole ring **H**), 7.49 (d, *J* = 8.0 Hz, 1 Ar**H**), 7.49 (d, *J* = 8.0 Hz, 1 Ar**H**), 7.31 (d, *J* = 7.6 Hz, 2 Ar**H**), 6.95 (d, *J* = 8.6 Hz, 1 Ar**H**), 4.17 (q, *J* = 6.9 Hz, OC**H**₂CH₃), 3.73 (s, COOC**H**₃), 3.65 (s, NC**H**₂), 3.56-3.57 (m, 2 azetidine ring **H**), 3.35-3.36 (m, 3 azetidine ring **H**), 1.51 (t, *J* = 6.8 Hz, OCH₂C**H**₃);
¹³C NMR (CDCl₃, 100 MHz) *δ* 173.52 (**NC**Se), 171.97 (**C**(O)), 155.97, 145.63, 139.48, 137.94, 132.51, 129.93, 129.19, 128.46, 127.34, 126.34, 123.49, 113.06 (ArC), 64.92 (O**C**H₂CH₃), 62.94 (N**C**H₂), 56.84 (azetidine ring-C), 52.00, 33.97 (azetidine ring-**C**), 14.64 (OCH₂**C**H₃).

### Compound 15. Sodium 1-(4-(2-(3-chloro-4-ethoxyphenyl)-1,3-selenazole-5-yl)benzyl)azetidine-3-carboxylate

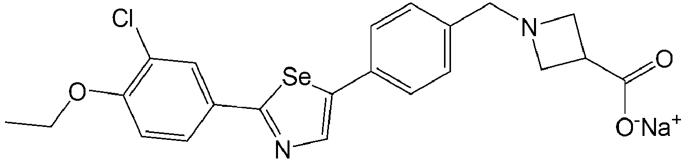

Whild solid;
Yield: 90%;
¹H NMR (CD₃OD, 400 MHz) *δ* 8.01 (s, selenazole ring-**H**), 7.96 (d, *J* = 2.2 Hz, 1 Ar**H**), 7.80 (dd, J = 2.2, 8.6 Hz, 1 Ar**H**), 7.56 (d, *J* = 8.1 Hz, 2 Ar**H**), 7.37 (d, *J* = 8.2 Hz, 2 Ar**H**), 7.15 (d, *J* = 8.7 Hz, 1 ArH), 4.19 (q, *J* = 6.9 Hz, OC**H**₂CH₃), 3.73 (s, NC**H**₂), 3.59-3.67 (m, 2 azetidine ring **H**), 3.36-3.40 (m, 2 azetidine ring-**H**), 3.21-3.32 (m, 1 azetidine ring-**H**), 1.48 (t, *J* = 7.0 Hz, OCH₂C**H**₃);
¹³C NMR (CD₃OD, 100 MHz) *δ* 179.46 (NCSe), 172.45 (C(O)), 156.19, 145.74, 138.95, 137.61, 132.37, 129.44, 127.47, 126.85, 126.59, 123.06, 113.16 (ArC), 64.66, 62.30 (O**C**H₂CH₃), 57.61, 36.51 (azetidine ring-**C**), 13.54 (OCH₂**C**H₃).

### Compound 16. Methyl 1-(4-(2-(4-methoxyphenyl)-1,3-selenazole-5-yl)benzyl)azetidine-3-carboxylate

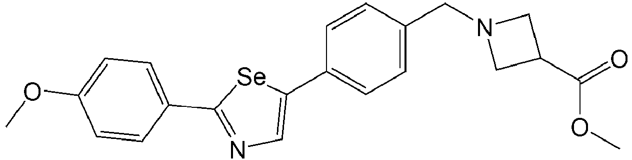

Yellow solid;
Yield: 55%;
¹H NMR (CDCl₃, 400 MHz) *δ* 7.93 (s, selenazole ring-**H**), 7.86 (dd, *J* = 2.0, 6.8 Hz, 2 Ar**H**), 7.51 (d, *J* = 8.1 Hz, 2 Ar**H**), 7.32 (d, *J* = 8.1 Hz, 2 Ar**H**), 6.97 (dd, *J* = 6.88 Hz, 2 Ar**H**), 3.89 (s, OC**H**₃), 3.74 (s, COOC**H**₃), 3.65 (s, NC**H**₂), 3.55-3.60 (m, 2 azetidine ring **H**), 3.33-3.41 (m, 3 azetidine ring-**H**);
¹³C NMR (CDCl₃, 100 MHz) *δ* 173.60 (**NC**Se), 173.54 (**C**(O)), 161.33, 144.83, 139.40, 137.81, 132.72, 129.49, 129.13, 128.30, 127.29, 114.37 (Ar**C**), 63.00 (N**C**H₂), 56.86 (azetidine ring-**C**), 55.44(O**C**H₃), 51.98 (azetidine ring-**C**), 33.98 (azetidine ring-**C**).

### Compound 17. 1-(4-(2-(4-methoxyphenyl)-1,3-selenazole-5-yl)benzyl)azetidine-3-carboxylic acid

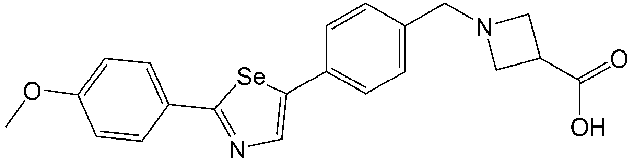

Ivory solid;
Yield: 78%;
¹H NMR (CD₃OD, 400 MHz) *δ* 8.08 (s, selenazole ring- **H**), 7.89 (dd, *J* = 1.9, 6.8 Hz, 2 Ar**H**), 7.75 (d, *J* = 8.2 Hz, 2 Ar**H**), 7.53 (d, *J* = 8.2 Hz, 1 Ar**H**), 7.03-7.06 (m, 2 Ar**H**), 4.75 (s, 2 NC**H**₂), 4.26-4.28 (m, 4 azetidine ring-**H**), 3.89 (s, OC**H**₃), 3.50-3.59 (m, 1 azetidine ring-**H**);
¹³C NMR (CD₃OD, 100 MHz) *δ* 161.65, 140.91, 134.20, 131.42, 130.40, 129.23, 127.77, 127.32, 126.60, 115.21, 56.22, 54.61, 46.58, 29.06.

### Compound 18. Methyl 1-(4-(2-(4-isopropoxyphenyl)-1,3-selenazole-5-yl)benzyl)azetidine-3-carboxylate

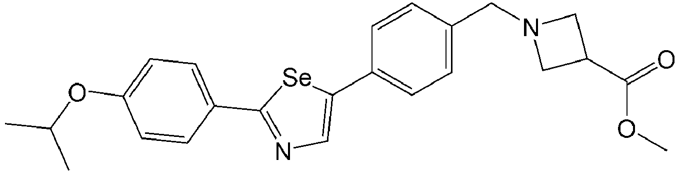

Yellow solid;
Yield: 31%;
¹H NMR (CDCl₃, 400 MHz) *δ* 7.91 (s, selenazole ring-**H**), 7.83 (d, *J* = 8.7 Hz, 2 Ar**H**), 7.49 (d, *J* = 8.1 Hz, 2 Ar**H**), 7.30 (d, *J* = 8.0 Hz, 2 Ar**H**), 6.93 (d, *J* = 8.7 Hz, 1 Ar**H**), 4.62 (sept, *J* = 6.0 Hz, OC**H**(CH₃)₂), 3.72 (s, COOC**H**₃), 3.63 (s, NC**H**₂), 3.55-3.56 (m, 2 azetidine ring-**H**), 3.35-3.39 (m, 3 azetidine ring-**H**), 1.37 (d, *J* = 6.0 Hz, OCH(C**H**₃)₂);
¹³C NMR (CDCl₃, 100 MHz) *δ* 173.65 (NCSe), 173.53 (C(O)), 159.74, 144.67, 139.38, 137.79, 132.72, 129.12, 128.31, 115.97 (Ar**C**), 70.04, 62.99 (N**C**H₂), 56.86 (azetidine ring-**C**), 51.96 (O**C**H₃), 33.98 (azetidine ring-**C**), 21.99 (OCH(**C**H₃)₂).

### Compound 19. 1-(4-(2-(4-isopropoxyphenyl)-1,3-selenazole-5-yl)benzyl)azetidine-3-carboxylic acid

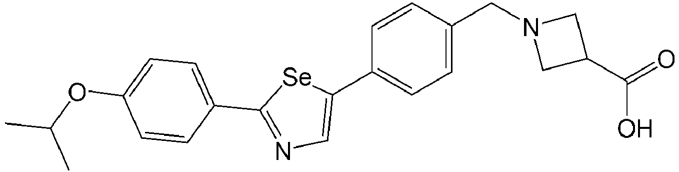

Yellow solid;
Yield: 94%;
¹H NMR (CD₃OD, 400 MHz) *δ* 8.07 (s, selenazole ring-**H**), 7.86 (d, *J* = 8.8 Hz, 2 Ar**H**), 7.45 (d, *J* = 8.2 Hz, 2 Ar**H**), 7.57 (d, *J* = 8.2 Hz, 2 Ar**H**), 7.01 (d, *J* = 8.84 Hz, 2 Ar**H**), 4.69-4.75 (m, OC**H**(CH₃)₂), 4.48 (s, 2 NC**H**₂), 4.33-4.42 (m, 4 azetidine ring-**H**), 3.73-3.79 (m, 1 azetidine ring-**H**), 1.37 (d, *J* = 6.0 Hz, OCH(C**H**₃)₂);
¹³C NMR (DMSO-*d*₆, 100 MHz) *δ* 173.34 (**NC**Se), 172.92 (**C**(O)), 159.97, 144.21, 140.97, 133.83, 130.88, 128.72, 127.62, 116.49 (Ar**C**), 72.28, 55.46 (N**C**H₂), 33.00 (azetidine ring-C), 20.21 (OCH(**C**H₃)₂).

### Compound 20. Ethyl 1-(4-(2-(4-isopropoxyphenyl)-1,3-selenazole-5-yl)benzyl)azetidine-3-carboxylate

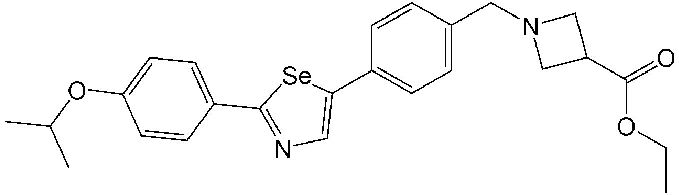

Ivory oil;
Yield: 70%;
¹H NMR (CDCl₃, 400 MHz) *δ* 7.92 (s, selenazole ring **H),** 7.84 (d, *J* = 8.7 Hz, 2 Ar**H**), 7.51 (d, *J* = 8.1 Hz, 2 Ar**H**), 7.34 (d, *J* = 8.0 Hz, 2 Ar**H**), 6.94 (d, *J* = 8.8 Hz, 2 Ar**H**), 4.64 (sept, *J* = 6.0 Hz, OC**H**(CH₃)₂), 4.20 (q, *J* = 7.1 Hz, COOC**H**₂CH₃), 3.84 (s, NC**H**₂), 3.63-3.69 (m, 2 azetidine ring **H),** 3.37-3.39 (m, 3 azetidine ring **H),** 1.39 (d, *J* = 6.0 Hz, OCH(C**H**₃)₂), 1.27 (t, *J* = 7.1 Hz, COOCH₂C**H**₃);
¹³C NMR (CDCl₃, 100 MHz) *δ* 173.78 (**NC**Se), 173.04 (**C**(O)), 159.78, 144.60, 139.45, 129.24, 129.14, 128.33, 127.34, 116.01 (Ar**C**), 70.09 (COO**C**H₂CH₃), 60.91 (N**C**H₂), 56.72 (azetidine ring-**C**), 34.02 (azetidine ring-**C**), 21.99 (OCH(**C**H₃)₂), 14.19 (COOCH₂**C**H₃).

### Compound 21. Methyl 1-(4-(2-(3-isopropoxyphenyl)-1,3-selenazole-5-yl)benzyl)azetidine-3-carboxylate

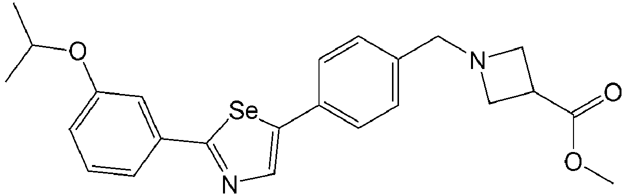

Yellow solid;
Yield: 56%;
¹H NMR (CDCl₃, 400 MHz) *δ* 7.97 (s, selenazole ring-**H**), 7.53-4.48 (m, 3 Ar**H**), 7.45 (d, *J* = 7.68 Hz, 1 Ar**H**), 7.32-7.36 (m, 3 Ar**H**), 6.97-7.00 (m, 1 Ar**H**), 4.65-4.71 (m, C**H**(CH₃)₂), 3.74 (s, COOC**H**₃), 3.65 (s, NC**H**₂), 3.57 (br s, 2 azetidine ring-**H**), 3.36-3.38 (m, 3 azetidine ring-**H**), 1.40 (d, *J* = 6.0 Hz, CH(C**H**₃)₂);
¹³C NMR (CDCl₃, 100 MHz) *δ* 173.76 (NCSe), 173.54 (**C**(O)), 158.39, 146.01, 139.55, 138.07, 137.67, 132.56, 130.06, 129.15, 127.38, 119.44, 118.10, 113.39 (Ar**C**), 70.15, 63.00 (N**C**H₂), 56.88 (azetidine ring-**C**), 51.97, 33.99 (azetidine ring-**C**), 22.05 (CH(**C**H₃)₂).

### Compound 22. 1-(4-(2-(3-isopropoxyphenyl)-1,3-selenazole-5-yl)benzyl)azetidine-3-carboxylic acid

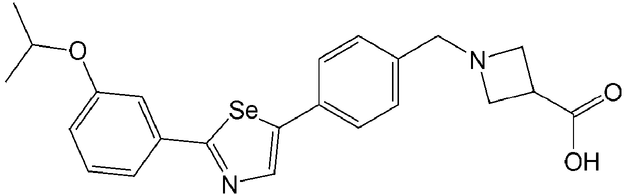

Yellow solid;
Yield: 25%;
¹H NMR (CD₃OD, 400 MHz) *δ* 8.14 (s, selenazole ring-**H**), 7.77 (d, *J* = 8.2 Hz, 2 Ar**H**), 7.56 (d, *J* = 8.2 Hz, 2 Ar**H**), 7.47 (d, *J* = 8.5 Hz, 2 Ar**H**), 7.38 (t, *J* = 7.8 Hz, 1 Ar**H**), 7.05-7.07 (m, Ar**H**), 4.68-4.74 (m, OC**H**(CH₃)₂), 4.46 (s, 2 NC**H**₂), 4.31-4.39 (m, 4 azetidine ring-**H**), 3.68-3.74 (m, 1 azetidine ring-**H**), 1.37 (d, *J* = 6.0 Hz, OCH(C**H**₃)₂);
¹³C NMR (CD₃OD, 100 MHz) *δ* 175.25 (NCSe), 172.14 (C(O)), 158.55, 144.93, 140.06, 137.08, 135.11, 130.66, 130.03, 129.65, 127.69, 127.19, 119.14, 117.98, 113.08 (ArC), 69.91, 57.60, 55.56, 32.52 (azetidine ring-**C**), 20.21 (OCH(**C**H₃)₂).

### Compound 23. Methyl 1-(4-(2-(2-isopropoxyphenyl)-1,3-selenazole-5-yl)benzyl)azetidine-3-carboxylate

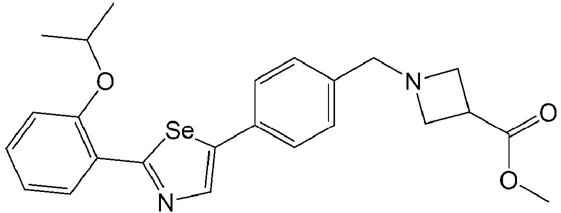

Transparent oil;
Yield: 33%;
¹H NMR (CDCl₃, 400 MHz) *δ* 8.44 (dd, *J* = 1.6, 7.8 Hz, 2 Ar**H**), 8.08 (s, selenazole ring-**H**), 7.56 (d, *J* = 8.1 Hz, 2 Ar**H**), 7.37-7.41 (m, Ar**H**), 7.32 (d, *J* = 8.1 Hz, 2 Ar**H**), 7.04-7.09 (m, 2 ArH), 4.85-4.95 (m, OC**H**(CH₃)₂), 3.73 (s, COOC**H**₃), 3.65 (s, NC**H**₂), 3.56-3.57 (m, 2 azetidine ring **H**), 3.35-3.39 (m, 3 azetidine ring-**H**), 1.56 (d, *J* = 6.0 Hz, CH(C**H**₃)₂);
¹³C NMR (CDCl₃, 100 MHz) *δ* 173.59 (**NC**Se), 165.86 (**C**(O)), 154.28, 145.55, 141.24, 138.18, 137.43, 133.34, 130.44, 129.11, 127.30, 125.55, 120.71, 113.06 (Ar**C**), 71.63, 63.08 (N**C**H₂), 56.86 (azetidine ring-**C**), 51.98, 34.01 (azetidine ring-**C**), 22.36 (CH(**C**H₃)₂).

### Compound 24. 1-(4-(2-(2-isopropoxyphenyl)-1,3-selenazole-5-yl)benzyl)azetidine-3-carboxylic acid

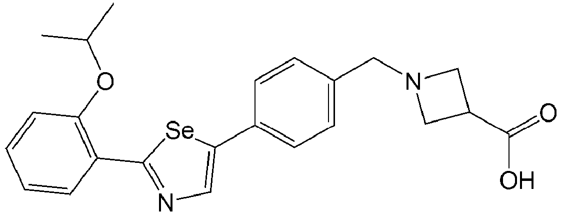

Yellow solid;
Yield: 98%;
¹H NMR (CD₃OD, 400 MHz) *δ* 8.34 (dd, *J* = 1.6, 7.9 Hz, 2 Ar**H**), 8.19 (s, selenazole ring-**H**), 7.78 (d, *J* = 8.2 Hz, 2 Ar**H**), 7.55 (d, *J* = 8.2 Hz, 2 Ar**H**), 7.49 (m, 1 Ar**H**), 7.25 (d, *J* = 8.3 Hz, 1 Ar**H**), 7.09 (t, *J* = 7.24 Hz, 1 Ar**H**), 5.01-5.07 (m, OC**H**(CH₃)₂), 4.45 (s, 2 NC**H**₂), 4.32-4.37 (m, 4 azetidine ring-**H**), 3.63-3.69 (m, 1 azetidine ring-**H**), 1.57 (d, *J* = 6.0 Hz, OCH(C**H**₃)₂);
¹³C NMR (CD₃OD, 100 MHz) *δ* 172.84 (**NC**Se), 167.14 (**C**(O)), 154.52, 144.27, 138.45, 135.86, 131.00, 130.55, 129.23, 127.54, 126.57, 124.71, 120.39, 113.32 (ArC), 71.79, 57.74, 55.86, 32.97 (azetidine ring-**C**), 21.18 (OCH(**C**H₃)₂).

### Compound 25. Ethyl 1-(4-(2-(2-isopropoxyphenyl)-1,3-selenazole-5-yl)benzyl)azetidine-3-carboxylate

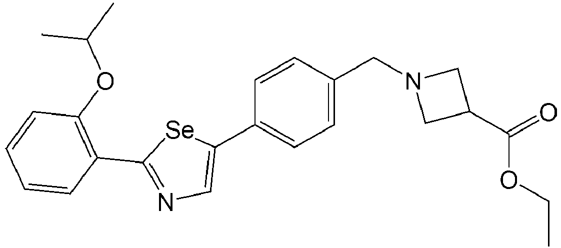

Ivory oil;
Yield: 70%;
¹H NMR (CDCl₃, 400 MHz) *δ* 8.43 (dd, *J* = 1.4, 7.7 Hz, 1 Ar**H**), 8.09 (s, selenazole ring **H),** 7.58-7.60 (m, 2 Ar**H**), 7.38-7.44 (m, 2 Ar**H**), 7.05-7.10 (m, 1 Ar**H**), 4.87-4.96 (m, OC**H**(CH₃)₂), 4.23 (q, *J* = 7.1 Hz, COOC**H**₂CH₃), 3.75-3.89 (m, NC**H**₂), 3.57-3.68 (m, 3 azetidine ring **H),** 3.43-3.49 (m, 1 azetidine ring **H),** 1.57 (d, *J* = 6.0 Hz, OCH(C**H**₃)₂), 1.31 (t, *J* = 7.1 Hz, COOCH₂C**H**₃);
¹³C NMR (CDCl₃, 100 MHz) *δ* 180.76 (**NC**Se), 168.76 (**C**(O)), 165.98, 161.15, 154.31, 138.36, 130.49, 129.45, 127.46, 127.32, 125.53, 120.73, 113.06 (Ar**C**), 71.67 (COO**C**H₂CH₃), 62.21, 61.11 (N**C**H₂), 56.35 (azetidine ring-**C**), 33.79 (azetidine ring-**C**), 29.56, 22.36 (OCH(**C**H₃)₂), 14.17 (COOCH₂**C**H₃).

### Compound 26. Methyl 1-(4-(2-(pyridine-2-yl)-1,3-selenazole-5-yl)benzyl)azetidine-3-carboxylate

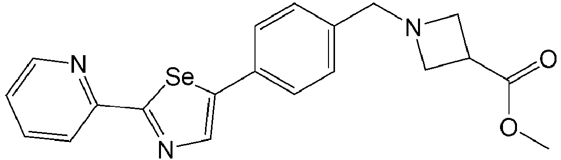

Whild solid;
Yield: 50%;
¹H NMR (CDCl₃, 400 MHz) *δ* 8.61-8.63 (m, 1 Ar**H**), 8.14 (d, *J* = 7.6 Hz, 1 Ar**H**), 8.07 (s, 1 Ar**H**), 7.80 (t, *J* = 7.3 Hz, 1 Ar**H**), 7.56 (d, *J* = 7.6 Hz, 2 Ar**H**), 7.36 (d, *J* = 8.0 Hz, 3 ArH), 3.72-3.74 (m, COOC**H**₃, NC**H**₂), 3.66 (br s, 2 azetidine ring-**H**), 3.42 (br s, 3 azetidine ring-**H**);
¹³C NMR (CDCl₃, 100 MHz) *δ* 175.79, 173.27, 153.37, 149.68, 148.06, 140.46, 136.97, 133.27, 129.34, 127.51, 124.51, 118.43 (ArC), 62.51 (N**C**H₂), 56.60 (azetidine ring-C), 52.07 (COO**C**H₃), 33.79 (azetidine ring-**C**).

### Compound 27. Sodium 1-(4-(2-(pyridine-2-yl)-1,3-selenazole-5-yl)benzyl)azetidine-3-carboxylate

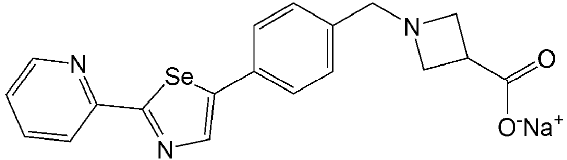

Whild solid;
Yield: 54%;
¹H NMR (CD₃OD, 400 MHz) *δ* 8.58 (d, *J* = 4.4 Hz, 1 Ar**H**), 8.12-8.16 (m, 2 Ar**H**), 7.90-7.94 (m, 1 Ar**H**), 7.64 (d, *J* = 8.2 Hz, 2 ArH), 7.46-7.47 (m, 1 Ar**H**), 7.45-7.38 (m, 2 Ar**H**), 3.68 (s, NC**H**₂), 3.56-3.60 (m, 2 azetidine ring-**H**), 3.37-3.41 (m, 2 azetidine ring-**H**), 3.21-3.25 (m, 1 Ar**H**);
¹³C NMR (CD₃OD, 100 MHz) *δ* 179.57, 179.08, 175.60, 152.85, 149.57, 148.22, 139.96, 137.82 ,137.28, 132.61, 129.48, 126.97, 124.80, 118.06 (Ar**C**), 62.31 (N**C**H₂), 57.59 (azetidine ring-**C**), 36.50 (azetidine ring-**C**).

### Compound 28. Methyl 1-(4-(2-(pyridine-3-yl)-1,3-selenazole-5-yl)benzyl)azetidine-3-carboxylate

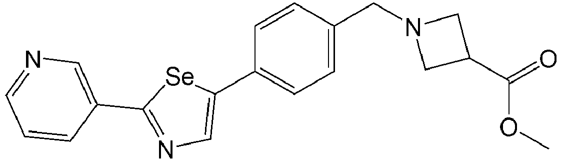

Whild solid;
Yield: 50%;
¹H NMR (CD₃OD, 400 MHz) *δ* 9.10 (s, pyridine-**H**), 8.62-8.64 (m, 1 Ar**H**), 8.32-8.35 (m, 1 Ar**H**), 8.14 (s, 1 Ar**H**), 7.63 (d, *J* = 8.1 Hz, 2 Ar**H**), 7.54-5.57 (m, 1 Ar**H**), 7.38 (d, *J* = 8.1 Hz, 2 Ar**H**), 3.73 (s, COOC**H**₃), 3.69 (s, NC**H**₂), 3.56-3.59 (m, 2 azetidine ring-**H**), 3.34-3.38 (m, 3 azetidine ring-**H**);
¹³C NMR (CD₃OD, 100 MHz) *δ* 174.78, 171.12, 151.35, 149.04, 147.94, 141.13, 139.04, 135.84, 134.00, 136.61, 130.81, 128.52, 125.74 (Ar**C**), 63.42 (N**C**H₂), 57.52 (azetidine ring-**C**), 52.53 (COO**C**H₃), 34.93 (azetidine ring-**C**).

### Compound 29. Sodium 1-(4-(2-(pyridine-3-yl)-1,3-selenazole-5-yl)benzyl)azetidine-3-carboxylate

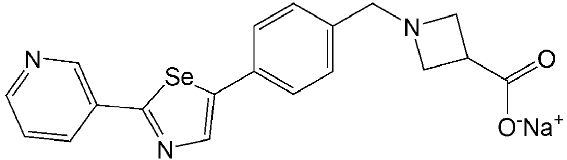

Whild solid;
Yield: 95%;
¹H NMR (CD₃OD, 400 MHz) *δ* 9.09 (s, pyridine-**H**), 8.61-8.62 (m, 1 Ar**H**), 8.31-8.34 (m, 1 Ar**H**), 8.13 (s, 1 Ar**H**), 7.62 (d, *J* = 8.1 Hz, 2 Ar**H**), 7.53-7.56 (m, 1 Ar**H**), 7.37 (d, *J* = 8.1 Hz, 2 Ar**H**), 3.65 (s, NC**H**₂), 3.52-3.56 (m, 2 azetidine ring-**H**), 3.33-3.37 (m, 2 azetidine ring-**H**), 3.16-3.22 (m, 1 Ar**H**);
¹³C NMR (CD₃OD, 100 MHz) *δ* 179.62, 179.19, 169.88, 149.97, 147.84, 146.43, 139.62, 137.98, 134.60, 132.57, 132.03, 129.57, 127.10, 124.47 (Ar**C**), 62.21 (N**C**H₂), 57.54 (azetidine ring-**C**), 36.46 (azetidine ring-**C**).

### Compound 30. Methyl 1-(4-(2-(4-tert-butylphenyl)-1,3-selenazole-5-yl)benzyl)pyrrolidine-3-carboxylate

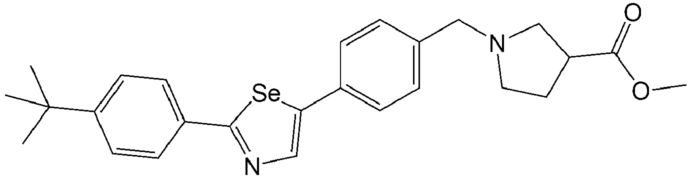

Yellow solid;
Yield: 52%;
¹H NMR (CDCl₃, 400 MHz) *δ* 7.97 (s, selenazole ring-**H**), 7.87 (d, *J* = 8.4 Hz, 2 ArH), 7.47-7.54 (m, 4 Ar**H**), 7.37 (d, *J* = 8.0 Hz, 2 Ar**H**), 3.71 (s, COOC**H**₃), 3.66 (s, NC**H**₂), 3.04-3.10 (m, 1 pyrrolidine ring-**H**), 2.90-2.94 (m, 1 pyrrolidine ring-**H**), 2.67-2.76 (m, 2 pyrrolidine ring-**H**), 2.55-2.59 (m, 1 pyrrolidien ring-**H**), 2.11-2.17 (m, 2 pyrrolidine ring-**H**), 1.38 (s, Ar(C**H**₃)₃);
¹³C NMR (CDCl₃, 100 MHz) *δ* 175.47 (**NC**Se), 173.85 (**C**(O)), 153.65, 145.51, 139.53, 139.19, 133.53, 129.42,127.26, 126.60, 126.00 (Ar**C**), 59.64 (N**C**H₂), 56.67, 53.76, 51.90, 41.99, 34.93, 31.19 (Ar(**C**H₃)₃), 27.69.

### Compound 31. Sodium 1-(4-(2-(4-tert-butylphenyl)-1,3-selenazole-5-yl)benzyl)pyrrolidine-3-carboxylate

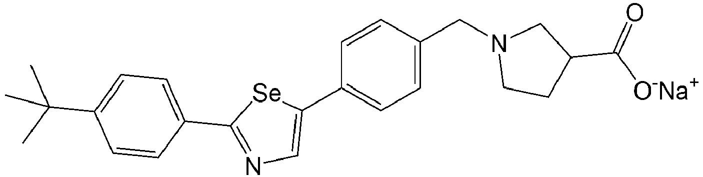

Whild solid;
Yield: 95%;
¹H NMR (CD₃OD, 400 MHz) *δ* 8.01 (s, 1 selenazole ring-**H**), 7.83 (dd, *J* = 1.6, 6.7 Hz, 2 Ar**H**), 7.58 (d, *J* = 8.2 Hz, 2 Ar**H**), 7.49-7.51 (m, 2 Ar**H**), 7.40 (d, *J* = 8.2 Hz, 2 Ar**H**), 3.65 (d, *J* = 5.2 Hz, NC**H**₂), 2.93-3.04 (m, 2 pyrrolidine ring-**H**), 2.80-2.82 (m, 1 pyrrolidine ring-**H**), 2.62-2.66 (m, pyrrolidine ring-**H**), 2.48-2.55 (m, pyrrolidine ring-**H**), 2.04-2.12 (m, 2 pyrrolidine ring-**H**), 1.36 (s, Ar(C**H**₃)₃);
¹³C NMR (CD₃OD, 100 MHz) *δ* 181.62 (N**C**Se), 174.47 (**C**(O)), 153.86, 145.71, 138.88, 13871, 133.33, 132.32, 129.87, 126.77, 126.23, 125.81 (Ar**C**), 59.64 (N**C**H₂), 57.72, 53.75, 45.16, 34.41, 30.16 (C(**C**H₃)₃), 28.24.

### Compound 32. Methyl 1-(4-(2-(4-tert-butylphenyl)-1,3-selenazole-5-yl)benzyl)piperidine-4-carboxylate

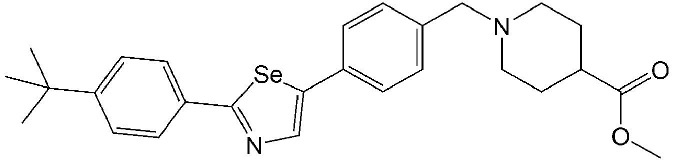

Whild solid;
Yield: 12%;
¹H NMR (CDCl₃, 400 MHz) *δ* 7.98 (s, selenazole ring-**H**), 7.86 (d, *J* = 8.4 Hz, 2 Ar**H**), 7.47-7.55 (m, 4 Ar**H**), 7.38-7.39 (m, 2 Ar**H**), 3.71 (s, COOC**H**₃), 3.55 (s, NC**H**₂), 2.89-2.91 (m, 2 piperidine ring-**H**), 2.34 (br s, 1 piperidine ring-**H**), 2.12-2.20 (m, 2 piperidine ring-**H**),1.83-1.91 (m, 4 piperidine ring-**H**), 1.60 (s, Ar(C**H**₃)₃);
¹³C NMR (CDCl₃, 100 MHz) *δ* 175.64 (N**C**Se), 173.87 (C(O)), 153.67, 145.51, 139.50, 133.77, 132.52, 129.71, 127.18, 126.58, 125.99 (ArC), 62.76 (N**C**H₂), 52.90, 51.63, 50.98, 41.02, 34.93, 31.16 (Ar(**C**H₃)₃), 28.27, 27.14.

### Compound 33. Methyl 1-(4-(2-(3-chloro-4-isopropoxyphenyl)-1,3-selenazole-5-yl)benzyl)pyrrolidine-3-carboxylate

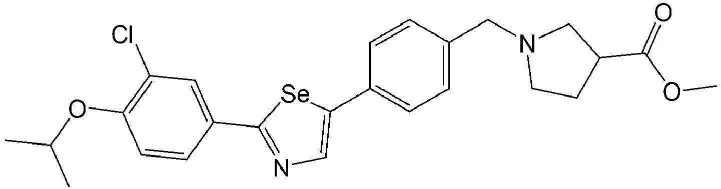

Yellow solid;
Yield: 47%;
¹H NMR (CDCl₃, 400 MHz) *δ* 7.92-7.89 (m, 1 Ar**H**), 7.88 (s, 1 Ar**H**), 7.68 (dd, *J* = 2.1, 8.6 Hz, 1 Ar**H**), 7.45 (d, *J* = 8.0 Hz, 2 Ar**H**), 7.32 (d, *J* = 8.0 Hz, Ar**H**), 6.63 (d, J = 8.6 Hz, 1 Ar**H**), 4.61 (sept, *J* = 6.0 Hz, OC**H**(CH₃)₂), 3.66 (s, COOC**H**₃), 3.61 (d, *J* = 3.6 Hz, NC**H**₂), 2.99-3.05 (m, 1 pyrrolidine ring **H**), 2.84-2.89 (m, 1 pyrrolidine ring-**H**), 2.54-2.70 (m, 2 pyrrolidine ring-**H**), 2.50-2.52 (m, 1 pyrrolidine ring-**H**), 2.06-2.12 (m, 2 pyrrolidine ring-**H**), 1.38 (d, *J* = 6.0 Hz, OCH(C**H**₃)₂);
¹³C NMR (CDCl₃, 100 MHz) *δ* 175.44 (N**C**Se), 171.86 (**C**(O)), 155.17, 145.74, 139.42, 139.28, 132.32, 129.96, 129.43, 128.58, 127.22, 126.21, 124.63,115.12 (Ar**C**), 72.11 (O**C**H(CH₃)₂), 59.60 (N**C**H₂), 56.65, 53.76, 51.89, 41.97, 27.69 (pyrrolidine ring-**C**), 21.99 (OCH(**C**H₃)₂).

### Compound 34. Sodium 1-(4-(2-(3-chloro-4-isopropoxyphenyl)-1,3-selenazole-5-yl)benzyl)pyrrolidine-3-carboxylate

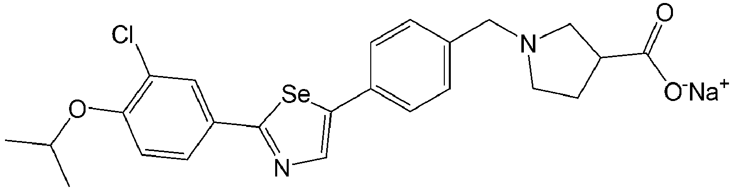

Whild solid;
Yield: 93%;
¹H NMR (CD₃OD, 400 MHz) *δ* 8.02 (s, selenazole ring-**H**), 7.97 (d, *J* = 2.2 Hz, 1 Ar**H**), 7.80 (dd, *J* = 2.2, 8.6 Hz, 1 Ar**H**), 7.60 (d, *J* = 8.1 Hz, 2 Ar**H**), 7.43 (d, *J* = 8.1 Hz, 2 Ar**H**), 7.18 (d, *J* = 8.8 Hz, 1 Ar**H**), 4.75 (sept, *J* = 6.0 Hz, OC**H**(CH₃)₂), 3.64-3.72 (m, NC**H**₂), 3.03-3.08 (m, 1 pyrrolidine ring **H**), 2.91-2.99 (m, 1 pyrrolidine ring-**H**), 2.79-2.88 (m, 1 pyrrolidine ring-**H**), 2.60-2.65 (m, 1 pyrrolidine ring-**H**), 2.50-2.54 (m, 1 pyrrolidine ring-**H**), 2.06-2.13 (m, 2 pyrrolidine ring-**H**), 1.38 (d, *J* = 6.0 Hz, OCH(C**H**₃)₂);
¹³C NMR (CD₃OD, 100 MHz) *δ* 181.67 (N**C**Se), 172.16 (**C**(O)), 155.18, 145.75, 138.93, 138.63, 132.18, 129.83, 129.56, 129.34, 127.59, 126.71, 126.55, 124.02, 114.80 (Ar**C**), 71.66 (O**C**H(CH₃)₂), 59.70 (N**C**H₂), 57.76, 53.78, 45.13, 28.30 (pyrrolidine ring-**C**), 20.97 (OCH(**C**H₃)₂).

### Compound 35. Ethyl 2-(4-(2-(3-chloro-4-isopropoxyphenyl)-1,3-selenazole-5-yl)benzylamino)acetate

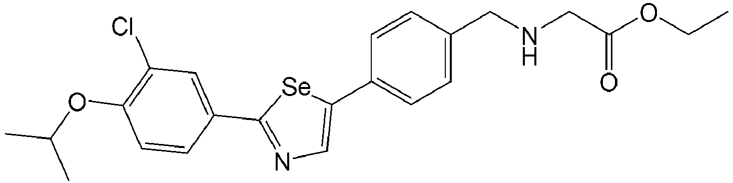

Ivory solid;
Yield: 31%;
¹H NMR (CDCl₃, 400 MHz) *δ* 7.90-7.93 (m, selenazole ring-**H**, 1 Ar**H**), 7.71 (dd, *J* = 2.2 Hz, 8.6 Hz, 2 Ar**H**), 7.49 (d, *J* = 8.1 Hz, 2 Ar**H**), 7.35 (d, *J* = 8.1 Hz, 2 Ar**H**), 6.96 (d, *J* = 8.7 Hz, 1 Ar**H**), 4.63 (sept, *J* = 6.0 Hz, OC**H**(CH₃)₂), 4.19 (q, *J* = 7.1 Hz, OC**H**₂CH₃), 3.82 (s, NC**H**₂Ar), 3.41 (s, COC**H**₂NH), 1.40 (d, *J* = 6.0 Hz, OCH(C**H**₃)₂) 1.27 (t, *J* = 7.1 Hz, OCH₂C**H**₃);
¹³C NMR (CDCl₃, 100 MHz) *δ* 172.39 (N**C**Se), 172.02 (C(O)), 155.21, 145.63, 139.85, 139.49, 132.52, 129.97, 129.00, 128.66, 127.40, 126.18, 124.67, 115.17 (Ar**C**), 72.15 (O**C**H(CH₃)₂), 60.84 (N**C**H₂Ar), 52.85, 50.06, 21.99 (OCH(**C**H₃)₂), 14.25 (OCH₂**C**H₃).

### Compound 36. Sodium 2-(4-(2-(3-chloro-4- isopropoxyphenyl)-1,3-selenazole-5-yl)benzylamino)acetate

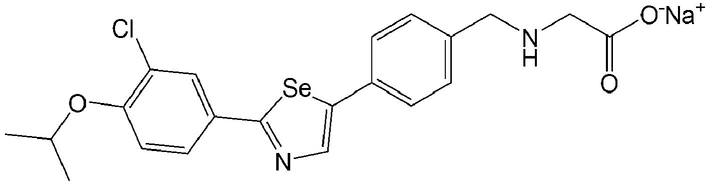

Whild solid;
Yield: 90%;
¹H NMR (CD₃OD, 400 MHz) *δ* 8.01 (s, selenazole ring-**H**), 7.96 (s, 1 Ar**H**), 7.79 (d, *J* = 7.2 Hz, 1 Ar**H**), 7.60 (d, *J* = 7.7 Hz, 2 Ar**H**), 7.43 (d, *J* = 7.8 Hz, 2 Ar**H**), 7.17 (d, *J =* 8.6 Hz, 1 Ar**H**), 4.75 (sept, *J* = 6.2 Hz, OC**H**(CH₃)₂), 3.78 (s, NC**H**₂Ar), 3.20 (s, COC**H**₂NH), 1.40 (d, *J* = 6.0 Hz, OCH(C**H**₃)₂) 1.27 (t, *J* = 7.1 Hz, OCH₂C**H**₃);
¹³C NMR (CDCl₃, 100 MHz) *δ* 177.26 (N**C**Se), 172.49 (**C**(O)), 155.28, 145.90, 139.77, 138.83, 132.11, 129.37, 129.13, 127.65, 126.90, 126.49, 124.07, 114.99 (Ar**C**), 71.77 (O**C**H(CH₃)₂), 52.37 51.92, 20.85 (OCH(**C**H₃)₂).

### Compound 37. Methyl 3-(4-(2-(3-chloro-4-isopropoxyphenyl)-1,3-selenazole-5-yl)benzylamino)propanoate

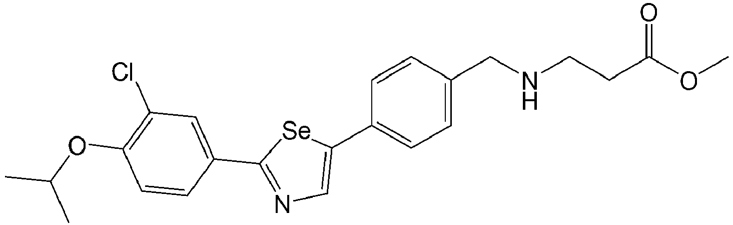

Yellow solid;
Yield: 41%;
¹H NMR (CDCl₃, 400 MHz) *δ* 7.91 (d, *J* = 2.2 Hz, 1 Ar**H**), 7.88 (s, selenazole ring-**H**), 7.69 (dd, *J* = 2.2 Hz, 8.6 Hz, 1 Ar**H**), 7.47 (d, *J* = 8.1 Hz, 2 Ar**H**), 7.33 (d, *J* = 8.1 Hz, 2 Ar**H**), 6.94 (d, *J* = 8.7 Hz, 1 Ar**H**), 4.61 (sept, *J* = 6.0 Hz, OC**H**(CH₃)₂), 3.83 (s, NC**H**₂Ar), 3.67 (s, COOC**H**₃), 2.89 (t, *J* = 6.4 Hz, NHC**H**₂CH₂COOCH₃), 2.54 (t, *J* = 6.4 Hz, NHC**H**₂CH₂COOCH₃), 1.39 (d, *J* = 6.0 Hz, OCH(C**H**₃)₂);
¹³C NMR (CDCl₃, 100 MHz) *δ* 173.18 (N**C**Se), 171.91 (**C**(O)), 155.18, 145.71, 140.40, 139.43, 132.32, 129.96, 128.81, 128.61, 127.35, 127.15, 126.20, 124.64, 115.14 (Ar**C**), 72.13 (O**C**H(CH₃)₂), 53.27 (N**C**H₂Ar), 51.66, 44.39, 34.49, 21.99 (OCH(**C**H₃)₂).

### Compound 38. Sodium 3-(4-(2-(3-chloro-4-isopropoxyphenyl)-1,3-selenazole-5-yl)benzylamino)propanoate

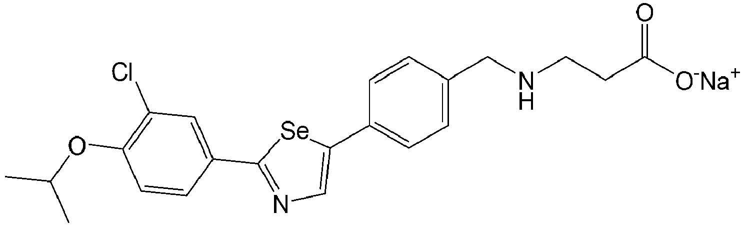

Ivory solid;
Yield: 65%;
¹H NMR (CD₃OD, 400 MHz) *δ* 8.09 (s, selenazole ring-**H**), 7.98 (d, *J* = 2.2 Hz, 1 Ar**H**), 7.83-7.81 (dd, *J* = 2.3 Hz, 8.6 Hz, 1 Ar**H**), 7.74 (d, *J* = 8.2 Hz, 2 Ar**H**), 7.55 (d, *J =* 8.1 Hz, 2 Ar**H**), 7.19 (d, *J* = 8.7 Hz, 1 Ar**H**), 4.78 (sept, *J* = 6.0 Hz, OC**H**(CH₃)₂), 4.22 (s, NC**H**₂Ar), 3.14-3.20 (m, NHC**H**₂CH₂COOCH₃), 2.52 (t, *J* = 6.4 Hz, NHCH₂C**H**₂COOH), 1.41 (d, *J* = 6.0 Hz, OCH(C**H**₃)₂);
¹³C NMR (CD₃OD, 100 MHz) *δ* 173.18 (N**C**Se), 171.91 (**C**(O)), 155.18, 145.71, 140.40, 139.43, 132.32, 129.96, 128.81, 128.61, 127.35, 127.15, 126.20, 124.64, 115.14 (Ar**C**), 72.13 (O**C**H(CH₃)₂), 53.27 (N**C**H₂Ar), 51.66, 44.39, 34.49, 21.99 (OCH(**C**H₃)₂).

### Compound 39. Methyl 1-(4-(5-(4-tert-butylphenyl)-1,3-selenazole-2-yl)benzyl)azetidine-3-carboxylate

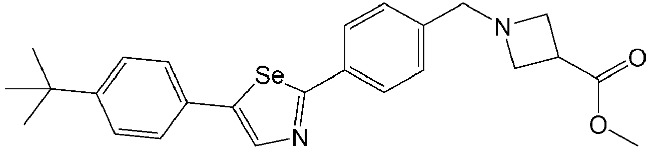

Ivory solid;
Yield: 65%;
¹H NMR (CD₃OD, 400 MHz) *δ* 8.09 (s, selenazole ring-**H**), 7.98 (d, *J* = 2.2 Hz, 1 Ar**H**), 7.83-7.81 (dd, *J* = 2.3 Hz, 8.6 Hz, 1 Ar**H**), 7.74 (d, *J* = 8.2 Hz, 2 Ar**H**), 7.55 (d, *J =* 8.1 Hz, 2 Ar**H**), 7.19 (d, *J* = 8.7 Hz, 1 Ar**H**), 4.78 (sept, *J* = 6.0 Hz, OC**H**(CH₃)₂), 4.22 (s, NC**H**₂Ar), 3.14-3.20 (m, NHC**H**₂CH₂COOCH₃), 2.52 (t, *J* = 6.4 Hz, NHCH₂C**H**₂COOH), 1.41 (d, *J* = 6.0 Hz, OCH(C**H**₃)₂);
¹³C NMR (CD₃OD, 100 MHz) *δ* 173.18 (N**C**Se), 171.91 (**C**(O)), 155.18, 145.71, 140.40, 139.43, 132.32, 129.96, 128.81, 128.61, 127.35, 127.15, 126.20, 124.64, 115.14 (Ar**C**), 72.13 (O**C**H(CH₃)₂), 53.27 (N**C**H₂Ar), 51.66, 44.39, 34.49, 21.99 (OCH(**C**H₃)₂).

### Compound 40. Sodium 1-(4-(5-(4-tert-butylphenyl)-1,3-selenazole-2-yl)benzyl)azetidine-3-carboxylate

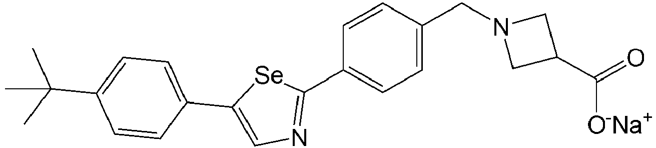

Whild solid;
Yield: 87%;
¹H NMR (CD₃OD, 400 MHz) *δ* 8.03 (s, 1 selenazole ring-**H**), 7.91 (d, *J* = 8.2 Hz, 2 Ar**H**), 7.58 (d, *J* = 8.4 Hz, 2 Ar**H**), 7.44-7.49 (m, 4 Ar**H**), 3.80 (s, NC**H**₂), 3.66-3.70 (m, 2 azetidine ring-**H**), 3.48-3.52 (m, 2 azetidine ring-**H**), 3.25-3.32 (m, 1 azetidine ring-**H**), 1.37 (s, Ar(C**H**₃)₃);
¹³C NMR (CD₃OD, 100 MHz) *δ* 179.00 (NCSe), 151.65, 138.67, 135.34, 133.19, 130.41, 129.44, 128.51, 126.62, 126.48, 125.80, 123.76 (ArC), 57.58 (N**C**H₂), 36.36, 34.15, 34.00, 30.35 (**C**(CH₃)₃), 22.81.

### Compound 41. Methyl 1-(4-(5-(3-chloro-4-isopropoxyphenyl)-1,3-selenazole-2-yl)benzyl)azetidine-3-carboxylate

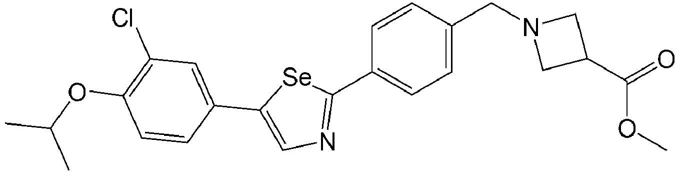

Yellow solid;
Yield: 41%;
¹H NMR (CDCl₃, 400 MHz) *δ* 7.85 (s, selenazole ring-**H**), 7.82 (d, *J* = 8.2 Hz, 2 Ar**H**), 7.56 (d, *J* = 2.2 Hz, 1 Ar**H**), 7.33-7.38 (m, 3 Ar**H**), 6.95 (d, *J* = 8.6 Hz, 1 Ar**H**), 4.61-4.58 (m, OC**H**(CH₃)₂), 3.79 (s, COOC**H**₃), 3.71 (s, NC**H**₂), 3.54-3.56 (m, 2 azetidine ring **H**), 3.33-3.37 (m, 3 azetidine ring-**H**), 1.40 (d, *J* = 6.0 Hz, OCH(C**H**₃)₂);
¹³C NMR (CDCl₃, 100 MHz) *δ* 173.52 (N**C**Se), 172.49 (**C**(O)), 153.71, 144.46, 140.08, 139.37, 135.33,132.84, 129.10, 127.20, 126.86, 126.60, 124.79, 115.97 (Ar**C**), 72.32 (O**C**H(CH₃)₂), 63.04 (N**C**H₂), 56.89, 51.99, 33.99 (azetidine ring-**C**), 22.01 (OCH(**C**H₃)₂).

### Compound 42. Sodium 1-(4-(5-(3-chloro-4-isopropoxyphenyl)-1,3-selenazole-2-yl)benzyl)azetidine-3-carboxylate

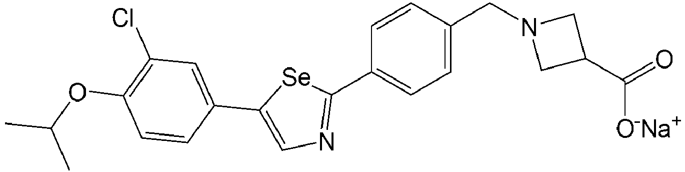

Whild solid;
Yield: 90%;
¹H NMR (DMSO-*d*₆, 400 MHz) *δ* 7.65 (d, *J* = 8.2 Hz, 2 Ar**H**), 7.28-7.41 (m, 7 Ar**H**), 5.70-5.75 (m, 1 isoxazoline ring-**H**), 3.75-3.80 (m, 1 isoxazoline ring-**H**), 3.73 (s, COOC**H**₃), 3.69 (s, NC**H**₂), 3.64 (br, 2 azetidine ring-**H**), 3.30-3.36 (m, 3 azetidine ring-**H**, 1 isoxazoline ring-**H**);
¹³C NMR (CDCl₃, 100 MHz) *δ* 173.49 (**C**(O)), 155.94 (isoxazoline ring-**C**), 140.97, 139.96, 128.79, 128.77, 128.37, 128.22, 126.84, 125.88 (Ar**C**), 82.53 (isoxazoline ring-**C**), 62.99 (N**C**H₂), 56.86 (azetidine ring-**C**), 51.99 (COO**C**H₃), 43.21 (isoxazoline ring-**C**), 33.96 (azetidine ring-**C**).

### Compound 43. Methyl 1-(4-(5-phenyl-4,5-dihydroisoxazole-3-yl)benzyl)azetidine-3-carboxylate

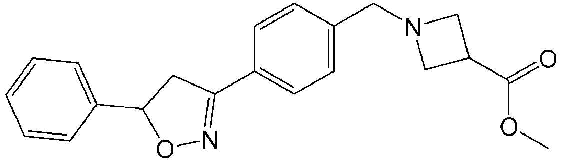

Transparent oil;
Yield: 57%;
¹H NMR (CDCl₃, 400 MHz) *δ* 7.65 (d, *J* = 8.2 Hz, 2 Ar**H**), 7.28-7.41 (m, 7 Ar**H**), 5.70-5.75 (m, 1 isoxazoline ring-**H**), 3.75-3.80 (m, 1 isoxazoline ring-**H**), 3.73 (s, COOC**H**₃), 3.69 (s, NC**H**₂), 3.64 (br, 2 azetidine ring-**H**), 3.30-3.36 (m, 3 azetidine ring-**H**, 1 isoxazoline ring-**H**);
¹³C NMR (CDCl₃, 100 MHz) *δ* 173.49 (**C**(O)), 155.94 (isoxazoline ring-**C**), 140.97, 139.96, 128.79, 128.77, 128.37, 128.22, 126.84, 125.88 (Ar**C**), 82.53 (isoxazoline ring-**C**), 62.99 (N**C**H₂), 56.86 (azetidine ring-**C**), 51.99 (COO**C**H₃), 43.21 (isoxazoline ring-**C**), 33.96 (azetidine ring-**C**);
HPLC purity: 3.3 min, 96.0%;
HRMS (M + H)⁺ (ESI⁺) 351.1707 [M + H]⁺ (calcd for C₂₁H₂₂N₂O₃H⁺ 351.1709).

### Compound 44. 1-(4-(5-phenyl-4,5-dihydroisoxazole-3-yl)benzyl)azetidine-3-carboxylic acid

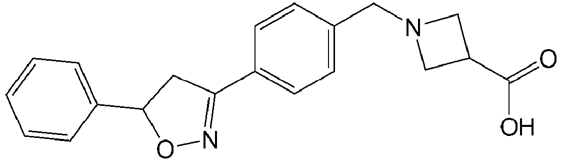

Whild solid;
Yield: 97%;
¹H NMR (DMSO-*d*₆, 400 MHz) *δ* 12.62 (br, COO**H**), 7.75 (d, *J* = 8.2 Hz, 2 Ar**H**), 7.65 (d, *J* = 8.2 Hz, 2 Ar**H**), 7.36-7.40 (m, 4 Ar**H**), 7.31-7.35 (m, 1 Ar**H**), 5.73-5.77 (m, 1 isoxazoline ring-**H**), 4.40 (s, NC**H**₂), 4.04-4.12 (m, 4 azetidine ring **H**), 3.85-3.92 (m, 1 isoxazoline ring-**H**), 3.63-3.67 (m, 1 azetidine ring-**H**), 3.40-3.44 (m, 1 isoxazoline ring-**H**);
¹³C NMR (DMSO-*d*₆, 100 MHz) *δ* 171.96 (**C**(O)), 156.62 (isoxazoline ring-**C**), 141.26, 133.09, 131.02, 130.39, 129.12, 128.63, 127.47, 126.67 (Ar**C**), 82.73 (isoxazoline ring-**C**), 56.74 (N**C**H₂), 54.58 (azetidine ring-**C**), 42.48 (isoxazoline ring-**C**), 32.51 (azetidine ring-**C**);
HPLC purity: 3.6 min, 96.8%;
HRMS (M + H)⁺ (ESI⁺) 337.1551 [M + H]⁺ (calcd for C₂₀H₂₁N₃O₃H⁺ 337.1552).

### Compound 45. Methyl 1-(4-(5-(2-bromophenyl)-4,5-dihydroisoxazole-3-yl)benzyl)azetidine-3-carboxylate

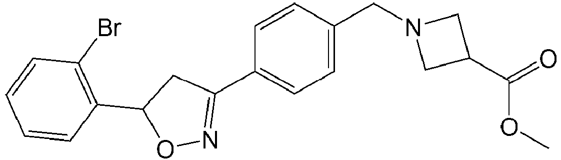

Transparent oil;
Yield: 28%;
¹H NMR (CDCl₃, 400 MHz) *δ* 7.64 (d, *J* = 8.4 Hz, 2 Ar**H**), 7.55-7.59 (m, 2 Ar**H**), 7.29-7.35 (m, 3 Ar**H**), 7.15-7.20 (m, 1 Ar**H**), 5.96-6.01 (m, 1 isoxazoline ring-**H**), 3.93-4.00 (m, 1 isoxazoline ring-**H**), 3.72 (s, COOC isoxazoline ring-**H**), 3.93-4.00 (m, 1 isoxazoline ring-**H**), 3.72 (s, COOC**H**₃), 3.71 (s, NC**H**₂), 3.52-3.64 (m, 2 azetidine ring-**H**), 3.33-3.36 (m, 3 azetidine ring-**H**), 3.17-3.23 (m, 1 isoxazoline ring-**H**);
¹³C NMR (CDCl₃, 100 MHz) *δ* 173.48 (**C**(O)), 155.84 (isoxazoline ring-**C**), 140.65, 140.17, 132.74, 129.36, 128.75, 128.09, 127.83, 127.43, 126.88, 120.84 (Ar**C**), 81.42 (isoxazoline ring-**C**), 63.00 (N**C**H₂), 56.86 (azetidine ring-**C**), 51.97 (COO**C**H₃), 42.98 (isoxazoline ring-**C**), 33.96 (azetidine ring-**C**);
HPLC purity: 5.09 min, 97.9%;
HRMS (M + H)⁺ (ESI⁺) 429.0817 [M + H]⁺ (calcd for C₂₁H₂₁BrN₂O₃H⁺ 429.0814).

### Compound 46. 1-(4-(5-(2-bromophenyl)-4,5-dihydroisoxazole-3-yl)benzyl)azetidine-3-carboxylic acid

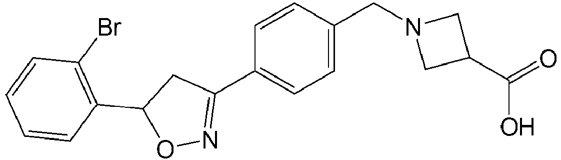

Whild solid;
Yield: 60%;
¹H NMR (DMSO-*d*₆, 400 MHz) *δ* 7.78 (d, *J* = 8.0 Hz, 2 Ar**H**), 7.69 (d, *J* = 8.0 Hz, 1 Ar**H**), 7.58 (d, *J* = 8.4 Hz, 2 ArH), 7.45-7.46 (m, 2 Ar**H**), 7.28-7.32 (m, 1 Ar**H**), 5.93-5.98 (m, 1 isoxazoline ring-**H**), 4.40 (s, NC**H**₂), 4.12-4.14 (m, 4 azetidine ring-**H**), 4.01-4.05 (m, 1 isoxazoline ring-**H**), 3.59-3.64 (m, 1 azetidine ring-**H**), 3.34-3.37 (m, 1 isoxazoline ring-**H);**
¹³C NMR (DMSO-*d*₆, 100 MHz) *δ* 181.85, 177.21, 156.43 (isoxazoline ring-**C**), 140.43, 133.36, 130.99, 130.50, 130.21, 128.64, 127.69, 127.53, 121.38 (Ar**C**), 86.90 (isoxazoline ring-**C**), 81.74, 56.97 (N**C**H₂), 55.04 (azetidine ring-**C**), 42.34 (isoxazoline ring-**C**), 32.52 (azetidine ring-**C**);
HPLC purity: 4.6 min, 99.3%;
HRMS (M + H)⁺ (ESI⁺) 415.0659 [M + H]⁺ (calcd for C₂₀H₁₉BrN₂O₃H⁺ 415.0657).

### Compound 47. Methyl 1-(4-(5-(3-bromophenyl)-4,5-dihydroisoxazole-3-yl)benzyl)azetidine-3-carboxylate

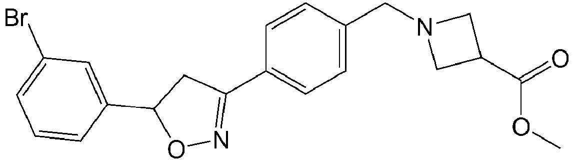

Transparent oil;
Yield: 35%;
¹H NMR (CDCl₃, 400 MHz) *δ* 7.62 (d, *J* = 8.4 Hz, 2 Ar**H**), 7.53 (s, 1 Ar**H**), 7.42 (d, *J* = 7.6 Hz, 1 Ar**H**), 7.29-7.32 (m, 3Ar**H**), 7.20-7.24 (m, 1 Ar**H**), 5.65-5.69 (m, 1 isoxazoline ring-**H**), 3.73-3.80 (m, 1 isoxazoline ring-**H**), 3.70 (s, COOC**H**₃), 3.62 (s, NC**H**₂), 3.50-3.55 (m, 2 azetidine ring-**H**), 3.25-3.35 (m, 3 azetidine ring-**H**, 1 isoxazoline ring-**H**);
¹³C NMR (CDCl₃, 100 MHz) *δ* 173.47 (**C**(O)), 155.87 (isoxazoline ring-**C**), 143.39, 140.23, 131.23, 130.37, 128.85, 128.78, 128.00, 126.86, 124.43, 122.80 (Ar**C**), 81.50 (isoxazoline ring-**C**), 62.97 (N**C**H₂), 56.86 (azetidine ring-**C**), 51.97 (COO**C**H₃), 43.27 (isoxazoline ring-**C**), 33.96 (azetidine ring-**C**);
HPLC purity: 4.7 min, 98.2%;
HRMS (M + H)⁺ (ESI⁺) 429.0816 [M + H]⁺ (calcd for C₂₁H₂₁BrN₂O₃H⁺ 429.0814).

### Compound 48. 1-(4-(5-(3-bromophenyl)-4,5-dihydroisoxazole-3-yl)benzyl)azetidine-3-carboxylic acid

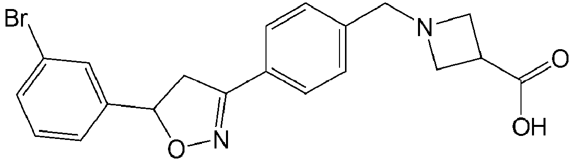

Whild solid;
Yield: 96%;
¹H NMR (DMSO-*d*₆, 400 MHz) *δ* 7.75 (d, *J* = 8.0 Hz, 2 Ar**H**), 7.66 (d, *J =* 8.0 Hz, 2 Ar**H**), 7.59 (s, 1 Ar**H**), 7.54 (d, *J* = 8.8 Hz, 1 Ar**H**), 7.34-7.42 (m, 2 Ar**H**), 5.76-5.81 (m, 1 isoxazoline ring-**H**), 4.42 (s, NC**H**₂), 4.08-4.12 (m, 5 azetidine ring-**H**), 3.86-3.93 (m, 1 isoxazoline ring-**H**), 3.43-3.49 (m, 1 isoxazoline ring-**H**);
¹³C NMR (DMSO-*d*₆, 100 MHz) *δ* 171.03 (**C**(O)), 156.72 (isoxazoline ring-**C**), 144.11, 139.56, 132.05, 131.40, 131.03, 130.19, 129.31, 127.55, 125.66, 122.31 (Ar**C**), 81.71 (isoxazoline ring-**C**), 54.43 (N**C**H₂), 52.75 (azetidine ring-**C**), 42.54 (isoxazoline ring-**C**), 32.28 (azetidine ring-**C**);
HPLC purity: 5.1 min, 98.6%;
HRMS (M + H)⁺ (ESI⁺) 415.0662 [M + H]⁺ (calcd for C₂₀H₁₉BrN₂O₃H⁺ 415.0657).

### Compound 49. Methyl 1-(4-(5-(4-bromophenyl)-4,5-dihydroisoxazole-3-yl)benzyl)azetidine-3-carboxylate

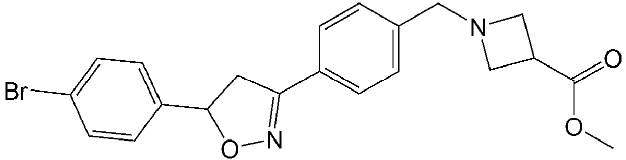

Whild solid;
Yield: 16%;
¹H NMR (CDCl₃, 400 MHz) *δ* 7.64 (d, *J* = 8.4 Hz, 2 Ar**H**), 7.49-7.53 (m, 2 Ar**H**), 7.36 (d, *J* = 10.0 Hz, 2 Ar**H**), 7.29-7.32 (m, 2 Ar**H**), 5.67-5.72 (m, 1 isoxazoline ring-**H**), 3.77-3.82 (m, 1 isoxazoline ring-**H**), 3.75 (s, COOC**H**₃), 3.68 (s, NC**H**₂), 3.52-3.65 (m, 2 azetidine ring-**H**), 3.26-3.39 (m, 3 azetidine ring-**H**, 1 isoxazoline ring-**H**);
¹³C NMR (CDCl₃, 100 MHz) *δ* 173.52 (**C**(O)), 155.91 (isoxazoline ring-**C**), 140.19, 140.05, 131.87, 128.80, 128.08, 127.56, 126.85, 122.11 (Ar**C**), 81.75 (isoxazoline ring-**C**), 62.99 (N**C**H₂), 56.87 (azetidine ring-**C**), 52.00 (COO**C**H₃), 43.22 (isoxazoline ring-**C**), 33.96 (azetidine ring-**C**);
HPLC purity: 9.1 min, 98.3%;
HRMS (M + H)⁺ (ESI⁺) 429.0817 [M + H]⁺ (calcd for C₂₁H₂₁BrN₂O₃H⁺ 429.0814).

### Compound 50. 1-(4-(5-(4-bromophenyl)-4,5-dihydroisoxazole-3-yl)benzyl)azetidine-3-carboxylic acid

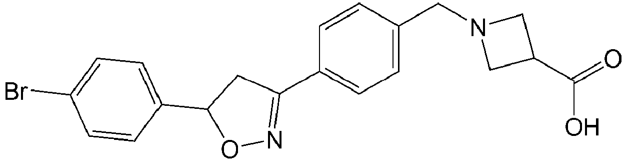

Whild solid;
Yield: 58%;
¹H NMR (DMSO-*d*₆, 400 MHz) *δ* 7.58-7.63 (m, 4 Ar**H**), 7.32-7.37 (m, 4 Ar**H**), 5.69-5.74 (m, 1 isoxazoline ring-**H**), 3.81-3.96 (m, 1 isoxazoline ring-**H**), 3.50 (s, NC**H**₂), 3.22-3.26 (m, 2 azetidine ring-**H**, 1 isoxazoline ring-**H**), 3.04-3.07 (m, 2 azetidine ring-**H**), 2.74-2.78 (m, 1 azetidine ring-**H**);
¹³C NMR (CD₃OD, 100 MHz) *δ* 178.90 (**C**(O)), 156.47, 140.34, 131.50, 129.45, 129.08, 127.64, 126.81, 121.59 (Ar**C**, isoxazoline ring-**C**), 81.97, 57.12, 42.37, 35.83, 22.75;
HPLC purity: 4.8 min, 99.1%;
HRMS (M + H)⁺ (ESI⁺) 415.0659 [M + H]⁺ (calcd for C₂₀H₁₉BrN₂O₃H⁺ 415.0657).

### Compound 51. 1-(4-(5-(4-hydroxyphenyl)-4,5-dihydroisoxazole-3-yl)benzyl)azetidine-3-carboxylic acid

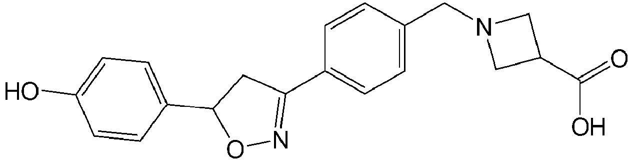

Whild solid;
Yield: 66%;
¹H NMR (DMSO-*d*₆, 400 MHz) *δ* 9.66 (s, COO**H**), 7.66 (d, *J* = 8.2 Hz, 2 Ar**H**), 7.41 (d, *J* = 8.0 Hz, 2 Ar**H**), 7.19 (d, *J* = 8.6 Hz, 2 Ar**H**), 6.78 (d, *J* = 8.5 Hz, 2 Ar**H**), 5.60 (t, *J =* 10.4 Hz, 1 isoxazoline ring-**H**), 3.73-3.80 (m, 3**H**), 3.55 (br, NC**H**₂), 3.40 (br, 1**H**), 3.30-3.37 (m, 3**H**);
¹³C NMR (DMSO-*d*₆, 100 MHz) *δ* 158.00, 156.79, 131.03, 129.59, 129.15, 128.65, 128.24, 127.12, 115.79 (Ar**C**, isoxazoline ring-**C**), 82.75 (isoxazoline ring-**C**), 56.29 (N**C**H₂), 49.74 (azetidine ring-**C**), 42.09 (isoxazoline ring-**C**), 33.69 (azetidine ring-**C**);
HPLC purity: 1.9 min, 98.6%;
HRMS (M + H)⁺ (ESI⁺) 353.1500 [M + H]⁺ (calcd for C₂₀H₂₀N₂O₄H⁺ 353.1501).

### Compound 52. Methyl 1-(4-(5-(4-(chloromethyl)phenyl)-4,5-dihydroisoxazole-3-yl)benzyl)azetidine-3-carboxylate

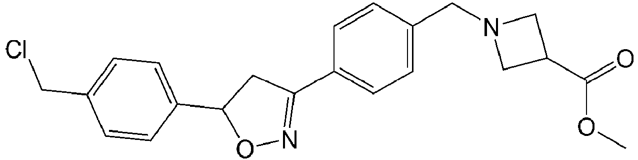

Transparent oil;
Yield: 23%;
¹H NMR (CDCl₃, 400 MHz) *δ* 7.64 (d, *J* = 8.4 Hz, 2 Ar**H**), 7.37-7.41 (m, 4 Ar**H**), 7.33 (d, *J* = 8.0 Hz, 2 Ar**H**), 5.71-5.75 (m, 1 isoxazoline ring-**H**), 4.58 (s, ArC**H**₂Cl), 3.75-3.81 (m, 1 isoxazoline ring-**H**), 3.73 (s, COOC**H**₃), 3.65 (s, NC**H**₂), 3.53-3.56 (m, 2 azetidine ring-**H**), 3.28-3.36 (m, 2 azetidine ring-**H**, 1 isoxazoline ring-**H**);
¹³C NMR (CDCl₃, 100 MHz) *δ* 173.49 (**C**(O)), 155.94 (isoxazoline ring-**C**), 141.28, 140.10, 137.47, 129.02, 128.79, 128.20, 126.84, 126.26 (Ar**C**), 82.06 (isoxazoline ring-**C**), 62.97 (N**C**H₂), 56.86 (azetidine ring-**C**), 51.99 (COO**C**H₃), 45.82 (Ar**C**H₂Cl), 43.21 (isoxazoline ring-**C**), 33.96 (azetidine ring-**C**);
HPLC purity: 10.1 min, 96.9%;
HRMS (M + H)⁺ (ESI⁺) 399.1480 [M + H]⁺ (calcd for C₂₂H₂₃ClN₂O₃H⁺ 399.1475).

### Compound 53. Sodium 1-(4-(5-(4-(chloromethyl)phenyl)-4,5-dihydroisoxazole-3-yl)benzyl)azetidine-3-carboxylate

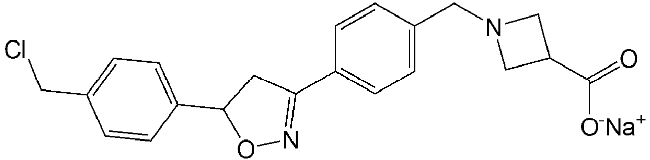

Whild solid;
Yield: 45%;
¹H NMR (DMSO-*d*₆, 400 MHz) *δ* 7.63 (d, *J* = 8.2 Hz, 2 Ar**H**), 7.39-7.47 (m, 4 Ar**H**), 7.32 (d, *J* = 8.2 Hz, 2 Ar**H**), 5.70-5.75 (m, 1 isoxazoline ring-**H**), 4.77 (s, C**H**₂Cl), 3.83-3.90 (1 isoxazoline ring-**H**), 3.49 (s, NC**H**₂), 3.40-3.42 (m, 1 isoxazoline ring-**H**), 3.17-3.25 (m, 2 azetidine ring-**H**), 3.02-3.06 (m, 2 azetidine ring-**H**), 2.67-2.73 (m, 1 azetidine ring-**H**);
¹³C NMR (DMSO-*d*₆, 100 MHz) *δ* 172.43 (C(O)), 156.75 (isoxazoline ring-**C**), 141.47, 140.02, 139.01, 129.48, 129.36, 128.48, 127.04, 126.63 (ArC), 82.32 (isoxazoline ring-C), 57.76 (N**C**H₂), 53.12, 52.18, 46.75, 43.86, 42.51;
HPLC purity: 5.6 min, 98.1%;
HRMS (M + H)⁺ (ESI⁺) 385.1320 [M + H]⁺ (calcd for C₂₁H₂₁ClN₂O₃H⁺ 385.1319).

### Compound 54. Methyl 1-(4-(5-(4-ethylphenyl)-4,5-dihydroisoxazole-3-yl)benzyl)azetidine-3-carboxylate

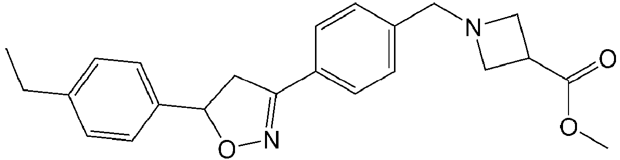

Whild solid;
Yield: 46%;
¹H NMR (CDCl₃, 400 MHz) *δ* 7.63 (d, *J* = 8.2 Hz, 2 Ar**H**), 7.29-7.32 (m, 4 Ar**H**), 7.19 (d, *J* = 8.0 Hz, 2 Ar**H**), 5.69 (dd, *J* = 4.2, 10.8 Hz, 1 isoxazoline ring-**H**), 3.72-3.76 (m, 1 isoxazoline ring-**H**), 3.70 (s, COOC**H**₃), 3.62 (s, NC**H**₂), 3.50-3.55 (m, 2 azetidine ring-**H**), 3.29-3.35 (m, 3 azetidine ring-**H**, 1 isoxazoline ring-**H**), 2.64 (q, *J* = 7.6 Hz, C**H**₂CH₃), 1.22 (t, *J* = 7.6 Hz, CH₂C**H**₃);
¹³C NMR (CDCl₃, 100 MHz) *δ* 173.52 (**C**(O)), 155.98 (isoxazoline ring-**C**), 144.43, 139.96, 138.11, 128.76, 128.47, 128.24, 126.82, 125.98 (Ar**C**), 82.57 (isoxazoline ring-**C**), 63.05 (N**C**H₂), 56.89 (azetidine ring-**C**), 51.98 (COO**C**H₃), 43.08 (isoxazoline ring-**C**), 33.99 (azetidine ring-**C**), 28.58 (**C**H₂CH₃), 15.57 (CH₂**C**H₃);
HPLC purity: 4.5 min, 98.4%;
HRMS (M + H)⁺ (ESI⁺) 379.2021 [M + H]⁺ (calcd for C₂₃H₂₆N₂O₃H⁺ 379.2022).

### Compound 55. Sodium 1-(4-(5-(4-ethylphenyl)-4,5-dihydroisoxazole-3-yl)benzyl)azetidine-3-carboxylate

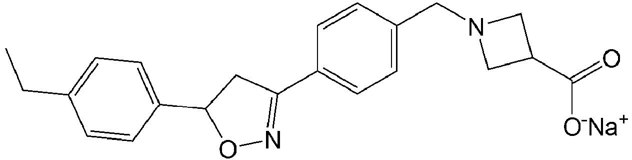

Whild solid;
Yield: 100%;
¹H NMR (CD₃OD, 400 MHz) *δ* 7.67 (d, *J* = 8.2 Hz, 2 Ar**H**), 7.37 (d, *J* = 8.2 Hz, 2 Ar**H**), 7.30 (d, *J* = 8.1 Hz, 2 Ar**H**), 7.21 (d, *J* = 8.0 Hz, 2 Ar**H**), 5.68 (dd, *J* = 8.8, 10.7 Hz, 1 isoxazoline ring-**H**), 3.82 (dd, *J* = 10.8, 17.0 Hz, 1 isoxazoline ring-**H**), 3.66 (s, NC**H**₂), 3.53 (t, *J* = 8.0 Hz, 2 azetidine ring-**H**), 3.32-3.37 (m, 2 azetidine ring-**H**, 1 isoxazoline ring-**H**), 3.18-3.24 (m, 1 azetidine ring-**H**), 2.63 (q, *J* = 7.6 Hz, alkyl chain-C**H**₂), 1.82 (t, *J* = 7.6 Hz, alkyl chain-C**H**₃);
¹³C NMR (CD₃OD, 100 MHz) *δ* 179.57 (**C**(O)), 156.75 (isoxazoline ring-**C**), 144.32, 139.64, 139.64, 138.11, 128.97, 127.82, 126.54, 125.81 (ArC), 82.71 (isoxazoline ring-**C**), 62.45 (N**C**H₂), 57.69 (azetidine ring-**C**), 42.28 (isoxazoline ring-**C**), 36.54 (azetidine ring-**C**), 28.16 (**C**H₂CH₃), 14.75 (CH₂**C**H₃);
HPLC purity: 5.0 min, 97.8%;
HRMS (M + H)⁺ (ESI⁺) 365.1864 [M + H]⁺ (calcd for C₂₂H₂₄N₂O₃H⁺ 365.1865).

### Compound 56. Sodium 1-(4-(5-(4-propylphenyl)-4,5-dihydroisoxazole-3-yl)benzyl)azetidine-3-carboxylate

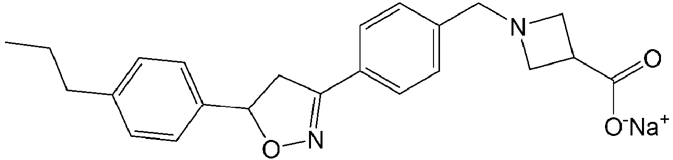

Whild solid;
Yield: 112%;
¹H NMR (CD₃OD, 400 MHz) *δ* 7.67 (d, *J* = 8.3 Hz, 2 Ar**H**), 7.37 (d, *J* = 8.3 Hz, 2 Ar**H**), 7.29 (d, *J* = 8.1 Hz, 2 ArH), 7.19 (d, *J* = 8.1 Hz, 2 Ar**H**), 5.67 (dd, *J* = 8.8, 10.7 Hz, 1 isoxazoline ring-**H**), 3.82 (dd, *J* = 10.8, 17.0 Hz, 1 isoxazoline ring-**H**), 3.65 (s, NC**H**₂), 3.53 (t, *J* = 8.1 Hz, 2 azetidine ring-**H**), 3.32-3.37 (m, 2 azetidine ring-**H**, 1 isoxazoline ring-**H**), 3.18-3.22 (m, 1 azetidine ring-**H**), 2.58 (t, *J* = 7.8 Hz, alkyl chain-C**H**₂), 1.59-1.65 (m, alkyl chain-C**H**₂), 0.92 (t, *J* = 7.3 Hz, alkyl chain-C**H**₃);
¹³C NMR (CD₃OD, 100 MHz) *δ* 179.55 (**C**(O)), 156.77 (isoxazoline ring-**C**), 142.65, 139.66, 138.16, 128.98, 128.46, 126.54, 125.73 (Ar**C**), 82.74 (isoxazoline ring-**C**), 62.47 (N**C**H₂) 57.67 (azetidine ring-**C**), 42.38 (isoxazoline ring-**C**), 37.32 (alkyl chain-**C**H₂), 36.54 (azetidine ring-**C**), 24.31 (alkyl chain-**C**H₂), 12.67 (alkyl chain-**C**H₃);
HPLC purity: 5.8 min, 99.3%;
HRMS (M + H)⁺ (ESI⁺) 379.2021 [M + H]⁺ (calcd for C₂₃H₂₆N₂O₃H⁺ 379.2022).

### Compound 57. Methyl 1-(4-(5-(4-tert-butylphenyl)-4,5-dihydroisoxazole-3-yl)benzyl)azetidine-3-carboxylate

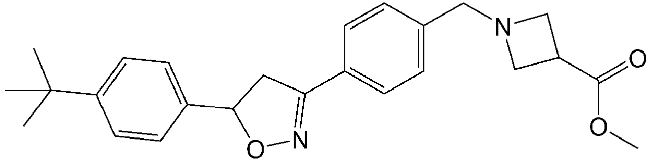

Whild solid;
Yield: 45%;
¹H NMR (DMSO-*d*₆, 400 MHz) *δ* 7.64 (d, *J* = 7.9 Hz, 2 Ar**H**), 7.39 (d, *J* = 8.2 Hz, 2 Ar**H**), 7.32 (dd, *J* = 3.4, 8.3 Hz, 4 Ar**H**), 5.71 (dd, *J* = 8.3, 10.9 Hz, 1 isoxazoline ring-**H**), 3.68-3.79 (m, 1 isoxazoline ring-**H**, COOC**H**₃), 3.63 (s, NC**H**₂), 3.57-3.60 (m, 1 azetidine ring-**H**, 1 isoxazoline ring-**H**), 3.39-3.42 (m, 2 azetidine ring-**H**), 3.21-3.36 (m, 2 azetidine ring-**H**), 1.28 (s, C(C**H**₃)₃);
¹³C NMR (DMSO-*d*₆, 75 MHz) *δ* 173.33 (**C**(O)), 162.33, 156.74, 151.04, 138.32, 132.53, 129.58, 127.14, 126.39, 125.81 (Ar**C**, isoxazoline ring-**C**), 82.31 (isoxazoline ring-**C**), 56.52 (azetidine ring-**C**), 52.19 (COO**C**H₃), 49.06 (azetidine ring-**C**), 42.45 (isoxazoline ring-**C**), 34.74 (**C**(CH₃)₃), 33.61 (azetidine ring-**C**), 31.55 (C(**C**H₃)₃);
HPLC purity: 9.8 min, 95.5%;
HRMS (M + H)⁺ (ESI⁺) 407.2336 [M + H]⁺ (calcd for C₂₅H₃₀N₂O₃H⁺ 407.2335).

### Compound 58. Sodium 1-(4-(5-(4-tert-butylphenyl)-4,5-dihydroisoxazole-3-yl)benzyl)azetidine-3-carboxylate

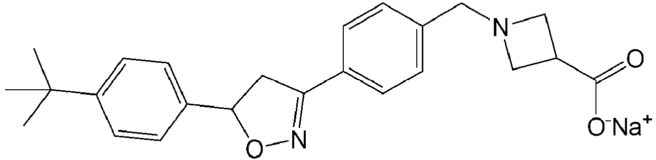

Whild solid;
Yield: 96%;
¹H NMR (DMSO-*d*₆, 400 MHz) *δ* 7.63 (d, *J* = 8.2 Hz, 2 Ar**H**), 7.41 (d, *J* = 8.3 Hz, 2 Ar**H**), 7.30-7.33 (m, 4 Ar**H**), 5.65-5.69 (m, 1 isoxazoline ring-**H**), 3.78-3.85 (m, 1 isoxazoline ring-**H**), 3.50 (s, NC**H**₂), 3.36-3.40 (m, 1**H**), 3.25 (t, *J* = 7.6 Hz, 2**H**), 3.06 (t, *J* = 7.0 Hz, 2**H**), 2.74 (t, *J* = 7.7 Hz, 1**H**), 1.27 (s, C(C**H**₃)₃);
¹³C NMR (DMSO-*d*₆, 100 MHz) *δ* 176.87 (**C**(O)), 156.79 (isoxazoline ring-**C**), 151.01, 141.80, 138.36, 128.97, 128.16, 126.98, 126.43, 125.82 (ArC), 82.12 (isoxazoline ring-**C**), 63.26 (N**C**H₂), 58.85, 37.35, 34.76, 31.57 (C(**C**H₃)₃);
HPLC purity: 6.0 min, 99.3%;
HRMS (M + H)⁺ (ESI⁺) 393.2176 [M + H]⁺ (calcd for C₂₄H₂₈N₂O₃H⁺ 393.2178).

### Compound 59. Methyl 1-(4-(5-(3-cyano-4-isopropoxyphenyl)-4,5-dihydroisoxazole-3-yl)benzyl)azetidine-3-carboxylate

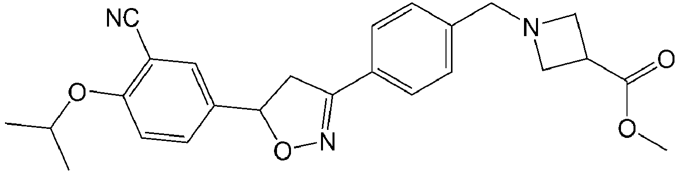

Transparent oil;
Yield: 54%;
¹H NMR (CDCl₃, 400 MHz) *δ* 7.63 (d, *J* = 8.2 Hz, 2 Ar**H**), 7.52-7.56 (m, 2 Ar**H**), 7.33 (d, *J* = 8.2 Hz, 2 Ar**H**), 6.96 (d, *J* = 8.7 Hz, 1 Ar**H**), 5.65-5.70 (m, 1 isoxazoline ring-**H**), 4.65 (sept, *J* = 6.0 Hz, OC**H**(CH₃)₂), 3.70-3.81 (m, 1 isoxazoline ring-**H**), 3.65 (s, COOC**H**₃), 3.54 (s, NC**H**₂), 3.31-3.37 (m, 2 azetidine ring-**H**), 3.24-3.30 (m, 3 azetidine ring-**H**, 1 isoxazoline ring-**H**), 1.41 (d, *J* = 6.0 Hz, OCH(C**H**₃)₂);
¹³C NMR (CDCl₃, 100 MHz) *δ* 173.48 (**C**(O)), 159.77, 155.99 (isoxazoline ring-**C**), 140.32, 133.33, 131.87, 131.40, 128.81, 127.93, 126.85, 116.32, 114.04 (Ar**C**), 103.12, 81.09 (isoxazoline ring-**C**), 72.08 (O**C**H(CH₃)₂), 62.97 (N**C**H₂), 56.86 (azetidine ring-**C**), 51.97 (COO**C**H₃), 43.02 (isoxazoline ring-**C**), 33.95 (azetidine ring-**C**), 21.79 (OCH(**C**H₃)₂);
HPLC purity: 4.3 min, 98.0%;
HRMS (M + H)⁺ (ESI⁺) 434.2080 [M + H]⁺ (calcd for C₂₅H₂₇N₃O₄H⁺ 434.2080).

### Compound 60. 1-(4-(5-(3-cyano-4-isopropoxyphenyl)-4,5-dihydroisoxazole-3-yl)benzyl)azetidine-3-carboxylic acid

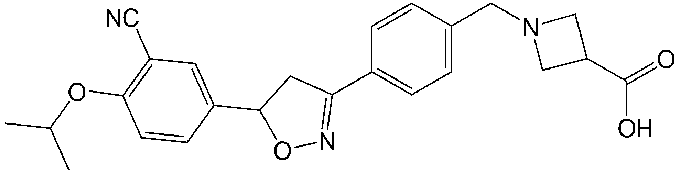

Whild solid;
Yield: 64%;
¹H NMR (CD₃OD, 400 MHz) *δ* 7.84 (d, *J* = 8.3 Hz, 2 Ar**H**), 7.64-7.67 (m, 2 Ar**H**), 7.56 (d, *J* = 8.3 Hz, 2 Ar**H**), 7.21-7.23 (m, 1 Ar**H**), 5.74-5.79 (m, 1 isoxazoline ring-**H**), 4.76-4.91 (m, OC**H**(CH₃)₂), 4.47 (s, NC**H**₂), 4.28-4.36 (m, 4 azetidine ring-**H**), 3.85-3.92 (m, 1 isoxazoline ring-**H**), 3.61-3.69 (m, 1 azetidine ring-**H**), 3.32-3.44 (m, 1 isoxazoline ring-**H**), 1.40 (d, *J* = 6.0 Hz, OCH(C**H**₃)₂);
¹³C NMR (CD₃OD, 100 MHz) *δ* 173.04 (**C**(O)), 159.77, 156.27, 133.44, 132.29, 131.73, 131.15, 130.89, 130.20, 127.38, 115.84, 114.13 (Ar**C**, isoxazoline ring-**C**), 102.23, 81.64 (isoxazoline ring-**C**), 71.85 (O**C**H(CH₃)₂), 57.65 (N**C**H₂), 55.92 (azetidine ring-**C**), 41.91 (isoxazoline ring-**C**), 32.99 (azetidine ring-**C**), 20.67 (OCH(**C**H₃)₂);
HPLC purity: 8.9 min, 96.0%;
HRMS (M + H)⁺ (ESI⁺) 420.1924 [M + H]⁺ (calcd for C₂₄H₂₅N₃O₄H⁺ 420.1923).

### Compound 61. Methyl 1-(4-(5-(3-chloro-4-ethoxyphenyl)-4,5-dihydroisoxazole-3-yl)benzyl)azetidine-3-carboxylate

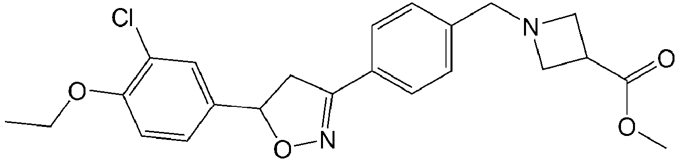

Whild solid;
Yield: 83%;
¹H NMR (CDCl₃, 400 MHz) *δ* 7.62 (d, *J* = 8.2 Hz, 2 Ar**H**), 7.38 (d, *J* = 2.1 Hz, 1 Ar**H**), 7.31 (d, *J* = 8.2 Hz, 2 Ar**H**), 7.21 (dd, *J* = 2.1, 8.4 Hz, 1 Ar**H**), 6.89 (d, *J* = 8.5 Hz, 1 Ar**H**), 5.63 (dd, *J* = 8.2, 10.8 Hz, 1 isoxazoline ring-**H**), 4.09 (q, *J* = 6.9 Hz, OC**H**₂CH₃), 3.68-3.75 (m, 1 isoxazoline ring-**H**), 3.69 (s, COOC**H**₃), 3.62 (s, NC**H**₂), 3.50-3.53 (m, 2 azetidine ring-**H**), 3.24-3.34 (m, 3 azetidine ring-**H**, 1 isoxazoline ring-**H**), 1.45 (t, *J* = 7.0 Hz, OCH₂C**H**₃-);
¹³C NMR (CDCl₃, 100 MHz) *δ* 173.51 (**C**(O)), 155.96 (isoxazoline ring-**C**), 154.37, 140.09, 133.82, 128.79, 127.93, 126.84, 125.35, 123.17, 113.39 (Ar**C**), 81.67 (isoxazoline ring-**C**), 64.86 (N**C**H₂), 63.02 (O**C**H₂CH₃), 56.87 (azetidine ring-**C**), 51.98 (COO**C**H₃), 43.05 (isoxazoline ring-**C**), 33.96 (azetidine ring-**C**), 14.66 (OCH₂**C**H₃);
HPLC purity: 4.7 min, 98.5%;
HRMS (M + H)⁺ (ESI⁺) 429.1581 [M + H]⁺ (calcd for C₂₃H₂₅ClN₂O₄H⁺ 429.1581).

### Compound 62. Sodium 1-(4-(5-(3-chloro-4-ethoxyphenyl)-4,5-dihydroisoxazole-3-yl)benzyl)azetidine-3-carboxylate

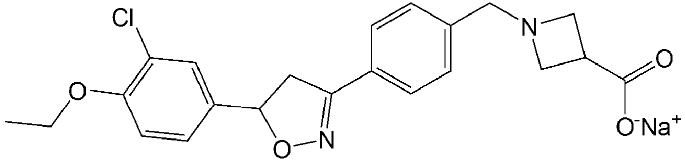

Yellow solid;
Yield: 86%;
¹H NMR (CD₃OD, 400 MHz) *δ* 7.67 (d, *J* = 8.2 Hz, 2 Ar**H**), 7.37-7.40 (m, 3 Ar**H**), 7.28 (dd, *J* = 2.1, 8.5 Hz, 1 Ar**H**), 7.05 (d, *J* = 8.5 Hz, 1 Ar**H**), 5.65 (dd, *J* = 8.6, 10.7 Hz, 1 isoxazoline ring-**H**), 4.11 (q, *J* = 6.9 Hz, OC**H**₂CH₃), 3.83 (dd, *J* = 10.8, 18.2 Hz, 1 isoxazoline ring-**H**), 3.67 (s, NC**H**₂), 3.55 (t, *J* = 8.0 Hz, 2 azetidine ring-**H**), 3.34-3.38 (m, 2 azetidine ring-**H**, 1 isoxazoline ring-**H**), 3.19-3.25 (m, 1 azetidine ring-**H**), 1.42 (t, *J* = 6.9 Hz, OCH₂C**H**₃-);
¹³C NMR (CD₃OD, 100 MHz) *δ* 179.50 (**C**(O)), 157.55, 154.16, 133.82, 129.46, 128.15, 127.54, 126.93, 125.76, 122.41, 113.82 (Ar**C**, isoxazoline ring-**C**), 81.79 (isoxazoline ring-**C**), 64.99 (N**C**H₂), 61.45 (O**C**H₂CH₃), 57.27 (azetidine ring-**C**), 42.35 (isoxazoline ring-**C**), 36.09 (azetidine ring-**C**), 13.74 (OCH₂**C**H₃);
HPLC purity: 5.1 min, 95.9%;
HRMS (M + H)⁺ (ESI⁺) 415.1424 [M + H]⁺ (calcd for C₂₂H₂₃ClN₂O₄H⁺ 415.1425).

### Compound 63. Methyl 1-(4-(5-(3-chloro-4-isopropoxyphenyl)-4,5-dihydroisoxazole-3-yl)benzyl)azetidine-3-carboxylate

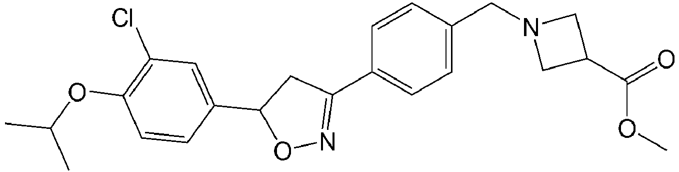

Yellow oil;
Yield: 44%;
¹H NMR (CDCl₃, 400 MHz) *δ* 7.61 (d, *J* = 8.1 Hz, 2 Ar**H**), 7.37 (d, *J* = 2.0 Hz, 1 Ar**H**), 7.30 (d, *J* = 8.0 Hz, 2 Ar**H**), 7.19 (dd, *J* = 2.0, 8.4 Hz, 1 Ar**H**), 6.91 (d, *J* = 8.4 Hz, 1 Ar**H**), 5.61 (dd, *J* = 8.2, 10.8 Hz, 1 isoxazoline ring-**H**), 4.51-4.57 (m, OC**H**(CH₃-)₂), 3.72 (dd, *J* = 10.9, 16.7 Hz, 1 isoxazoline ring-**H**), 3.69 (s, COOC**H**₃), 3.63 (s, NC**H**₂), 3.52-3.54 (m, 2 azetidine ring-**H**), 3.26-3.36 (m, 1 isoxazoline ring-**H**, 3 azetidine ring-**H**), 1.35 (d, *J* = 6.0 Hz, OCH(C**H**₃-)₂);
¹³C NMR (CDCl₃, 100 MHz) *δ* 173.49 (**C**(O)), 156.01 (isoxazoline ring-**C**), 153.54, 140.09, 134.06, 128.78, 128.21, 128.05, 126.82, 125.27, 124.45, 115.97 (Ar**C**), 81.66 (isoxazoline ring-**C**), 72.19 (O**C**H(CH₃-)₂), 62.98 (N**C**H₂), 56.85 (azetidine ring-**C**), 51.96 (COO**C**H₃), 42.98 (isoxazoline ring-**C**), 33.95 (azetidine ring-**C**), 21.99 (OCH(**C**H₃-)₂);
HPLC purity: 5.3 min, 97.5%;
HRMS (M + H)⁺ (ESI⁺) 443.1740 [M + H]⁺ (calcd for C₂₄H₂₇ClN₂O₄H⁺ 443.1738).

### Compound 64. Sodium 1-(4-(5-(3-chloro-4-isopropoxyphenyl)-4,5-dihydroisoxazole-3-yl)benzyl)azetidine-3-carboxylate

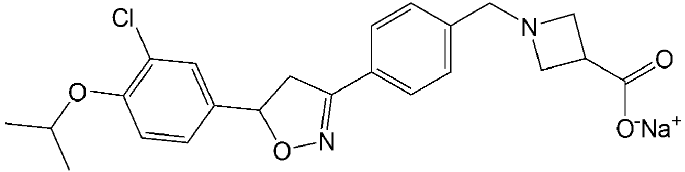

Whild solid;
Yield: 98%;
¹H NMR (CD₃OD, 400 MHz) *δ* 7.69 (d, *J* = 8.1 Hz, 2 Ar**H**), 7.38-7.42 (m, 3 Ar**H**), 7.28 (dd, *J* = 2.1, 8.4 Hz, 1 Ar**H**), 7.07 (d, *J* = 8.5 Hz, 1 Ar**H**), 5.66 (dd, *J* = 8.5, 10.6 Hz, 1 isoxazoline ring-**H**), 4.61-4.89 (m, OC**H**(CH₃-)₂), 3.84 (dd, *J* = 10.8, 17.0 Hz, 1 isoxazoline ring-**H**), 3.67 (s, NC**H**₂), 3.54 (t, *J* = 8.0 Hz, 2 azetidine ring-**H**), 3.32-3.40 (m, 1 isoxazoline ring-**H**, 2 azetidine ring-**H**), 3.20-3.26 (m, 1 azetidine ring-**H**), 1.35 (d, *J* = 6.0 Hz, OCH(C**H**₃-)₂);
¹³C NMR (CD₃OD, 100 MHz) *δ* 179.60 (**C**(O)), 156.79 (isoxazoline ring-**C**), 153.42, 139.76, 134.15, 128.98, 128.29, 127.75, 126.57, 125.33, 123.78, 115.65 (Ar**C**), 81.77 (isoxazoline ring-**C**), 71.68 (O**C**H(CH₃-)₂), 62.48 (N**C**H₂), 57.69 (azetidine ring-**C**), 42.29 (isoxazoline ring-**C**), 36.54 (azetidine ring-**C**), 20.91 (OCH(**C**H₃-)₂);
HPLC purity: 5.7 min, 98.1%;
HRMS (M + H)⁺ (ESI⁺) 429.1582 [M + H]⁺ (calcd for C₂₃H₂₅ClN₂O₄H⁺ 429.1581).

### Compound 65. Methyl 1-(4-(5-(4-isopropoxy-3-(trifluoromethyl)phenyl)-4,5-dihydroisoxazole-3-yl)benzyl)azetidine-3-carboxylate

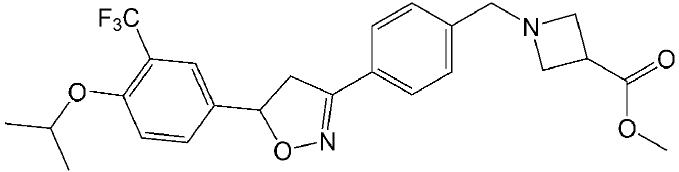

Transparent oil;
Yield: 35%;
¹H NMR (CDCl₃, 400 MHz) *δ* 7.62 (d, *J* = 8.0 Hz, 2Ar**H**), 7.54 (s, 1 Ar**H**), 7.48 (d, *J* = 8.5 Hz, 1 Ar**H**), 7.31 (d, *J* = 8.0 Hz, 2 Ar**H**), 6.98 (d, *J* = 8.6 Hz, 1 Ar**H**), 5.68 (dd, *J =* 8.7, 10.5 Hz, 1 isoxazoline ring-**H**), 4.60-4.63 (m, OC**H**(CH₃-)₂), 3.74-3.77 (m, 1 isoxazoline ring-**H**), 3.73 (s, COOC**H**₃), 3.69 (s, NC**H**₂), 3.61-3.63 (m, 2 azetidine ring-**H**), 3.25-3.32 (m, 1 isoxazoline ring-**H**, 3 azetidine ring-**H**), 1.33 (d, *J* = 6.0 Hz, OCH(C**H**₃-)₂);
¹³C NMR (CDCl₃, 100 MHz) *δ* 173.49 (**C**(O)), 156.02 (isoxazoline ring-**C**), 140.19, 132.11, 130.69, 128.79, 128.16, 126.83 (Ar**C**), 125.05 (q, *J*_{C-F} = 4.9 Hz), 123.48 (q, *J*_{C-F} = 270.8 Hz), 120.02 (q, *J*_{C-F} = 30.5 Hz), 114.57 (Ar**C**), 81.75 (isoxazoline ring-**C**), 71.42 (O**C**H(CH₃-)₂), 63.00 (N**C**H₂), 56.87 (azetidine ring-**C**), 51.95 (COO**C**H₃), 43.04 (isoxazoline ring-**C**), 33.96 (azetidine ring-**C**), 21.79 (OCH(**C**H₃-)₂);
HPLC purity: 5.1 min, 99.4%;
HRMS (M + H)⁺ (ESI⁺) 477.2006 [M + H]⁺ (calcd for C₂₅H₂₇F₃N₂O₄H⁺ 477.2001).

### Compound 66. Sodium 1-(4-(5-(4-isopropoxy-3-(trifluoromethyl)phenyl)-4,5-dihydroisoxazole-3-yl)benzyl)azetidine-3-carboxylate

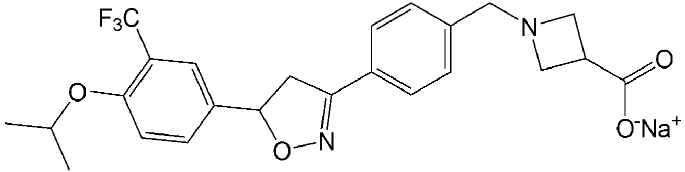

Whild solid;
Yield: 92%;
¹H NMR (CD₃OD, 400 MHz) *δ* 7.71 (d, *J* = 8.1 Hz, 2 Ar**H**), 7.55-7.57 (m, 2 Ar**H**), 7.43 (d, *J* = 8.1 Hz, 2 Ar**H**), 7.18 (d, *J* = 8.3 Hz, 1 Ar**H**), 5.72 (dd, *J* = 8.7, 10.6 Hz, 1 isoxazoline ring-**H**), 4.74 (sept, *J* = 6.0 Hz, OC**H**(CH₃-)₂), 3.93 (s, NC**H**₂), 3.37-3.88 (m, 1 isoxazoline ring-**H**, 2 azetidine ring-**H**), 3.63-3.68 (m, 2 azetidine ring-**H**), 3.24-3.38 (m, 1 isoxazoline ring-**H**, 1 azetidine ring-**H**), 1.32 (d, *J* = 6.0 Hz, OCH(C**H**₃-)₂);
¹³C NMR (CD₃OD, 100 MHz) *δ* 178.16 (**C**(O)), 156.63, 155.95, 136.92, 132.37, 130.95, 129.44, 129.19 (ArC, isoxazoline ring-**C**), 124.55 (q, *J*_{C-F} = 5.3 Hz), 123.65 (q, *J*_{C-F} = 270.0 Hz), 119.76, 119.45, 119.15, 118.85 (q, *J*_{C-F} = 30.3 Hz), 114.55 (Ar**C**), 81.93 (isoxazoline ring-**C**), 71.03 (O**C**H(CH₃-)₂), 60.55 (N**C**H₂), 57.24, 42.22, 35.80, 20.74 (OCH(**C**H₃-)₂);
HPLC purity: 3.8 min, 100%;
HRMS (M + H)⁺ (ESI⁺) 463.1848 [M + H]⁺ (calcd for C₂₄H₂₅F₃N₂O₄H⁺ 463.1845).

### Compound 67. Methyl 1-(4-(5-(pyridine-2-yl)-4,5-dihydroisoxazole-3-yl)benzyl)azetidine-3-carboxylate

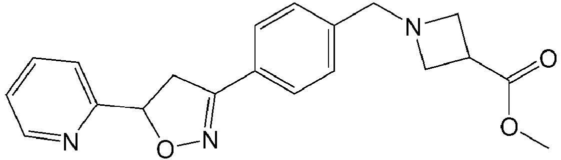

Transparent oil;
Yield: 46%;
¹H NMR (CDCl₃, 400 MHz) *δ* 8.57 (d, *J* = 4.1 Hz, 1 Ar**H**), 7.70 (td, *J* = 1.7, 7.6 Hz, 1 Ar**H**), 7.64 (d, *J* = 8.2 Hz, 2 Ar**H**), 7.56 (d, *J* = 7.8 Hz, 1 Ar**H**), 7.31 (d, *J* = 8.2 Hz, 2 Ar**H**), 7.20-7.23, (m, 1 Ar**H**), 5.81-5.85 (m, 1 isoxazoline ring-**H**), 3.78-3.85 (m, 1 isoxazoline ring-**H**), 3.66-3.74 (m, COOC**H**₃, 1 isoxazoline ring-**H**), 3.62 (s, NC**H**₂), 3.49-3.55 (m, 2 azetidine ring-**H**), 3.30-3.36 (m, 3 azetidine ring-**H**);
¹³C NMR (CDCl₃, 100 MHz) *δ* 173.45 (**C**(O)), 159.93, 156.31, 149.36, 140.00, 136.96, 128.74, 128.16, 126.93, 122.91, 120.57 (Ar**C**, isoxazoline ring-**C**), 82.40 (isoxazoline ring-**C**), 62.95 (N**C**H₂), 56.81 (azetidine ring-**C**), 51.96 (COO**C**H₃), 41.48 (isoxazoline ring-**C**), 33.92 (azetidine ring-**C**);
HPLC purity: 5.1 min, 98.8%;
HRMS (M + H)⁺ (ESI⁺) 352.1661 [M + H]⁺ (calcd for C₂₀H₂₁N₃O₃H⁺ 352.1661).

### Compound 68. Sodium 1-(4-(5-(pyridine-2-yl)-4,5-dihydroisoxazole-3-yl)benzyl)azetidine-3-carboxylate

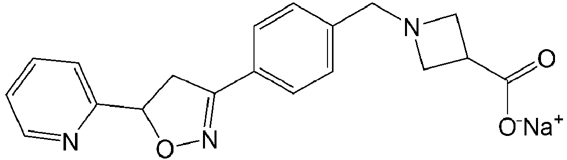

Yellow oil;
Yield: 53%;
¹H NMR (CD₃OD, 400 MHz) *δ* 8.90 (d, *J* = 5.6 Hz, 1 Ar**H**), 8.69-8.73 (m, 1 Ar**H**), 8.23 (d, *J* = 8.0 Hz, 1 Ar**H**), 8.10 (d, *J* = 6.8 Hz, 1 Ar**H**), 7.84 (d, *J* = 8.0 Hz, 2 Ar**H**), 7.66-7.71, (m, 2 Ar**H**), 6.29-6.34 (m, 1 isoxazoline ring-**H**), 4.20-4.61 (m, 7**H**), 3.37-3.89 (m, 2**H**), 3.32-3.33 (m, 1**H);**
¹³C NMR (CD₃OD, 100 MHz) *δ* 171.98, 171.47, 156.73, 154.70, 147.33, 141.82, 132.25 (d, *J* = 21.8 Hz), 130.55 (d, *J* = 8.2 Hz), 129.73, 127.73, 126.62, 124.85 (Ar**C**, isoxazoline ring-**C**), 78.14 (isoxazoline ring-**C**), 57.26 (d, *J* = 22.8 Hz, N**C**H₂), 55.27 (d, *J* = 25.6 Hz, azetidine ring-**C**), 41.89 (isoxazoline ring-**C**), 32.26 (azetidine ring-**C**);
HPLC purity: 5.1 min, 95.3%;
HRMS (M + H)⁺ (ESI⁺) 338.1503 [M + H]⁺ (calcd for C₁₉H₁₉N₃O₃H⁺ 338.1505).

### Compound 69. Methyl 1-(4-(5-(3-chloro-4-isopropoxyphenyl)-4,5-dihydroisoxazole-3-yl)benzyl)pyrrolidine-3-carboxylate

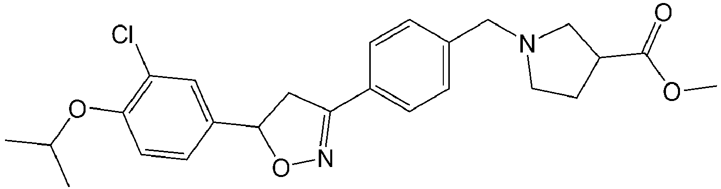

Yellow oil;
Yield: 53%;
¹H NMR (CDCl₃, 400 MHz) *δ* 7.63 (d, *J* = 8.1 Hz, 2 Ar**H**), 7.35-7.39 (m, 3 Ar**H**), 7.21 (dd, *J* = 2.1, 8.4 Hz, 1 Ar**H**), 6.92 (d, *J* = 8.5 Hz, 1 Ar**H**), 5.63 (dd, *J* = 8.2, 10.8 Hz, 1 isoxazoline ring-**H**), 4.52-4.55 (m, OC**H**(CH₃-)₂), 3.73 (dd, *J* = 10.9, 16.6 Hz, 1 isoxazoline ring-**H**), 3.67 (s, OC**H**₃), 3.65 (d, *J* = 4.0 Hz, NC**H**₂), 3.30 (dd, *J* = 8.2, 16.6 Hz, 1 isoxazoling ring-**H**), 3.01-3.05 (m, 1 pyrrolidine ring-**H**), 2.87 (t, *J* = 9.0 Hz, 1 pyrrolidine ring-**H**), 2.64-2.68 (m, 2 pyrrolidine ring-**H**), 2.54 (q, *J* = 7.6 Hz, 1 pyrrolidine ring-**H**), 2.08-2.12 (m, 2 pyrrolidine ring-**H**), 1.36 (d, *J* = 6.0 Hz, OCH(C**H**₃)₂);
¹³C NMR (CDCl₃, 100 MHz) *δ* 175.43 (**C**(O)), 156.01, 153.59, 141.27, 134.11, 129.09, 128.14, 128.09, 126.77, 125.24, 124.56, 116.02 (Ar**C**, isoxazoline ring-**C**), 81.66 (isoxazoline ring-**C**), 72.25 (O**C**H(CH₃-)₂), 59.67 (N**C**H₂), 56.63, 53.75 (pyrrolidine ring-**C**), 51.92 (O**C**H₃), 43.07 (pyrrolidine ring-**C**), 41.97 (isoxazoline ring-**C**), 27.69 (pyrrolidine ring-**C**), 22.01 (OCH(**C**H₃-)₂);
HPLC purity: 5.3 min, 98.7%;
HRMS (M + H)⁺ (ESI⁺) 457.1895 [M + H]⁺ (calcd for C₂₅H₂₉ClN₂O₄H⁺ 457.1894).

### Compound 70. Sodium 1-(4-(5-(3-chloro-4-isopropoxyphenyl)-4,5-dihydroisoxazole-3-yl)benzyl)pyrrolidine-3-carboxylate

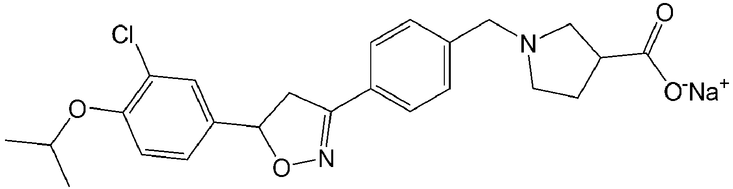

Yellow solid;
Yield: 100%;
¹H NMR (CD₃OD, 400 MHz) *δ* 7.66 (d, *J* = 8.0 Hz, 2 Ar**H**), 7.38-7.42 (m, 3 Ar**H**), 7.25 (dd, *J* = 1.5, 8.4 Hz, 1 Ar**H**), 7.04 (d, *J* = 8.5 Hz, 1 Ar**H**), 5.63 (t, *J* = 10.2 Hz, 1 isoxazoline ring-**H**), 5.61-5.65 (m, OC**H**(CH₃-)₂), 3.80 (dd, *J* = 10.8, 17.0 Hz, 1 isoxazoline ring-**H**), 3.61-3.69 (m, NC**H**₂), 3.31-3.36 (m, 1 isoxazoline ring-**H**), 2.89-2.99 (m, 2 pyrrolidine ring-**H**), 2.72-2.77 (m, 1 pyrrolidine ring-**H**), 2.61 (t, *J* = 8.0 Hz, 1 pyrrolidine ring-**H**), 2.49 (q, *J* = 8.2 Hz, 1 pyrrolidine ring-**H**), 2.00-2.09 (m, 2 pyrrolidine ring-**H**), 1.31 (d, *J* = 6.0 Hz, OCH(C**H**₃-)₂);
¹³C NMR (CD₃OD, 100 MHz) *δ* 181.64 (**C**(O)), 156.81, 153.42, 140.79, 134.16, 129.43, 128.19, 127.75, 126.48, 125.35, 123.78, 115.66 (Ar**C**, isoxazoline ring-**C**), 81.75 (isoxazoline ring-**C**), 71.69 (O**C**H(CH₃-)₂), 59.73 (N**C**H₂), 57.75, 53.78, 45.15 (pyrrolidine ring-**C**), 42.32 (isoxazoline ring-**C**), 28.26 (pyrrolidine ring-**C**), 20.93 (OCH(**C**H₃-)₂);
HPLC purity: 5.3 min, 98.7%;
HRMS (M + H)⁺ (ESI⁺) 443.1741 [M + H]⁺ (calcd for C₂₄H₂₇ClN₂O₄H⁺ 443.1738).

### Compound 71. Sodium 1-(4-(5-(3-chloro-4-isopropoxyphenyl)-4,5-dihydroisoxazole-3-yl)benzyl)piperidine-4-carboxylate

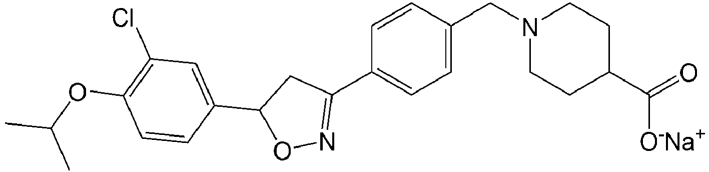

Whild solid;
Yield: 99%;
¹H NMR (CD₃OD, 400 MHz) *δ* 7.69 (d, *J* = 8.2 Hz, 2 Ar**H**), 7.40-7.44 (m, 3 Ar**H**), 7.28 (dd, *J* = 2.1, 8.4 Hz, 1 Ar**H**), 7.08 (d, *J* = 8.5 Hz, 1 Ar**H**), 5.66 (dd, *J* = 8.6, 10.6 Hz, 1 isoxazoline ring-**H**), 4.60-4.67 (m, OC**H**(CH₃-)₂), 3.84 (dd, *J* = 10.8, 17.0 Hz, 1 isoxazoline ring-**H**), 3.61 (s, NC**H**₂), 3.35-3.40 (m, 1 isoxazoline ring-**H**), 2.92 (d, *J* = 11.6 Hz, 2 piperidine ring-**H**), 2.10-2.20 (m, 3 piperidine ring-**H**), 1.86 (s, 2 piperidine ring-**H**), 1.73-1.80 (m, 2 piperidine ring-**H**), 1.33 (d, *J* = 6.0 Hz, OCH(C**H**₃-)₂);
¹³C NMR (CD₃OD, 100 MHz) *δ* 207.53 (**C**(O)), 156.81 (isoxazoline ring-**C**), 153.57, 141.36, 134.28, 129.94, 127.75, 126.45, 125.29, 123.79, 115.65, 113.63 (Ar**C**), 81.81 (isoxazoline ring-**C**), 71.67 (O**C**H(CH₃-)₂), 62.15 (N**C**H₂), 53.05 (piperidine ring-**C**), 42.29 (isoxazoline ring-**C**), 28.62 (piperidine ring-**C**), 20.86 (OCH(**C**H₃-)₂);
HPLC purity: 4.9 min, 98.3%;
HRMS (M + H)⁺ (ESI⁺) 457.1896 [M + H]⁺ (calcd for C₂₅H₂₉ClN₂O₄H⁺ 457.1894).

### Experimental Example 1: Verification of Activity of S1P₁ Agonist

In order to confirm the effect on S1P₁ activity of Compounds 1 to 71 synthesized according to the above Examples 1 to 3, an *in vitro* assay was performed using a cell-based evaluation system, and the results are shown in Table 1.

In order to analyze the efficacy of functional antagonists, two S1P₁ overexpressing cell lines were used, which enables to measure the degree of internalization of the receptor through β-arrestin that occurs after GPCR activation. A cell line that measures the degree of β-arrestin recruitment to the intracellular end of the receptor after S1P₁ receptor activation and a cell line that measures the degree of endosome formation by internalization that occurs after S1P₁ receptor activation were used, and all of the cell lines above and the materials used in the exemplary embodiments below were purchased from DiscoverX of Eurofins.

The S1P₁ receptor activity of the compounds of the present disclosure was verified according to the method provided by each manufacturer, and the degree of luminescence according to the treatment concentration of the compounds was measured to calculate the EC₅₀ value, which is the concentration showing half of its activity. Specifically, the cell lines were dispensed at a density of 1.0×10⁴ cells/well in an assay medium and cultured in an incubator for one day, and then the cells were lysed, and luminescence was measured by adding luminescent substrates. The two calculated EC₅₀ values are shown in Table 1 below. Among the 71 compounds, Compounds 13, 62, 64, and 66, which showed excellent activity, were additionally used in the subsequent Experimental Examples.

**[Table 1]**

| Comp ound | β-arrestin EC₅₀ (nM) | Internalization EC₅₀ (nM) | Comp ound | β-arrestin EC₅₀ (nM) | Internalization EC₅₀ (nM) |
|---|---|---|---|---|---|
| 1 | 11.1 | 173.1 | 37 | 7.88 | 13.5 |
| 2 | 2.12 | 2.20 | 38 | 0.58 | 0.14 |
| 3 | 23.6 | 116.5 | 39 | 39.2 | 159.6 |
| 4 | 70.1 | 141.35 | 40 | 10.6 | 23.1 |
| 5 | 3.64 | 0.87 | 41 | 0.90 | 9.52 |
| 6 | 0.37 | 0.065 | 42 | 0.81 | 0.37 |
| 7 | 1.17 | 4.33 | 43 | > 1,000 | > 1,000 |
| 8 | 29.9 | 33.1 | 44 | > 1,000 | > 1,000 |
| 9 | 1.82 | 1.59 | 45 | > 1,000 | > 1,000 |
| 10 | 0.45 | 0.36 | 46 | > 1,000 | > 1,000 |
| 11 | 1.49 | 12.6 | 47 | > 1,000 | > 1,000 |
| 12 | 1.41 | 1.48 | 48 | > 1,000 | > 1,000 |
| 13 | 0.22 | 0.049 | 49 | > 1,000 | > 1,000 |
| 14 | 1.13 | 4.91 | 50 | 567±16.4 | 414±5.80 |
| 15 | 0.54 | 0.27 | 51 | > 1,000 | > 1,000 |
| 16 | 48.53 | 139.5 | 52 | > 1,000 | > 1,000 |
| 17 | 25.85 | 81.3 | 53 | > 1,000 | > 1,000 |
| 18 | 3.24 | 9.26 | 54 | > 1,000 | > ±1,000 |
| 19 | 1.14 | 0.97 | 55 | 542±20.1 | 973±24.3 |
| 20 | 3.05 | 118.7 | 56 | 25.9±0.20 | 30.5±1.23 |
| 21 | 503 | 378.6 | 57 | > 1,000 | 286±4.78 |
| 22 | 8.59 | 97.35 | 58 | 34.6±0.27 | 112±8.69 |
| 23 | > 1,000 | > 1,000 | 59 | 95.7±3.96 | 346±6.92 |
| 24 | > 1,000 | > 1,000 | 60 | 18.1±0.14 | 34.6±0.31 |
| 25 | 252.5 | 346.7 | 61 | 100±0.50 | 122±4.80 |
| 26 | > 1,000 | > 1,000 | 62 | 20.4±0.67 | 16.8±0.46 |
| 27 | > 1,000 | > 1,000 | 63 | 23.1±0.69 | 45.3±3.24 |
| 28 | > 1,000 | > 1,000 | 64 | 7.03±0.09 | 11.8±0.28 |
| 29 | 635.1 | 378.6 | 65 | 86.6±1.05 | 38.5±0.81 |
| 30 | 8.50 | 205.4 | 66 | 5.57±0.09 | 5.09±0.08 |
| 31 | 2.59 | 4.21 | 67 | 392±5.42 | 594±14.9 |
| 32 | 580.4 | 518.6 | 68 | 346±6.08 | 237±6.13 |
| 33 | 1.79 | 11.9 | 69 | 74.4±1.93 | 146±5.38 |
| 34 | 1.08 | 0.58 | 70 | 11.1±0.13 | 14.3±0.44 |
| 35 | 21.05 | 11.5 | 71 | 111±2.74 | 80.6±3.04 |
| 36 | 2.03 | 2.62 | | | |

### Experimental Example 2: Verification of Decrease in Blood Lymphocyte Count

In order to confirm the effect of administration of the four compounds selected from Experimental Example 1, Compounds 13, 62, 64, and 66, on the decrease in the blood lymphocyte count in rodents *in vivo,* blood was collected before and after compound administration, and the decrease in the blood lymphocyte count over time was measured using an automatic blood counter, and the results are shown in FIG. 1.

Specifically, 5-week-old male rats (rat, wister, 160-180 g) were acclimated in a temperature- and humidity-controlled breeding room, and the four selected compounds were dissolved in 5% DMSO and 10% Kolliphor HS15 aqueous solution and orally administered at a dose of 10 mg/kg. Blood was collected from the tail vein with a syringe during inhalation anesthesia with 4% isoflurane and transferred to an EDTA-coated tube. The lymphocyte count was measured from the collected blood using an automatic blood counter. As a positive control group, rats administered with fingolimod, a known S1P₁ receptor modulator, were used.

As shown in FIG. 1, the lymphocyte count over time after compound administration was expressed as a percentage (%) based on the lymphocyte count of the rat before compound administration. As in the positive control group, fingolimod-administered group, it was confirmed that the group administered with the compounds of the Examples within 5 hours after drug administration induced a decrease in the lymphocyte count by 50% or less. This indicates that when the compounds of the present disclosure are orally administered to normal rats, a decrease in the blood lymphocyte count can be induced.

### Experimental Example 3: Verification of Alleviation of Symptoms in Multiple Sclerosis Animal Model

In order to confirm the effect of administration of the four compounds selected from the Experimental Example 1, Compounds 13, 62, 64, and 66, on the alleviation of symptoms in a multiple sclerosis animal model, the four compounds were orally administered to a MOG₃₅₋₅₅ peptide-induced multiple sclerosis model, and the changes in the clinical symptoms of stiffness and paralysis were specified and the results are shown in FIG. 2.

A rodent model of experimental autoimmune encephalomyelitis (EAE) induced by myelin oligodendrocyte glycoprotein (MOG), which is most widely used in multiple sclerosis research, was prepared.

Specifically, 10-week-old C57BL6/J male mice were acclimated in a temperature- and humidity-controlled breeding room and then subcutaneously injected with a thoroughly stirred mixture of MOG₃₅₋₅₅ peptide and complete Freund's adjuvant (CFA). Additionally, pertussis toxin dissolved in PBS was injected intraperitoneally on Day 0 and Day 1. The timeline of the preparation of the multiple sclerosis animal model of MOG-induced EAE and the outline of drug administration are shown in the upper part of the graphs of FIGS. 2A and 2G.

The compounds of the present disclosure were dissolved in 5% DMSO and 10% Kolliphor HS15 aqueous solutions and orally administered daily at doses of 0.3, 3, or 10 mg/kg. The negative control group was prepared by orally administering the same amount of vehicle daily, and the positive control group was prepared by orally administering fingolimod daily at a dose of 3 mg/kg, with the same composition as the compound of the present disclosure. The symptoms of the animal models of the negative control group, positive control group, and experimental group prepared as described above were evaluated and scored by applying the following criteria:
0: No abnormality,
0.5: Loss of tension in tail,
1.0: Loss of reflexes in tail,
2.0: Loss of reflexes in tail and sensory abnormalities in one leg,
3.0: Sensory abnormalities and paralysis in one leg,
3.5: Paralysis of both hind legs,
4.0: Paralysis of forelegs and hind legs,
5.0: Moribund or dead.

As a result of evaluating the efficacy of the drug using the aforementioned symptom criteria, as shown in FIGS. 2A and 2G, in the case of the negative control group that was not treated with the drug, the symptoms of multiple sclerosis became more severe over time, whereas when the compounds of the present invention were orally administered, the daily symptoms were alleviated or improved, and the symptom severity of the total degree of symptoms accumulated over the test period (FIG. 2B) and the maximum degree of symptom development (FIG. 2C) was alleviated in a concentration-dependent manner. Additionally, as shown in FIGS. 2D and 2E, respectively, regarding the change in daily body weight and the rate of final body weight change, the severity of the disease was lowered when the compounds of the present invention were administered, which significantly reduced the loss of body weight by reducing the overall inflammatory response and food intake restriction due to paralysis. Furthermore, as shown in FIGS. 2F and 2H, it was confirmed that when the compound of the present disclosure was administered, the onset of symptoms was delayed or suppressed.

In summary, it was confirmed that the compounds of the present disclosure improve the neurological clinical symptom criteria of the disease in an EAE animal model induced by the MOG peptide, indicating that the compounds of the present disclosure can be effectively used for the prevention or treatment of neurological autoimmune diseases, particularly multiple sclerosis.

Based on the above description, it will be understood by those skilled in the art that the present disclosure may be implemented in a different specific form without changing the technical spirit or essential characteristics thereof. In this regard, it should be understood that the above embodiments are not limitative, but illustrative in all aspects. The scope of the present disclosure is defined by the appended claims rather than by the preceding description, and therefore all changes and modifications that fall within metes and bounds of the claims, or equivalents of such metes and bounds are therefore intended to be embraced by the claims.

## Claims

1. A compound represented by Formula 1 below or a pharmaceutically acceptable salt thereof: 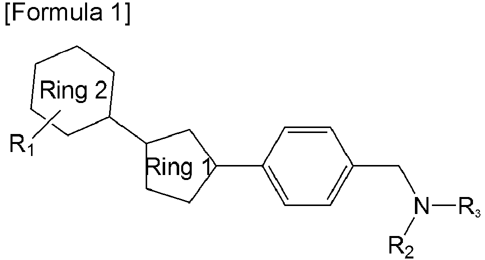 In the Formula 1,
Ring 1 is selenazolenyl or isoxazolenyl;
Ring 2 is C₆₋₁₀ aryl or 5 to 10-membered heteroaryl;
R₁ is one or more substituents identical or different from each other selected from the group consisting of hydrogen, cyano, halo(gen), hydroxy, C₁₋₆ alkyl, C₁₋₆ alkoxy, and C₁₋₆ haloalkyl;
R₂ and R₃ are each independently hydrogen, C₁₋₆ alkoxycarbonyl-C₁₋₆ alkyl, or sodium carboxy-C₁₋₆ alkyl; or
R₂ and R₃ are connected to each other to form an unsubstituted or substituted 3 to 10-membered heterocyclyl including the nitrogen to which the R₂ and R₃ are attached.

2. The compound or a pharmaceutically acceptable salt thereof of claim 1, which is represented by any one of Formulae 2 to 4 below:

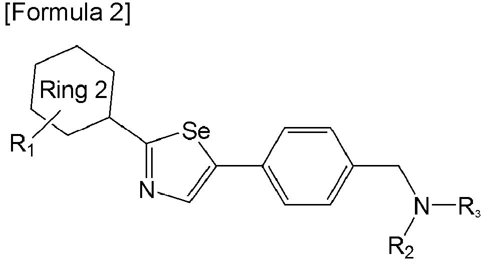

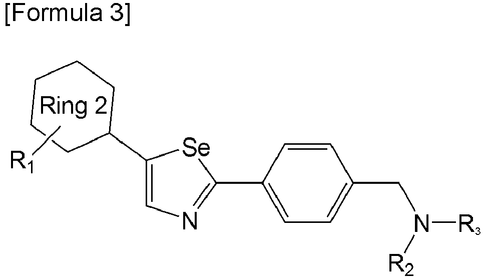

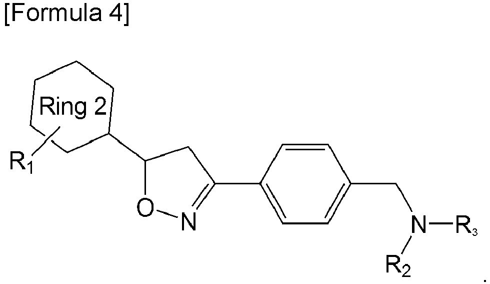

3. The compound or a pharmaceutically acceptable salt thereof of claim 1, wherein
Ring 2 is phenyl or pyridinyl.

4. The compound or a pharmaceutically acceptable salt thereof of claim 1, wherein
R₁ is none, cyano, bromo, chloro, hydroxy, ethyl, propyl, tert-butyl, methoxy, ethoxy, isopropoxy, chloromethyl, or trifluoromethyl.

5. The compound or a pharmaceutically acceptable salt thereof of claim 1, wherein
R₂ is hydrogen, and R₃ is ethoxycarbonylmethyl, methoxycarbonylethyl, sodium carboxymethyl, or sodium carboxyethyl.

6. The compound or a pharmaceutically acceptable salt thereof of claim 1, wherein
R₂ and R₃ are connected to each other to form azetidinyl, pyrrolidinyl, or piperidinyl, which is unsubstituted or substituted with one or more selected from the group consisting of carboxy, methoxycarbonyl, ethoxycarbonyl, tert-butoxycarbonyl, and sodium carboxy including the nitrogen to which the R₂ and R₃ are attached.

7. The compound or a pharmaceutically acceptable salt thereof of claim 1, wherein the compound is
1. Methyl 1-(4-(2-(4-tert-butylphenyl)-1,3-selenazole-5-yl)benzyl)azetidine-3-carboxylate;
2. Sodium 1-(4-(2-(4-tert-butylphenyl)-1,3-selenazole-5-yl)benzyl)azetidine-3-carboxylate;
3. Ethyl 1-(4-(2-(4-tert-butylphenyl)-1,3-selenazole-5-yl)benzyl)azetidine-3-carboxylate;
4. Tert-butyl 1-(4-(2-(4-tert-butylphenyl)-1,3-selenazole-5-yl)benzyl)azetidine-3-carboxylate;
5. Methyl 1-(4-(2-(3-isocyano-4-isopropoxyphenyl)-1,3-selenazole-5-yl)benzyl)azetidine-3-carboxylate;
6. Sodium 1-(4-(2-(3-isocyano-4-isopropoxyphenyl)-1,3-selenazole-5-yl)benzyl)azetidine-3-carboxylate;
7. Ethyl 1-(4-(2-(3-cyano-4-isopropoxyphenyl)-1,3-selenazole-5-yl)benzyl)azetidine-3-carboxylate;
8. Tert-butyl 1-(4-(2-(3-cyano-4-isopropoxyphenyl)-1,3-selenazole-5-yl)benzyl)azetidine-3-carboxylate;
9. Methyl 1-(4-(2-(4-isopropoxy-3-(trifluoromethyl)phenyl)-1,3-selenazole-5-yl)benzyl)azetidine-3-carboxylate;
10. 1-(4-(2-(4-isopropoxy-3-(trifluoromethyl)phenyl)-1,3-selenazole-5-yl)benzyl)azetidine-3-carboxylic acid;
11. Ethyl 1-(4-(2-(4-isopropoxy-3-(trifluoromethyl)phenyl)-1,3-selenazole-5-yl)benzyl)azetidine-3-carboxylate;
12. Methyl 1-(4-(2-(3-chloro-4-isopropoxyphenyl)-1,3-selenazole-5-yl)benzyl)azetidine-3-carboxylate;
13. Sodium 1-(4-(2-(3-chloro-4-isopropoxyphenyl)-1,3-selenazole-5-yl)benzyl)azetidine-3-carboxylate;
14. Methyl 1-(4-(2-(3-chloro-4-ethoxyphenyl)-1,3-selenazole-5-yl)benzyl)azetidine-3-carboxylate;
15. Sodium 1-(4-(2-(3-chloro-4-ethoxyphenyl)-1,3-selenazole-5-yl)benzyl)azetidine-3-carboxylate;
16. Methyl 1-(4-(2-(4-methoxyphenyl)-1,3-selenazole-5-yl)benzyl)azetidine-3-carboxylate;
17. 1-(4-(2-(4-methoxyphenyl)-1,3-selenazole-5-yl)benzyl)azetidine-3-carboxylic acid;
18. Methyl 1-(4-(2-(4-isopropoxyphenyl)-1,3-selenazole-5-yl)benzyl)azetidine-3-carboxylate;
19. 1-(4-(2-(4-isopropoxyphenyl)-1,3-selenazole-5-yl)benzyl)azetidine-3-carboxylic acid;
20. Ethyl 1-(4-(2-(4-isopropoxyphenyl)-1,3-selenazole-5-yl)benzyl)azetidine-3-carboxylate;
21. Methyl 1-(4-(2-(3-isopropoxyphenyl)-1,3-selenazole-5-yl)benzyl)azetidine-3-carboxylate;
22. 1-(4-(2-(3-isopropoxyphenyl)-1,3-selenazole-5-yl)benzyl)azetidine-3-carboxylic acid;
23. Methyl 1-(4-(2-(2-isopropoxyphenyl)-1,3-selenazole-5-yl)benzyl)azetidine-3-carboxylate;
24. 1-(4-(2-(2-isopropoxyphenyl)-1,3-selenazole-5-yl)benzyl)azetidine-3-carboxylic acid;
25. Ethyl 1-(4-(2-(2-isopropoxyphenyl)-1,3-selenazole-5-yl)benzyl)azetidine-3-carboxylate;
26. Methyl 1-(4-(2-(pyridine-2-yl)-1,3-selenazole-5-yl)benzyl)azetidine-3-carboxylate;
27. Sodium 1-(4-(2-(pyridine-2-yl)-1,3-selenazole-5-yl)benzyl)azetidine-3-carboxylate;
28. Methyl 1-(4-(2-(pyridine-3-yl)-1,3-selenazole-5-yl)benzyl)azetidine-3-carboxylate;
29. Sodium 1-(4-(2-(pyridine-3-yl)-1,3-selenazole-5-yl)benzyl)azetidine-3-carboxylate;
30. Methyl 1-(4-(2-(4-tert-butylphenyl)-1,3-selenazole-5-yl)benzyl)pyrrolidine-3-carboxylate;
31. Sodium 1-(4-(2-(4-tert-butylphenyl)-1,3-selenazole-5-yl)benzyl)pyrrolidine-3-carboxylate;
32. Methyl 1-(4-(2-(4-tert-butylphenyl)-1,3-selenazole-5-yl)benzyl)piperidine-4-carboxylate;
33. Methyl 1-(4-(2-(3-chloro-4-isopropoxyphenyl)-1,3-selenazole-5-yl)benzyl)pyrrolidine-3-carboxylate;
34. Sodium 1-(4-(2-(3-chloro-4-isopropoxyphenyl)-1,3-selenazole-5-yl)benzyl)pyrrolidine-3-carboxylate;
35. Ethyl 2-(4-(2-(3-chloro-4-isopropoxyphenyl)-1,3-selenazole-5-yl)benzylamino)acetate;
36. Sodium 2-(4-(2-(3-chloro-4- isopropoxyphenyl)-1,3-selenazole-5-yl)benzylamino)acetate;
37. Methyl 3-(4-(2-(3-chloro-4-isopropoxyphenyl)-1,3-selenazole-5-yl)benzylamino)propanoate;
38. Sodium 3-(4-(2-(3-chloro-4-isopropoxyphenyl)-1,3-selenazole-5-yl)benzylamino)propanoate;
39. Methyl 1-(4-(5-(4-tert-butylphenyl)-1,3-selenazole-2-yl)benzyl)azetidine-3-carboxylate;
40. Sodium 1-(4-(5-(4-tert-butylphenyl)-1,3-selenazole-2-yl)benzyl)azetidine-3-carboxylate;
41. Methyl 1-(4-(5-(3-chloro-4-isopropoxyphenyl)-1,3-selenazole-2-yl)benzyl)azetidine-3-carboxylate;
42. Sodium 1-(4-(5-(3-chloro-4-isopropoxyphenyl)-1,3-selenazole-2-yl)benzyl)azetidine-3-carboxylate;
43. Methyl 1-(4-(5-phenyl-4,5-dihydroisoxazole-3-yl)benzyl)azetidine-3-carboxylate;
44. 1-(4-(5-phenyl-4,5-dihydroisoxazole-3-yl)benzyl)azetidine-3-carboxylic acid;
45. Methyl 1-(4-(5-(2-bromophenyl)-4,5-dihydroisoxazole-3-yl)benzyl)azetidine-3-carboxylate;
46. 1-(4-(5-(2-bromophenyl)-4,5-dihydroisoxazole-3-yl)benzyl)azetidine-3-carboxylic acid;
47. Methyl 1-(4-(5-(3-bromophenyl)-4,5-dihydroisoxazole-3-yl)benzyl)azetidine-3-carboxylate;
48. 1-(4-(5-(3-bromophenyl)-4,5-dihydroisoxazole-3-yl)benzyl)azetidine-3-carboxylic acid;
49. Methyl 1-(4-(5-(4-bromophenyl)-4,5-dihydroisoxazole-3-yl)benzyl)azetidine-3-carboxylate;
50. 1-(4-(5-(4-bromophenyl)-4,5-dihydroisoxazole-3-yl)benzyl)azetidine-3-carboxylic acid;
51. 1-(4-(5-(4-hydroxyphenyl)-4,5-dihydroisoxazole-3-yl)benzyl)azetidine-3-carboxylic acid;
52. Methyl 1-(4-(5-(4-(chloromethyl)phenyl)-4,5-dihydroisoxazole-3-yl)benzyl)azetidine-3-carboxylate;
53. Sodium 1-(4-(5-(4-(chloromethyl)phenyl)-4,5-dihydroisoxazole-3-yl)benzyl)azetidine-3-carboxylate;
54. Methyl 1-(4-(5-(4-ethylphenyl)-4,5-dihydroisoxazole-3-yl)benzyl)azetidine-3-carboxylate;
55. Sodium 1-(4-(5-(4-ethylphenyl)-4,5-dihydroisoxazole-3-yl)benzyl)azetidine-3-carboxylate;
56. Sodium 1-(4-(5-(4-propylphenyl)-4,5-dihydroisoxazole-3-yl)benzyl)azetidine-3-carboxylate;
57. Methyl 1-(4-(5-(4-tert-butylphenyl)-4,5-dihydroisoxazole-3-yl)benzyl)azetidine-3-carboxylate;
58. Sodium 1-(4-(5-(4-tert-butylphenyl)-4,5-dihydroisoxazole-3-yl)benzyl)azetidine-3-carboxylate;
59. Methyl 1-(4-(5-(3-cyano-4-isopropoxyphenyl)-4,5-dihydroisoxazole-3-yl)benzyl)azetidine-3-carboxylate;
60. 1-(4-(5-(3-cyano-4-isopropoxyphenyl)-4,5-dihydroisoxazole-3-yl)benzyl)azetidine-3-carboxylic acid;
61. Methyl 1-(4-(5-(3-chloro-4-ethoxyphenyl)-4,5-dihydroisoxazole-3-yl)benzyl)azetidine-3-carboxylate;
62. Sodium 1-(4-(5-(3-chloro-4-ethoxyphenyl)-4,5-dihydroisoxazole-3-yl)benzyl)azetidine-3-carboxylate;
63. Methyl 1-(4-(5-(3-chloro-4-isopropoxyphenyl)-4,5-dihydroisoxazole-3-yl)benzyl)azetidine-3-carboxylate;
64. Sodium 1-(4-(5-(3-chloro-4-isopropoxyphenyl)-4,5-dihydroisoxazole-3-yl)benzyl)azetidine-3-carboxylate;
65. Methyl 1-(4-(5-(4-isopropoxy-3-(trifluoromethyl)phenyl)-4,5-dihydroisoxazole-3-yl)benzyl)azetidine-3-carboxylate;
66. Sodium 1-(4-(5-(4-isopropoxy-3-(trifluoromethyl)phenyl)-4,5-dihydroisoxazole-3-yl)benzyl)azetidine-3-carboxylate;
67. Methyl 1-(4-(5-(pyridine-2-yl)-4,5-dihydroisoxazole-3-yl)benzyl)azetidine-3-carboxylate;
68. Sodium 1-(4-(5-(pyridine-2-yl)-4,5-dihydroisoxazole-3-yl)benzyl)azetidine-3-carboxylate;
69. Methyl 1-(4-(5-(3-chloro-4-isopropoxyphenyl)-4,5-dihydroisoxazole-3-yl)benzyl)pyrrolidine-3-carboxylate;
70. Sodium 1-(4-(5-(3-chloro-4-isopropoxyphenyl)-4,5-dihydroisoxazole-3-yl)benzyl)pyrrolidine-3-carboxylate; or
71. Sodium 1-(4-(5-(3-chloro-4-isopropoxyphenyl)-4,5-dihydroisoxazole-3-yl)benzyl)piperidine-4-carboxylate.

8. A method for preparing the compound of any one of claims 1 to 7, and a pharmaceutically acceptable salt thereof, comprising: aminating an aldehyde group of a compound represented by Formula 5 below: 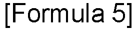 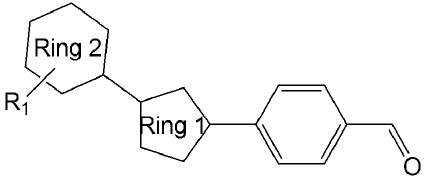 In the Formula 1,
Ring 1 is selenazolenyl or isoxazolenyl;
Ring 2 is C₆₋₁₀ aryl or 5 to 10-membered heteroaryl; and
R₁ is one or more substituents identical or different from each other selected from the group consisting of hydrogen, cyano, halo(gen), hydroxy, C₁₋₆ alkyl, C₁₋₆ alkoxy, and C₁₋₆ haloalkyl.

9. The method of claim 8, wherein the compound of Formula 5 is prepared by:
Step a of reacting R₁-Ring 2-nitrile or 4-(hydroxymethyl)benzonitrile with selenium and carbon monoxide to produce R₁-Ring 2-substituted selenoamide or 4-hydroxybenzoselenamide;
Step b of reacting the product of Step a with 2-bromo-1,1-diethoxyethane and p-toluenesulfonic acid to form a 1,3-selenazole ring in which Ring 2 is substituted at carbon 5 or phenyl is substituted at carbon 2;
Step c of halogenating the unsubstituted carbon 2 or carbon 5 of the 1,3-selenazole ring of the product of Step b; and
Step d of reacting the product of Step c with 4-formylphenylboronic acid or R₁-Ring 2-boronic acid to introduce an additional substituent at the halogenated carbon 2 or carbon 5.

10. The method of claim 8, wherein the compound of Formula 5 is prepared by:
Step a' of reacting N-hydroxy-4-(hydroxymethyl)benzimidoyl chloride with triethylamine to remove hydrochloric acid;
Step b' of reacting the product of Step a' with ethenyl-substituted R₁-Ring 2 to produce (4-(5-(R₁-Ring 2)-4,5-dihydroisoxazole-3-yl)phenyl)methanol; and
Step c' of oxidizing the hydroxyl group on the phenyl ring of the product of Step b'.

11. A pharmaceutical composition for preventing or treating multiple sclerosis (MS), comprising the compound of any one of claims 1 to 7 or a pharmaceutically acceptable salt thereof, as an active ingredient.

12. The pharmaceutical composition of claim 11, wherein the compound is contained in the form of its sodium salt.

13. The pharmaceutical composition of claim 11, wherein the compound acts as a sphingosine-1-phosphate receptor (S1P1) modulator.

14. The pharmaceutical composition of claim 11, which decreases the blood lymphocyte count.

15. The pharmaceutical composition of claim 11, which alleviates symptoms of stiffness or paralysis.

16. The pharmaceutical composition of claim 11, which delays the onset of symptoms of multiple sclerosis.

17. A food composition for preventing or alleviating multiple sclerosis, comprising the compound of any one of claims 1 to 7 or a food-acceptable salt thereof, as an active ingredient.

18. A feed composition for preventing or alleviating multiple sclerosis, comprising the compound of any one of claims 1 to 7 or feed-acceptable salt thereof, as an active ingredient.
